(19)

**Europäisches Patentamt**
**European Patent Office**
**Office européen des brevets**

(11) **EP 4 176 262 B1**

(12) **EUROPEAN PATENT SPECIFICATION**

(45) Date of publication and mention
of the grant of the patent:
**25.06.2025 Bulletin 2025/26**

(21) Application number: **21748661.2**

(22) Date of filing: **02.07.2021**

(51) International Patent Classification (IPC):
**G01N 33/543** (2006.01)

(52) Cooperative Patent Classification (CPC):
**G01N 33/5438**

(86) International application number:
**PCT/IB2021/055981**

(87) International publication number:
**WO 2022/003651 (06.01.2022 Gazette 2022/01)**

(54) **NANOBODY FUNCTIONALIZED ELECTROCHEMICAL TRANSISTORS AND METHODS OF MAKING AND USING THEREOF**

NANOKÖRPERFUNKTIONALISIERTE ELEKTROCHEMISCHE TRANSISTOREN UND
VERFAHREN ZUR HERSTELLUNG UND VERWENDUNG DAVON

TRANSISTORS ÉLECTROCHIMIQUES FONCTIONNALISÉS AUX NANOCORPS ET LEURS
MÉTHODES DE FABRICATION ET D'UTILISATION

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **02.07.2020 US 202063047547 P**
**27.10.2020 US 202063106218 P**

(43) Date of publication of application:
**10.05.2023 Bulletin 2023/19**

(73) Proprietor: **King Abdullah University Of Science
And Technology**
**Thuwal 23955-6900 (SA)**

(72) Inventors:
• **INAL, Sahika**
**Thuwal, 23955-6900 (SA)**
• **AROLD, Stefan T.**
**Thuwal, 23955-6900 (SA)**
• **GRUNBERG, Raik**
**Thuwal, 23955-6900 (SA)**
• **WUSTONI, Shofarul**
**THUWAL, 23955-6900 (SA)**
• **GUO, Keying**
**Thuwal, 23955-6900 (SA)**
• **KOKLU, Anil**
**Thuwal, 23955-6900 (SA)**

(74) Representative: **Ipsilon**
**Le Centralis**
**63, avenue du Général Leclerc**
**92340 Bourg-la-Reine (FR)**

(56) References cited:
**WO-A1-2018/048742      WO-A1-2018/122671**
**WO-A1-2021/130525      US-A1- 2019 331 673**
**US-A1- 2020 033 291**

• MACCHIA ELEONORA ET AL: "Organic
electrochemical transistor immuno-sensor
operating at the femto-molar limit of detection",
2017 7TH IEEE INTERNATIONAL WORKSHOP ON
ADVANCES IN SENSORS AND INTERFACES
(IWASI), IEEE, 2017, pages 68 - 72, XP033119536
• OH JU-HWAN ET AL: "Recent advances in
chemical functionalization of nanoparticles with
biomolecules for analytical applications",
ANALYTICAL AND BIOANALYTICAL
CHEMISTRY, vol. 407, no. 29, 2015, pages 8627 -
8645, XP035867547
• GUO KEYING ET AL: "Rapid single-molecule
detection of COVID-19 and MERS antigens via
nanobody-functionalized organic
electrochemical transistors", NATURE
BIOMEDICAL ENGINEERING, vol. 5, no. 7, 24
May 2021 (2021-05-24), pages 666 - 677,
XP037510041

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**(Cont. next page)**

- TORRICELLI FABRIZIO: "Enhanced multifunctional bioelectronics with integrated organic electrochemical transistor architectures", PROGRESS IN BIOMEDICAL OPTICS AND IMAGING, SPIE - INTERNATIONAL SOCIETY FOR OPTICAL ENGINEERING, vol. 11663, 5 March 2021 (2021-03-05), pages 116630P - 116630P, XP060141062

- FAHLMAN MATS ET AL: "Interfaces in organic electronics", NATURE REVIEWS MATERIALS, vol. 4, no. 10, 2019, pages 627 - 650, XP036898596

**Description**

**CROSS-REFERENCE TO RELATED APPLICATIONS**

This application claims the benefit of and priority to U.S. Provisional Application No. 63/047,547 filed March July 2, 2020 and U.S. Provisional Application No. 63/106,218 filed October 27, 2020.

**FIELD OF THE INVENTION**

This invention relates to organic electrochemical transistors (OECT)-based immunosensor devices and methods useful in detecting an analyte of interest in a sample.

**BACKGROUND OF THE INVENTION**

Reliable biomolecular diagnostics are an important tool for early detection of diseases, particularly for preventing the outbreak of infectious diseases such as HIV, Ebola and recently widespread coronavirus (SARS-CoV-2) which has caused one million fatalities worldwide, at the time of this writing. Early stage detection is particularly useful to identify and isolate infected patients without symptoms before spreading the disease. Thus, it is crucial to develop a detection method that can offer rapid, easy-use and accurate results. To date, the practical and commercially available detection methods are mostly based on polymerase chain reaction (PCR) or closely related methods. Although Reverse Transcription PCR (RT-PCR) offers accurate and high-sensitivity detection capability, it requires specialist labor, long assay preparation and expensive equipment and can thus only be performed in specialized labs. For this reason, sample-to-result turn-around times vary from several hours to several days. This delay is severely hampering the response to an unfolding epidemic. Rapid tests based on lateral-flow variants of the Enzyme-linked immunosorbent assay (ELISA) can be performed on the spot and give results in about 15 min. However, the lower signal amplification of this test format limits their sensitivity to approximately 1 million particles per mL which is too low for the detection of many infections (be they bacterial or viral). Moreover, lateral flow ELISA rapid tests cannot provide quantitative information on pathogen load, which is considered an important parameter for the diagnosis of individuals as well as in the management of containment measures. Hence, label-free, rapid, simple-to-operate and highly sensitive antigen sensors are of great interest for the development of point-of-care technology (POCT) or bedside diagnostics and would have many further applications beyond pathogen detection.

Organic electrochemical transistors (OECTs) have attracted attention as a promising alternative biosensing technology that can surpass other state-of-the-art electrical biosensors approaches such as the better-known Field Effect Transistors (FETs). OECTs possess an organic semi-conducting material in their channel and a transducing gate electrode immersed in an ionic conducting electrolyte (Rivnay et al., 2018). OECTs enable biosensing applications in an aqueous environment with low voltage operation (<1V) and exceptionally high amplification (Lin and Yan, 2012). OECTs detect target analytes by monitoring the capacitive or faradaic changes occurring in the device interfaces. To date, the actual biosensing signal is most commonly provided from enzymatic redox reactions that are fueled by a specific analyte. Analyte binding to an enzyme induces a redox reaction which then causes a potential drop across the device interface which is then amplified into a change of electrical current flowing from transistor source to drain terminals (Bernard et al., 2008; Ohayon et al., 2020). An important advantage of OECT biosensors is that their selectivity can be tuned by incorporating suitable bio-recognition elements without the need for fluorescent-, radio- or other labels (Wustoni et al., 2019; Wustoni et al., 2020). Despite a remarkable sensing performance, the development of immunosensors based on the OECT platform has been more challenging and sparse due to the non-redox nature of the antibody-antigen (Ab-Ag) interaction (Gentili et al., 2018). The lack of channel materials which are more suitable for sensing applications and are compatible with conventional electronic circuits hampers the widespread use of OECTs for sensing. Additionally, the fragility of most antibodies and the lack of control over the conformation and orientation of the immobilized antibodies when used as recognition units results in loss or reduced sensitivity (Trilling et al., 2013). Thus, the development of OECT-based immunosensors with stable molecular recognition units under full orientation control are highly desirable, to create a stable and sensitive biosensor.

It is an object to provide OECT-based immunosensors for use in diagnostic assays.

It is also an object of the present invention to provide methods for making OECT-based immunosensor for use in diagnostic assays.

It is still an object of the present invention to provide methods for detecting analyte in a biological sample, using OECT-based immunosensors.

WO 2018/048742 A1 discloses methods for patterning highly sensitive materials, such as organic materials, organic semiconductors, biomolecular materials, with photolithographic resolution.

Macchia Eleonora, et al., 2017 7th IEEE International Workshop On Advances In Sensors And Interfaces (IWASI), IEEE, (2017), pages 68-72, discloses an organic electrochemical transistor immune-sensor operating at the femto-molar limit of detection.

US 2019/331673 A1 discloses a method of functionalization of a gate electrode of a field-effect transistor sensor which includes forming a layer of biological recognition elements on a surface of said gate electrode.

WO 2021/130525 A1, filed before and published after the priority date of the present application, discloses a transistor-based biological assay system.

## SUMMARY OF THE INVENTION

The invention provides a biosensor according to claim 1, a method of integrating a biorecognition layer on an electrode according to claim 9, and a method of detecting the absence, the presence, or the concentration of an analyte in a biological sample according to claim 11.

OECT-based biosensors are disclosed herein, as well as methods of making and methods of use thereof. The biosensor includes an OECT and a biorecognition layer. The biorecognition layer includes two self-assembled monolayers (SAMs the first of which is formed from organic molecules, chemically modified as disclosed herein and referred to herein as Chem-SAM, and the second of which is formed through a specific biological autocatalytic coupling strategy (herein, Bio-SAM), as disclosed herein. The Bio-SAM includes a biorecognition element preferably, a nanobody (for example, VHH) or related antibody fragment. The biorecognition element includes a binding partner to an analyte of interest, preferably, a pathogen or component thereof in one preferred embodiment, the biorecognition element can bind to the SARS-CoV-2 receptor binding domain (RBD) or Spike protein (S1). In a preferred embodiment, the organic molecules in the SAM include thiols and a synthetic peptide chemically coupled to it.

The organization of the biorecognition element on the OECT surface is represented by the general formula:

$$N\text{-}L_1\text{-}AP_1\text{-}AP_2\text{-}L_2\text{-}\mathbf{B}\text{-} \qquad \text{Formula I}$$

Where N is one or more organic molecules capable of self-assembly to form a first SAM, $L_1$ is an optional first linker, $AP_1$ is the first peptide binding partner; $AP_2$ is the second peptide partner; $AP_1$ and $AP_2$ are binding partners, preferably, covalent binding partners; however, they can be members of an affinity pair; L2 is a second linker and B is the biorecognition element. The first SAM formed by N is chemically modified with $AP_1$, resulting in Chem-SAM. Binding or coupling of $AP_1$ and $AP_2$ results in a biologically self-assembled monolayer, herein, Bio-SAM. Thus, the biorecognition layer includes Chem-SAM and Bio-SAM.

In one particular embodiment, $AP_1$ is spyTag and $AP_2$ is spyCatcher.

The biorecognition layer is preferably integrated on the gate electrode of the OECT.

A preferred conducting polymer for the channel is poly(3,4-ethylenedioxythiophene) doped with poly(styrene sulfonate) (PEDOT:PSS) or any other mixed (ionic and electronic) semiconductor. The OECT can operate in depletion or in accumulation mode.

Also disclosed are methods for making OECT devices containing a binding partner for any analyte of interest, for example an antigen from any pathogen. The method includes functionalizing the sensing gate electrode of the OECT device with a biorecognition layer which includes the binding partner for the analyte of interest, as follows: (i) contacting at least a portion of the surface of the electrode with a first solution containing a plurality of organic molecules capable of self-assembling, to produce a SAM-modified surface and (ii) contacting the SAM-modified surface with one or more solutions containing a linker or linker elements and the binding partner for the analyte. Referring to Formula I, the surface of the electrode is contacted with a solution containing organic molecules capable of self-assembling into a SAM for form a first self-assembling mononalayer on the surface of electrode, and contacting this first self-assembling mononalayer with a composition containing $AP_1$ under conditions that result in chemical coupling of $AP_1$ to the first SAM (optionally connected through the first linker $L_1$), thus forming a Chem-SAM; contacting the Chem-SAM with a composition containing $AP_2$-$L_2$-B- under conditions resulting in conjugation of $AP_1$ and $AP_2$ to form a second SAM, which is biologically self-assembled, and referred to herein as Bio-SAM. In one preferred embodiment, the $AP_1$ and $AP_2$ are the spyTag and spyCatcher, respectively. Autocatalytic covalent binding between the spyTag/spyCatcher pair orients the analyte binding partner on the surface of the OECT device. In a particularly preferred embodiment, the method includes contacting the SAM-modified surface with a first solution containing one partner of spyTag/spyCatcher pair followed by contact with a second solution containing the second partner of the spyTag/spyCatcher pair which is in turn linked to the binding partner for the analyte of interest. In one preferred embodiment, the analyte of interest is the SARS-CoV-2 RBD and its binding partner is a specific nanobody.

Methods for identifying an analyte of interest in a sample are disclosed. The method includes contacting the sample with OECT-based biosensor which includes a binding partner for the analyte of interest. Exemplary samples include bodily fluids such as saliva, plasma, serum and blood.

## BRIEF DESCRIPTION OF THE DRAWINGS

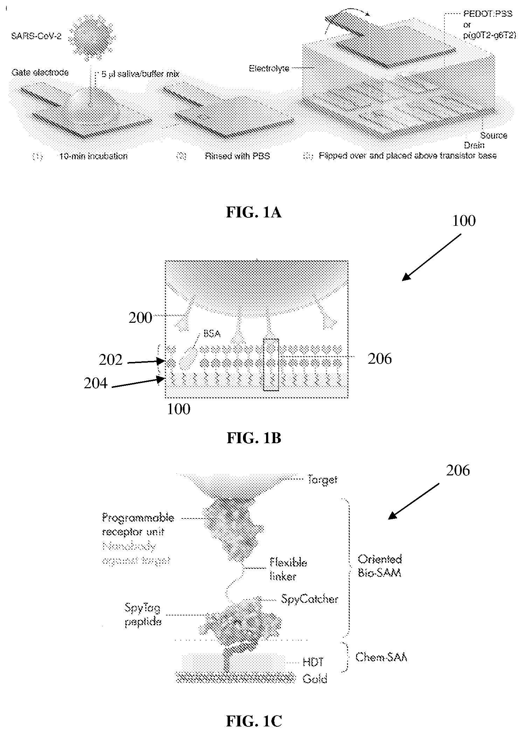

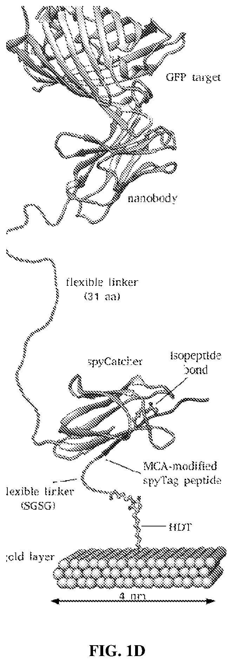

FIG. 1A-1C are schematic illustration of the nanobody-functionalized OECT sensor. FIG. 1A illustrates Operation of the disclosed device: The gate electrode is exposed to a mix of sample (such as saliva) and binding buffer (1), rinsed with PBS (2), and mounted on top of the OECT channel for signal acquisition (3). FIG. 1B is an illustration of gate functionalization layers of a section (100) of the gate electrode shown in step 2 of FIG. 1A. Chemical and biological monolayers (Chem-SAM (204) and Bio-SAM (204)) are self-assembled on the gate electrode surface, exemplifying the attachment of virus spike (200), to its binding partner on the Bio-SAM (204). FIG. 1C illustrates the molecular architecture of a section (206) of the gate functionalized layers shown in FIG. 1B. A synthetic SpyTag peptide is chemically coupled to the 1,6-hexanedithiol (HDT) monolayer to form a Chem-SAM (204). The nanobody-SpyCatcher fusion protein then attaches itself to this chemical layer through the autocatalytic formation of a covalent SpyCatcher-SpyTag bond, forming the Bio-SAM. The nanobody domain defines sensor specificity and is interchangeable. FIG. 1D shows a structural model of the complete biorecognition layer assembled from HDT and SpyTag (Chem-SAM) and nanobody-SpyCatcher fusion protein (Bio-SAM). Unstructured, flexible linker regions are indicated by dashed lines drawn to scale.

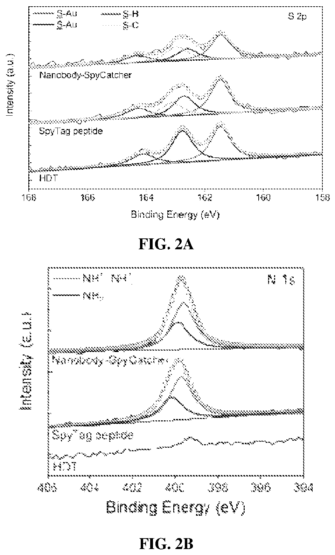

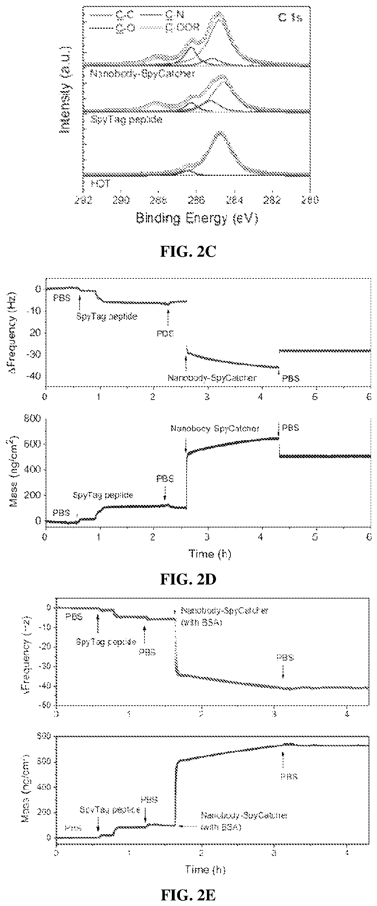

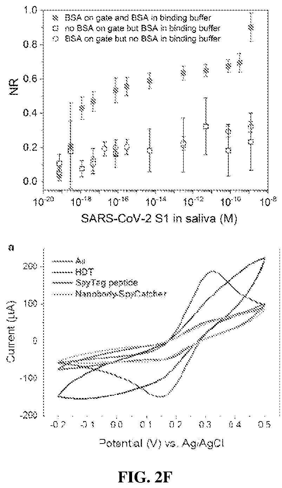

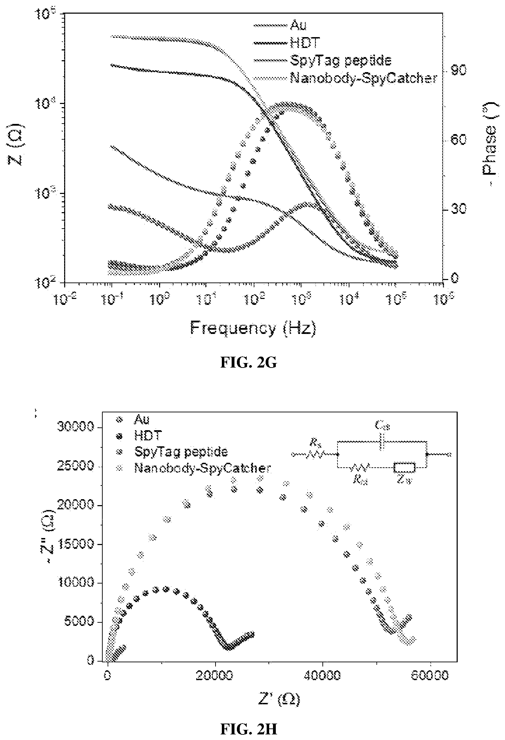

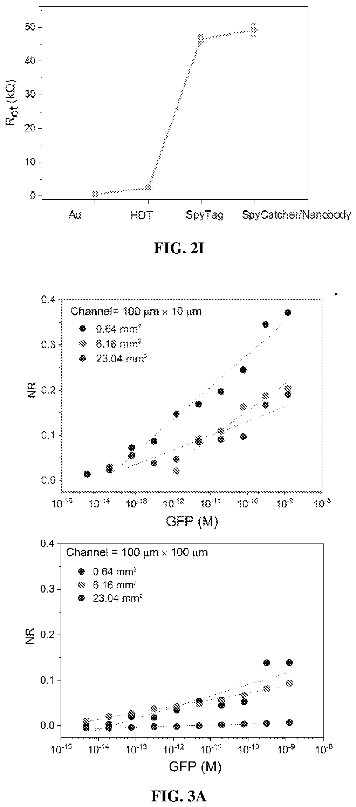

FIG. 2A is a high resolution S 2p XPS spectra of the gold electrode recorded after immobilization of HDT, SpyTag peptide, and the nanobody-SpyCatcher protein. The nanobody-SpyCatcher buffer contained BSA. FIG. 2B-2C show surface characterization of the Au electrode upon Chem-SAM and Bio-SAM immobilization. High-resolution of FIG. 2B) C 1s and FIG. 2C) N 1s XPS spectra of the gold electrode after HDT, SpyTag peptide, and the nanobody-SpyCatcher protein functionalization. The nanobody-SpyCatcher buffer contained BSA. FIG. 2D shows the QCM-D profile measuring the coupling of SpyTag peptide and nanobody-SpyCatcher fusion protein in real-time, starting from an HDT-coated gold electrode. FIG. 2E shows monitoring of the biofunctionalization of the gold surface using QCM-D. The top panel shows the change in QCM-D frequency of the sensor ($7^{th}$ overtone) over time as SpyTag peptide, PBS, nanobody-SpyCatcher and BSA and PBS was introduced to the system. The bottom panel presents the corresponding mass changes that the sensor undergoes during these steps. PBS was used to wash away the unbound molecules. FIGs. 2F-2I show electrochemical characterization of the biofunctionalized Au electrode. FIG. 2F (top panel) shows the effect of BSA on the performance of the OECT sensors in detecting SARS-CoV-2 S1 in raw saliva. The normalized response (NR) of OECT sensors prepared at various conditions: 1) the gate electrode functionalization solution did not contain BSA but BSA was added to the target binding buffer (hollow squares), 2) the gate electrode functionalization solution contained BSA but the binding buffer did not (hollow circles), and 3) BSA was added both to the functionalization solution and the binding buffer (solid squares)); Bottom panel is a cyclic voltammogram, FIG. 2G is a Bode plot (solid lines and dotted lines are corresponding to the magnitude and the phase of the impedance, respectively). FIG. 2H is a Nyquist plot of the gold electrode before and after the subsequent functionalization with HDT, SpyTag peptide, and the nanobody-SpyCatcher. Inset in FIG. 2H is the equivalent circuit model used to fit the impedance spectra. FIG. 2I is the calculated $R_{ct}$ and $C_{dl}$ changes of the Au electrode.

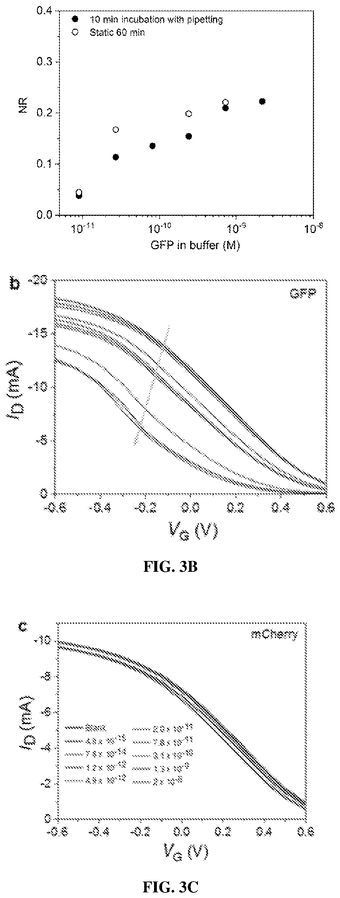

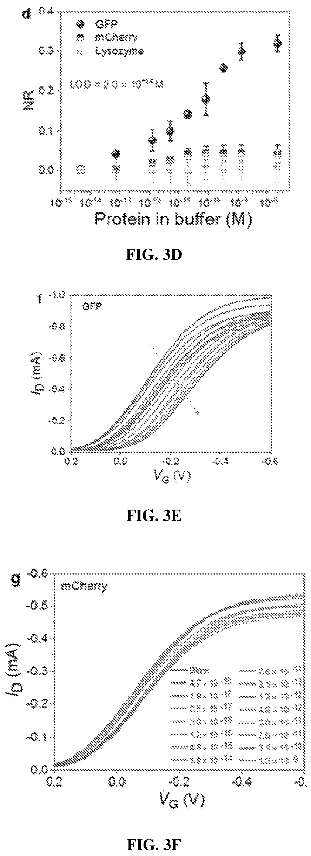

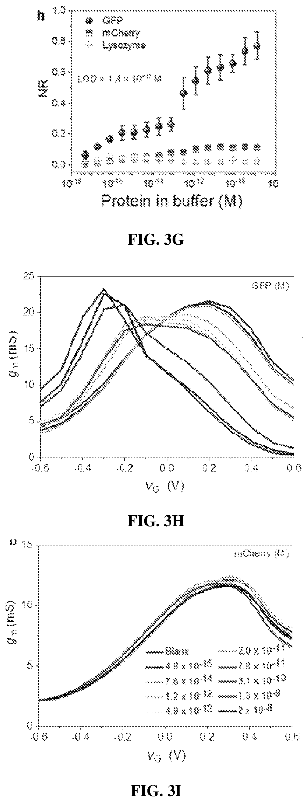

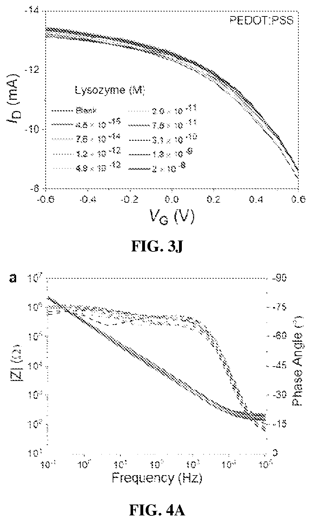

FIG. 3A shows the effect of channel and gate geometry on the performance of PEDOT:PSS OECT sensors. The GFP-functionalized Au electrodes with an area of 0.64 mm$^2$ (0.8 × 0.8 mm), 6.16 mm$^2$ (diameter = 2.8 mm), and 23.04 mm$^2$ (4.8 × 4.8 mm) are used to gate (top panel) channel with a width ($W$) 100 $\mu$m and length (L) 10 $\mu$m, and (bottom panel) channel with a $W$= 100 $\mu$m and L= 100 $\mu$m. FIGs. 3B-3G show performance of GFP nanobody-functionalized OECT biosensors. The steady-state characteristics of OECTs gated with a GFP nanobody-functionalized Au electrode exposed to FIG. 3B (bottom panel), FIG. 3E GFP, and FIG. 3C, FIG. 3F mCherry. FIG. 3H, 3G) The normalized response (NR) of the sensors to (GFP), mCherry, and lysozyme. In FIG. 3B (bottom panel - FIG. 3D, the channel is PEDOT:PSS, FIG. 3E-3G) the channel is p(g0T2-g6T2). Protein concentrations in the buffer range from 5 fM to 20 nM. In b and f, the decrease in $I_D$ (and the increase in protein content in the buffer) is indicated by the direction of the arrow. Error bars represent the standard deviation calculated using at least three gate electrodes. FIG. 3B, top panel shows the effect of of gate/target incubation time and protocol. GFP nanobodygates of an early (non-optimized) version of PEDOT:PSS OECT were exposed to increasing concentrations of GFP. For each protein concentration, the gate electrodes were incubated with the target solution for 10 min or for 60 min. During the 10 min, the solution was intermittently mixed by up and down pipetting on the electrode surface. The sensor performance was similar for the two incubation protocols. FIG. 3H-3I show transconductance vs gate voltage for the GFP nanobody-functionalized Au electrode gating a PEDOT:PSS channel. The protein in the incubation buffer was, FIG. 3H. GFP, and FIG. 3I, mCherry. The protein concentration ranges from 5 fM to 20 nM. The purple curve corresponds to the gate electrode incubated in the buffer only (blank). The $V_G$ that attains the max $g_m$ shifts from ca. 0.3 V to - 0.3 V with GFP binding. FIG. 3J shows sensor response to non-target proteins. Transfer characteristics of the GFP nanobody-functionalized Au electrode gating a PEDOT:PSS channel as the gate is exposed to various concentrations of lysozyme.

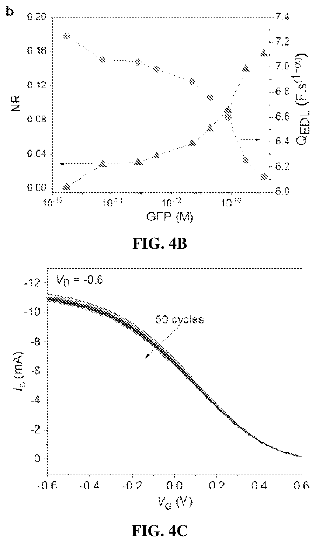

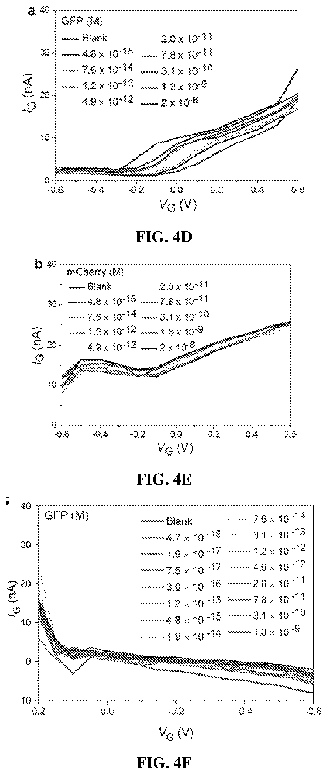

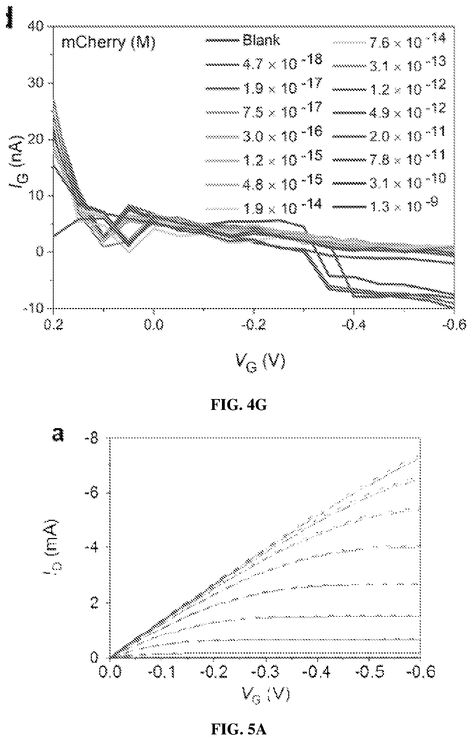

FIGs. 4A and 4B show electrochemical impedance spectra of the GFP nanobody-functionalized electrode upon GFP binding. **FIG. 4A** shows Bode plots of the electrode upon successive incubations in increasing GFP concentrations **FIG. 4B** shows the normalized change in in the corresponding constant phase element component obtained by equivalent circuit model analysis. **FIG. 4C** shows the cycling stability of a Au electrode gated OECT comprising a PEDOT:PSS channel. The decrease in the drain current is ~3% at $V_D = V_G$ = -0.6 V upon 50 I-V cycles. **FIGs. 4D** and **4E** show data on the gate currents of the GFP biosensors. The changes in the gate current ($I_G$) of the GFP nanobody-functionalized OECT when the gate is exposed to various concentrations of GFP or mCherry when the channel is PEDOT:PSS **(FIG. 4D** and **4E)** or p(g0T2-g6T2) **(FIG. 4F** and **4G).**

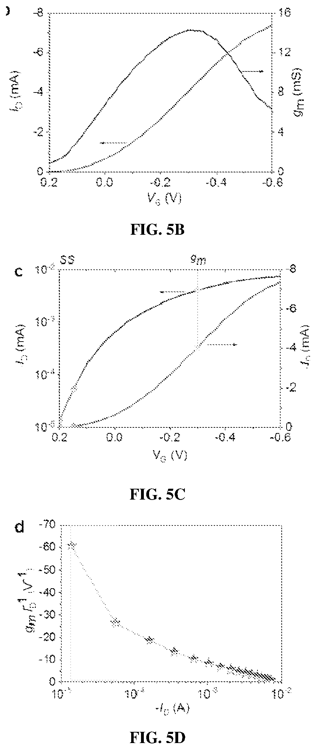

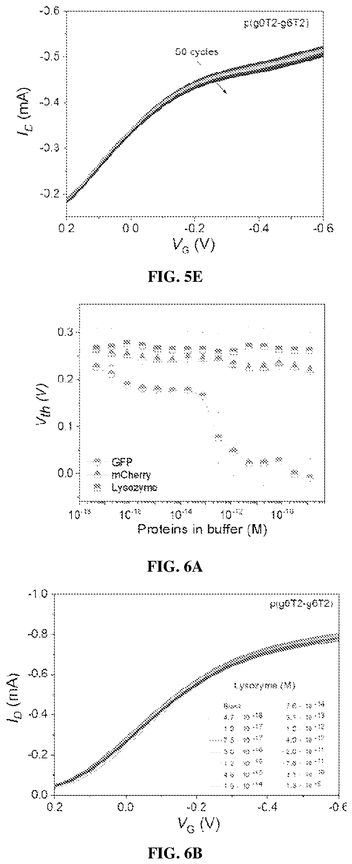

**FIG. 5A-5D** show steady-state characteristics of p(g0T2-g6T2) OECTs. **FIG. 5A,** Output curves ($I_D$ *vs.* $V_D$) and **FIG. 5B** shows the transfer curve (left axis) with the corresponding transconductance ($g_m$ *vs.* $V_G$, right axis). $V_D$ was -0.6 V. **FIG. 5C** shows transfer characteristics in the linear (right axis) and log scale (left axis). The operating points of peak transconductance ($g_m$, yellow stars) and peak subthreshold slope (SS, region highlighted in green) are marked. **FIG. 5D** shows transconductance efficiency ($g_m / I_D$) *vs.* $I_D$ for $V_D$=-0.6 V. **FIG. 5E** shows the cycling stability of a Au electrode gated OECT comprising a p(g0T2-g6T2) channel. The decrease in the drain current is -1% at $V_D = V_G$ = -0.1 V upon 50 I-V cycles.

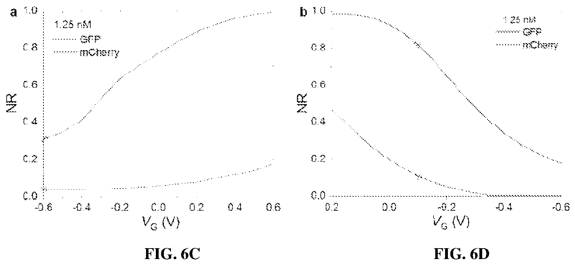

**FIG. 6A** shows the change in the threshold voltage ($V_{th}$) upon protein detection. The changes in $V_{th}$ of p(g0T2-g6T2) OECTs as the gate electrode is exposed to GFP, mCherry and Lysozyme at various concentrations. The $V_{th}$ shifts towards more negative values as more GFP molecules bind to the gate electrode. Error bars represent the standard deviation from at least three gate electrodes. **FIG. 6B** shows sensor response to non target proteins. Transfer characteristics of the GFP nanobody-functionalized Au electrode gated p(g0T2-g6T2) channel as the gate is exposed to various concentrations of lysozyme. **FIGs. 6C** and **6D** show the gate voltage dependence of sensor response. The normalized response curves as a function of $V_G$ for OECTs comprising PEDOT:PSS **(FIG. 6C)**p(g0T2-g6T2) (**FIG. 6D**). The gate electrode was incubated in 1.25 nM of protein solution. $V_D$ is -0.1 V and -0.6 V for p(g0T2-g6T2) and PEDOT:PSS, respectively. Although the NR is maximized at $V_G$= 0.6V for PEDOT:PSS, since the polymer is almost fully de-doped at this biasing condition (depletion mode), the sensor current response could not be well-resolved. NR at $V_G$=-0.6V was calculated.

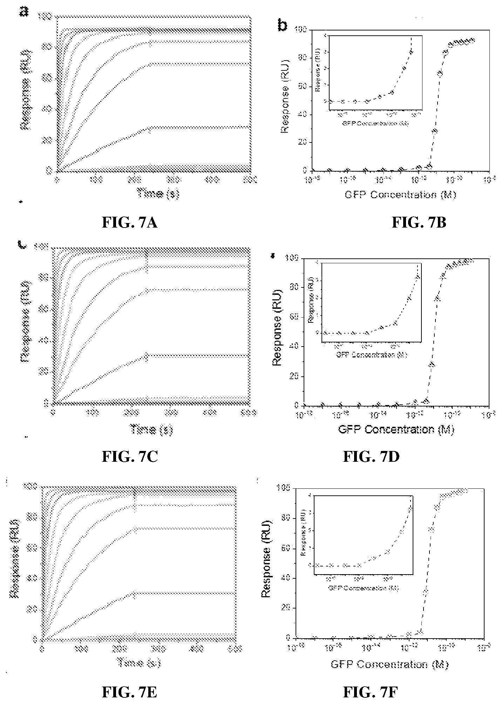

**FIG. 7A-7F** show SPR measurement of the GFP : nanobody interaction. His-tagged GFP nanobody was immobilized with a low loading level **(FIG 7A)** and, for comparison of SPR and OECT sensitivities, with a high loading level **(FIG, 7C, FIG, 7E)** on a Ni-NTA sensor chip and exposed to increasing concentrations of GFP. The kinetic off rate was too low to be quantified, indicating exceptional stability over time of the complex once formed. Analysis of steady-state binding levels **(FIG. 7B, FIG. 7D, FIG. 7F)** indicated first SPR signals for GFP concentrations as low as 1 pM ($10^{-12}$ M).

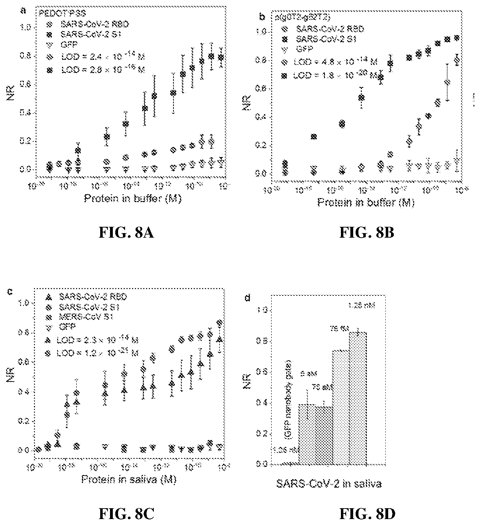

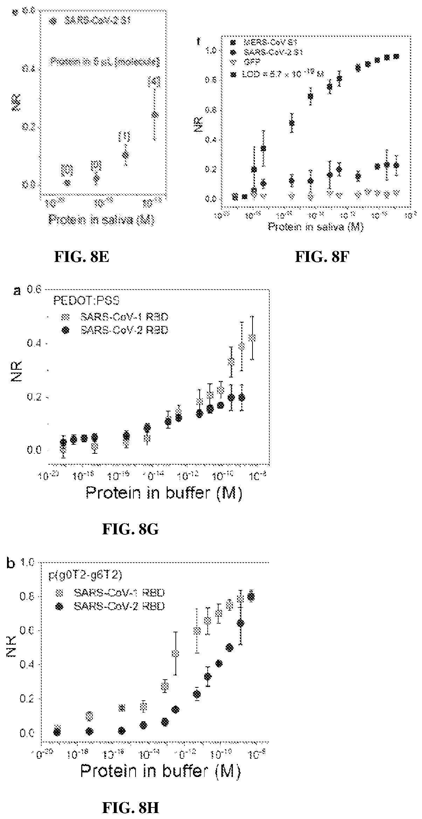

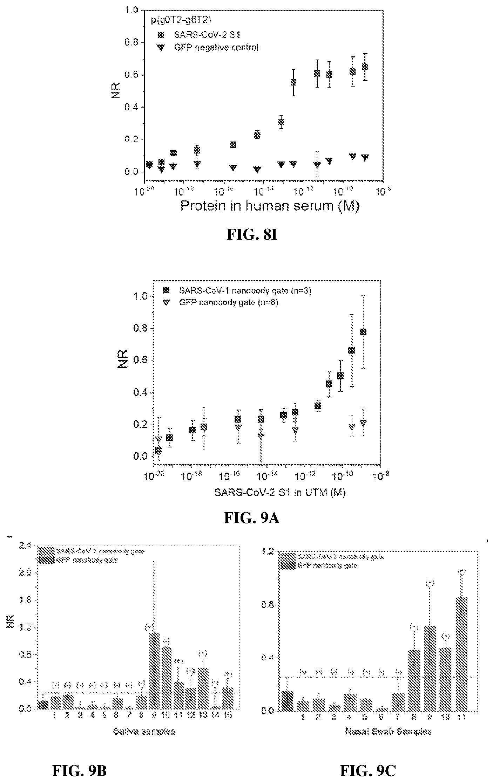

**FIGs. 8A-8F** show data on the performance of the SARS-CoV or MERS nanobody-functionalized OECTs in detecting SARS-CoV-2 or MERS-CoV. The normalized response (NR) of SARS-CoV nanobody-functionalized OECTs to SARS-CoV-2 RBD, spike protein (S1), and GFP for channels comprising **FIG. 8A)** PEDOT:PSS and **FIG. 8B)** p(g0T2-g6T2). **FIG. 8C)** The response of SARS-CoV nanobody-functionalized OECTs comprising p(g0T2-g6T2) channel to SARS-CoV-2 proteins, GFP and MERS-CoV-soike protein (S1 subunit) spiked in human saliva. **FIG.** 8D) The NR of SARS-CoV-2 sensors to randomly-selected saliva samples containing various amounts of SARS-CoV-2 S1. The blank measurements were obtained by incubating the gate electrodes in PBS. **FIG. 8E)** The NR of SARS-CoV-2 sensors at single molecule detection level. **FIG. 8F)** The response of MERS nanobody-functionalized OECTs comprising p(g0T2-g6T2) channel to MERS S1, SARS-CoV-2 S1 and GFP spiked in human saliva. In all measurements, error bars represent the standard deviation calculated from measurements with at least 3 gate electrodes. **FIGs. 8G-8H** show the SARS-CoV nanobody functionalized OECT response to SARS-CoV RBDs. Normalized response (NR) of the SARS-CoV-1 nanobody-functionalized electrodes gating **FIG. 8G** PEDOT:PSS and **FIG. 8H,** p(g0T2-g6T2) channel. The electrodes were exposed to various concentrations of SARS-CoV-1 RBD or SARS-CoV-2 RBD in a buffer. p(g0T2-g6T2) OECT has an LOD of $1.6 \times 10^{-17}$ M for SARS-CoV RBD compared to the PEDOT:PSS OECT with an LOD of $1.5 \times 10^{-15}$ M. **FIG. 8I** shows the OECT biosensor response to SARS-CoV-1 RBD in human serum. The NR of SARS-CoV-1 nanobody-functionalized electrode gating a p(g0T2-g6T2) channel to SARS-CoV-2 S1 and GFP spiked in human serum.

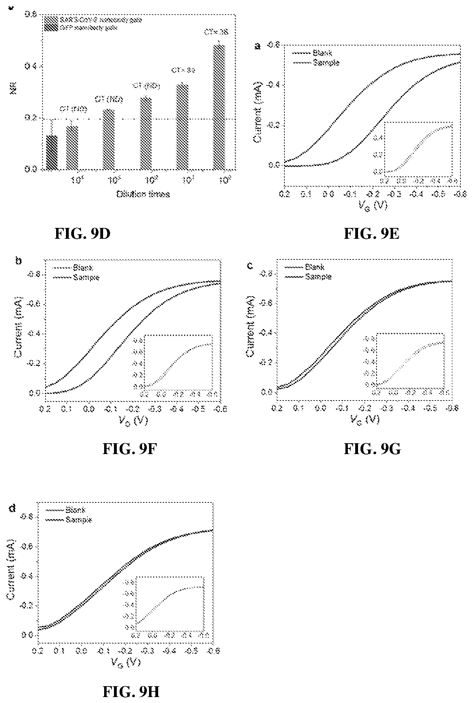

**FIG. 9A** shows the OECT biosensor response in universal transport media (UTM). The response of nanobody-functionalized OECTs comprising p(g0T2-g6T2) channel to SARS-CoV-2 S1 protein spiked in UTM. **FIGs. 9B-9C** show data on B-D OECT-based SARS-Cov-2 detection in clinical samples. TjesensorNR for FIG. 9B saliva samples (n=15) taken from healthy volunteers (1-7) and from hospitalized COVID-19 diagnosed patients (8-15). RT-PCR results for the same samples are indicated (+, positive) and (-, negative); **FIG. 9C** shows results from nasopharyngeal swab samples (n=11) from healthy volunteers (1-7) and from walk-in patients tested SARS-CoV-2 positive by RT-PCR (8-11). All RT-PCR results were CT > 30 indicating very low viral loads. The control experiments for the samples were performed by testing the specimens with GFP gate electrodes and the error bars represent the average results from at least 12 electrodes testing various specimens. The threshold line indicates mean current + standard deviations of signals collected from sensors with this control sensor exposed to the same samples. If the current change for any sample was higher than the threshold, the sample was considered as SARS-CoV-2 positive. FIG. 9D Sensor results from a 10-fold dilution series of nasal swab sample #8. RT-PCR results (Cycle threshold, CT) after RNA extraction from the same sample are given above each bar (ND= not detected). Error bars for the SARS-CoV-2 gates represent standard deviations determined from measurements with 2 gate electrodes and 3 channels for each sample. **FIG 9E-9H** show exemplary OECT biosensor response to patient samples. **FIG. 9E** Nasal swab sample 10, **FIG. 9F.** Saliva sample 13, **FIG. 9G.** Nasal swab sample 4, **FIG. 9H.** Saliva sample 4. The inset figures were obtained using the GFP nanobody gate. Nasal swab sample 10 and Saliva sample 13 were confirmed to be positive, while Nasal swab sample 4 and Saliva sample 4 were confirmed to be negative by RT-PCR.

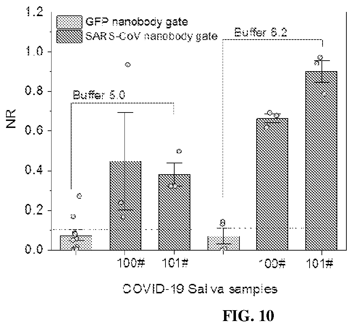

**FIG. 10** is a bar graph showing OECT normalized response (NR) measured from Covid-19 positive patient saliva samples. Two patient samples (#100, #102) were mixed 1:4 with binding buffer 5 or 6.2 and then incubated for 10 min on the SARS-CoV nanobody (VHH72) gate (red) or a control gate coated with GFP nanobody (negative control, blue). Values are the average NR from three gate electrodes independently exposed to the same sample. Error bars give standard error of the mean. The viral loads determined by RT-qPCR for the same samples are 2674 copies/mL (#100) and $1.8 \times 10^{11}$ copies/mL (101#).

## DETAILED DESCRIPTION OF THE INVENTION

Devices for sensitive and rapid analyte detection using an OECT Sensor are provided. The devices can be used for rapid detection of an infection by a pathogen such as a SARS-CoV-2 by detecting the presence of an antigen (analyte) specific for the pathogen, using a biofunctionalized-OECT Sensor, where the OECT Sensor is functionalized to include a binding partner for the antigen of interest.

The OECT biofunctionalized Sensor is characterized by: high sensitivity ($1.8 \times 10^{-17}$ M of target molecule, for example SARS-CoV-2-Spike protein, causes 5% change of readout signals) and selectivity (1nM of other species in the saliva contributes to only 0.5% the sensor readout), delivering accurate results in accordance with gold standard tests (correlated to RNA detection results with the conventional RT-PCR); fast detection time (around 15 minutes after the sample exposure to the sensor surface to the read-out); and low limit of detection (limit of detection ($2.8 \times 10^{-16}$ M or $1.8 \times 10^{-20}$ M of target molecule depending on the channel material, for example SARS-CoV-2-Spike protein, in buffer solution). Additionally, the OECT biofunctionalized Sensor gate electrode is disposable and easy to fabricate, with a path to reliable mass production.

The devices can be used with minute amount of solution (as little as 5 μl) from easily accessible samples including saliva (but also blood from a finger prick); they are accessible and affordable, allowing large-scale application; easy-to-use by minimally trained users or even by the patients themselves; can be operated with an inexpensive, portable, and hand-held readout unit (for example connected to a smartphone); can be designed to detect different targets using the same platform by modular exchange of the biological detection unit.

The device can have multiple channels in an area underneath the gate electrode, meaning that one sensing gate electrode can be used to get multiple readouts simultaneously, improving the sensor accuracy. These channels can be made of different organic materials.

## I. DEFINITIONS

"Affinity interactions" as used herein refers to the combination of non-covalent interactions between a ligand and its binding partner to form a complex.

"Affinity tags" as used herein are peptide sequences appended to proteins so that they can be purified from a crude biological source using an affinity technique.

"Covalent linkage", refers to a bond or organic moiety that covalently links molecules (e.g. fusion proteins) to a non-cellular surface.

As used herein, the term "polypeptide" refers to a chain of amino acids of any length, regardless of modification (e.g., phosphorylation or glycosylation).

As used herein, a "vector" is a replicon, such as a plasmid, phage, or cosmid, into which another DNA segment may be inserted so as to bring about the replication of the inserted segment. The vectors described herein can be expression vectors.

As used herein, an "expression vector" is a vector that includes one or more expression control sequences

As used herein, an "expression control sequence" is a DNA sequence that controls and regulates the transcription and/or translation of another DNA sequence.

As used herein, "operably linked" means incorporated into a genetic construct so that expression control sequences effectively control expression of a coding sequence of interest.

As used herein, "conservative" amino acid substitutions are substitutions wherein the substituted amino acid has similar structural or chemical properties.

As used herein, "non-conservative" amino acid substitutions are those in which the charge, hydrophobicity, or bulk of the substituted amino acid is significantly altered.

As used herein, the term "host cell" refers to prokaryotic and eukaryotic cells into which a recombinant expression vector can be introduced.

As used herein, "transformed" and "transfected" encompass the introduction of a nucleic acid (e.g., a vector) into a cell by a number of techniques known in the art.

## II. COMPOSITIONS AND DEVICES

The disclosed devices include an OECT engineered to include a biorecognition layer, including a biorecognition element. The biorecognition element is the component that can specifically interact with its cognate target. The OECT is an electrolyte gated transistor, that is, a three-terminal electronic device which includes a source electrode, a drain electrode, a channel, and a gate electrode. In some preferred embodiments, the biorecognition layer is integrated on the gate electrode of the OECT.

## A. Biorecognition layer

The biorecognition layer is designed to provide a stable complex, preferably, an immunocomplex between a biorecognition element in the biorecognition layer, and its binding partner, which is the analyte/antigen of interest. The biorecognition element is preferably, not a multidomain antibody. The biorecognition layer is preferably integrated on the surface of the gate electrode of the OECT.

The biorecognition layer includes two self-assembled monolayers (SAMs the first of which is formed from organic molecules, chemically modified as disclosed herein and referred to herein as Chem-SAM, and the second of which is formed through a specific biological autocatalytic coupling strategy (herein, Bio-SAM), as disclosed herein. The Bio-SAM includes biorecognition element preferably, a nanobody **(FIGs. 1B and 1C).** The organization of the biorecognition element on the OECT surface can be represented by the general formula:

$$N\text{-}L_1\text{-}AP_1\text{-}AP_2\text{-}L_2\text{-}B\text{-} \qquad \text{Formula } \mathbf{I}$$

Where **N** is one or more organic molecules capable of self-assembly to form a first SAM, $L_1$ is an optional first linker, $AP_1$ is the first peptide binding partner; $AP_2$ is the second peptide partner; $AP_1$ and $AP_2$ are binding partners, preferably, covalent binding partners; however, they can be members of an affinity pair; L2 is a second linker and **B** is the biorecognition element. The first SAM formed by N is chemically modified with $AP_1$, resulting in Chem-SAM. Binding of $AP_1$ and $AP_2$ results in a biologically self-assembled monolayer, herein, Bio-SAM. Thus the biorecognition layer includes Chem-SAM and Bio-SAM.

$L_1$ and $L_2$ are preferably peptide linkers sequences which are at least 2 amino acids in length. Preferably the peptide or polypeptide domains are flexible peptides or polypeptides. A "flexible linker" herein refers to a peptide or polypeptide containing two or more amino acid residues joined by peptide bond(s) that provides increased rotational freedom for two polypeptides linked thereby than the two linked polypeptides would have in the absence of the flexible linker. Exemplary flexible peptides/polypeptides include, but are not limited to, the amino acid sequences Gly-Ser, Gly-Ser-Gly-Ser (SEQ ID NO:9), Ala-Ser, Gly-Gly-Gly-Ser (SEQ ID NO:10), $(Gly_4\text{-}Ser)_3$ (SEQ ID NO:11), and $(Gly_4\text{-}Ser)_4$ (SEQ ID NO:12), GSGSGSGS (SEQ ID NO:13). Additional flexible peptide/polypeptide sequences are well known in the art. In one embodiment, $L_1$ is SGSG (SEQ ID NO:14).

The nanobody can be a naturally-derived from immunization of an animal or synthetically-derived (i.e., a sybody) or a combination thereof. Sybodies are disclosed for example, in Walter, et al., doi: https://doi.org/10.1101/2020.04.16.045419. In some preferred embodiments, the organic molecules forming the SAM include thiols. In some preferred embodiments, the linker is a biomolecular linker formed by the bioconjugation of two peptides, such as a SpyTag/SpyCatcher bioconjugation (Zakeri, et al., Proc. Natl. Acad. Sci., 109:E690-E697 (2012)).

In a particularly preferred embodiment, the biorecognition layer includes a SAM formed from an alkanedithiol (e.g. 1,6-hexanedithiol) and a SpyTag/SpyCatcher bioconjugation as the linker, which allows for controllable orientation of the nanobody functionalization, which also maximizes the capture density of nanobodies within a small sensor area (0.64 $mm^2$) and enhances the sensitivity of the OECT sensor. For example, at least 30 and up to $45 \times 10^{12}$ SpyTag peptides (such as about $39 \times 10^{12}$ SpyTag peptides) and at least 8 and up to $10 \times 10^{12}$ nanobody-SpyCatcher molecules (such as about $8.6 \times 10^{12}$ nanobody-SpyCatcher molecules) are coupled per $cm^2$ on the surface of the gate electrode of the OECT.

## i. Chem-SAM

The first-SAM on the OECT gate electrode is typically formed by reacting a plurality of organic molecules on the gate electrode surface of the OECT under conditions resulting in self-assembly, following which it is chemically functionalized to form Chem-SAM, by contacting with a binding partner required for formation of the second SAM, i.e., the Bio-SAM. In some preferred embodiments, the organic molecules in the first SAM include thiols or thiol derivatives. Thiols that are suitable to form the first SAM on the surface of the gate electrode of the OECT may be a monothiol, a dithiol, a trithiol, or a mixture thereof.

Typically, the thiols in the Chem-SAM on gate electrode surface of the OECT have a carbon chain containing up to 20 carbons, up to 18 carbons, up to 16 carbons, up to 15 carbons, up to 12 carbons, up to 10 carbons, up to 8 carbons, up to 6 carbons, at least 2 carbons, at least 3 carbons, in a range from 1 carbon to 20 carbons, from 2 carbons to 20 carbons, from 2 carbons to 18 carbons, from 2 carbons to 15 carbons, from 2 carbons to 12 carbons, from 2 carbons to 10 carbons, from 2 carbons to 8 carbons, or from 2 carbons to 6 carbons.

Exemplary thiols that can form the first SAM on the surface of the gate electrode of the OECT include, but are not limited to, alkane monothiols (e.g. methanethiol, ethanethiol, 2-propanethiol, butanethiol, pentanethiol, ter-butyl mercaptan, 1-hexanethiol, 1-octanethiol, 1-nonanethiol, 1-decanethiol, 1-undecanethiol, 1 dodecanethiol, 1-tridecanethiol, 1-tetradecanethiol, 1-pentadecanethiol, 1-hexadecanethiol, 1-octadecanethiol, 1-nonadecanethiol, and 1-icosanethiol, etc.), alkane dithiols (e.g. 1,2-ethanedithiol, 1,4-butanedithiol, 1,3-butanedithiol, 1,5-pentanethiol, 1,6-hexanedithiol (HDT), 1,7-octanedithiol, 1,8-nonanedithiol, 1,3-propanedithiol (PDT), etc.), 3-mercaptopropionic acid (MPA), thiophenol, dimercaptosuccinic acid, glutathione, cysteine, 2-mercaptoethanol, lipoic acid, and 1,4-benzenedimethanethiol. Additional exemplary thiols that can form a SAM on the surface of the gate electrode of the OECT are disclosed in Love, et al., Chem. Rev., 105:1103-1169 (2005).

The first SAM on the gate electrode surface of the OECT may be formed from a single type of thiol or a mixture of different types of thiols. For example, the first SAM on the surface of the gate electrode of the OECT is formed from a dithiol (e.g. HDT or PDT), a mixture of different types of dithiols (e.g. a mixture of HDT and PDT), or a mixture of a dithiol and a monothiol (e.g. a mixture of PDT and MPA).

In some preferred embodiments, the first SAM on the gate electrode surface of the OECT is formed from a dithiol (e.g. HDT) or a mixture of different types of dithiols. A first SAM formed from a dithiol (i.e. HS-R-SH) on the gate electrode surface can contain two types of sulfur species: a thiolate-type sulfur (i.e. the sulfur bound to a surface atom, such as Au-SR) and a tail thiol sulfur (i.e. free thiol, R-SH). The partially bound dithiol molecules are in a standing up configuration (formed as a result of the method of making the first SAM, exemplified in the examples below) with an upright molecular structure, where the dithiol molecules are bound to the gate electrode surface via the thiolate linkage from one of the thiol groups, and the other thiol group is free (i.e. unbounded) interfacing air or solution. When all thiol groups of the dithiol molecules are converted to thiolates linked to the gate electrode surface, the dithiol molecules are in a laying down configuration. In some preferred embodiments, the first SAM on the gate electrode surface of the OECT is formed from a dithiol, where the dithiol molecules are in the standing up configuration upon formation of the first SAM. The standing up configuration of the dithiol molecules in the first SAM allows for uniform orientation of the linker and the biorecognition elements such that the biorecognition events can be more precise and compact, which also maximizes the capture capability of the biorecognition elements within a small sensor area (such as a gate surface area of 0.64 mm$^2$) and enhances the sensitivity of the OECT sensor.

In a particularly preferred embodiment, the first SAM on the gate electrode surface of the OECT is formed from HDT where the HDT molecules are in a standing up configuration upon formation of the SAM.

Referring to Formula I:

$$N-L_1-AP_1-AP_2-L_2-B- \qquad \text{Formula I}$$

Chem-SAM is formed by $N-L_1-AP_1$, Where N is one or organic molecules capable of self-assembly and is self-assembled on the surface of the OECT gate electrode, $L_1$ is an optional first linker, $AP_1$ is the first peptide binding partner; $AP_2$ is the second peptide partner; $AP_1$ and $AP_2$ are binding partners, preferably, covalent binding partners; however, they can be members of an affinity pair; L2 is a second linker and B is the biorecognition element.

The first SAM is preferably directly conjugated to $AP_1$, preferably provided by a suitable conjugation reaction such functionalization with maleimide.

ii. **Bio-SAM**

The biorecognition layer integrated on the gate electrode of the OECT includes a second SAM, which is a biologically self-assembled monolayer formed as a result of covalent binding of two binding partners or affinity interactions of an affinity pair, thereby forming a Bio-SAM, which includes the biorecognition element, which can be a non-antibody protein or an antibody, and is preferably a non-antibody protein. In one preferred embodiment, the biorecognition element is a fusion protein as described herein.

Referring to Formula I:

$$N\text{-}L_1\text{-}AP_1\text{-}AP_2\text{-}L_2\text{-}B\text{-} \qquad \text{Formula I}$$

Bio-SAM is formed by interaction of the first peptide binding partner $AP_1$ with the second peptide partner, $AP_2$, resulting in the formation of a second SAM, the Bio-SAM, which includes the biorecognition element B, exposed for interaction with its binding partner in a sample with which it is contacted.

$AP_1$ or $AP_2$ in a particularly preferred embodiment include the spyTag/spyCatcher pair, with $AP_1$ is preferably, spyTag in some preferred embodiments, for example, AHIVMV**D**AYKPTK (SEQ ID NO: 6), i.e., spyTag, and in preferred embodiments the spyTag consists of SEQ ID NO: 6; VPTIVMVDAYKRYK (SEQ ID NO:7) i.e., spytag002 (Keeble, et al., Angew. Chem. Int. Ed. Engl. 2017, 56, 16521-16525) or RG VPH IVMVDAYK RYK (SEQ ID NO:8), i.e., spyTag 0003 (Keeble, et al: Proc Natl Acad Sci U S A. 2019 Dec 26; 116(52): 26523-26533).

A preferred SpyTag/SpyCatcher bioconjugation system is disclosed in Zakeri, et al., Proc. Natl. Acad. Sci., 109:E690-E697 (2012). It is based on a modified domain from a Streptococcus pyogenes surface protein (SpyCatcher), which recognizes a cognate 13-amino-acid peptide (SpyTag). Upon recognition, the two form a covalent isopeptide bond between the side chains of a lysine in SpyCatcher and an aspartate in SpyTag. The SpyTag/SpyCatcher bioconjugation is a robust method for conjugating recombinant proteins where the peptide SpyTag can spontaneously react with the protein SpyCatcher in a facile manner and with high specificity (Zakeri, et al., Proc. Natl. Acad. Sci., 109:E690-E697 (2012); Keeble, et al., Angew. Chem. Int. Ed. Engl. 2017, 56, 16521-16525. The peptide SpyTag (13 amino acids) spontaneously reacts with the protein SpyCatcher (12.3 kDa) to form an intermolecular isopeptide bond between the pair. In some preferred embodiments, the SpyTag peptide is functionalized with a maleimide functional group and the SpyCatcher is linked to the biorecognition elements (e.g. nanobodies), such that upon bioconjugation between the SpyTag and the SpyCatcher, the biorecognition elements are integrated on the gate electrode surface in a uniform orientation. In optional embodiments, the SpyTag may include a linker.

Other binding partners can be used as $AP_1/AP_2$, for example, snoopTag peptide, (Veggiani, et al., PNAS 2316 113 (5) 1202-1207) MoonTag / MoonCatcher (homologue of SpyCatcher); the snapTag labelling system; the Sortase reaction which is connecting two shorter peptides; Coating the surface with Streptavidin and then coupling a site-specifically biotinylated nanobody can also be employed. SnoopTag, is a peptide which binds covalently to SnoopCatcher protein (KLGDIEFIKVNK) (SEWQ ID NO: 18). A second generation, SnoopTagJr, has been developed to bind to either SnoopCatcher or DogTag (mediated by SnoopLigase) (KLGSIEFIKVNK) (SEQ ID NO: 19). DogTag is, a peptide which covalently binds to SnoopTagJr, mediated by Snoopligase (DIPATYEFTDGKHYTTNEPIPPK) (SEQ ID NO: 20); SdyTag is, a peptide which binds covalently to SdyCatcher protein (DPIVMIDNDKPIT) (SEQ ID NO:21) SdyTag/SdyCatcher has a kinetic-dependent cross-reactivity with SpyTag/SpyCatcher. These systems are known in the art (reviewed Hatlem, et al., Int J Mol Sci. 2019 May; 20(9): 2129)). NAP- and CLIP-tag protein labeling systems enable the specific, covalent attachment of virtually any molecule to a protein of interest. There are two steps to using this system: cloning and expression of the protein of interest as a SNAP-tag® fusion, and labeling of the fusion with the SNAP-tag substrate of choice. The SNAP-tag is a small protein based on human $O^6$-alkylguanine-DNA-alkyltransferase (hAGT), a DNA repair protein.

SNAP-tag substrates are dyes, fluorophores, biotin, or beads conjugated to guanine or chloropyrimidine leaving groups via a benzyl linker. In the labeling reaction, the substituted benzyl group of the substrate is covalently attached to the SNAP-tag. CLIP-tag™ is a modified version of SNAP-tag, engineered to react with benzylcytosine rather than benzylguanine derivatives. When used in conjunction with SNAP-tag, CLIP-tag enables the orthogonal and complementary labeling of two proteins simultaneously in the same cells.

*a. Non-antibody protein biorecognition element*

Preferred biorecognition elements for incorporation into the disclosed devices are not whole antibodies, but more compact recognition domains such as a nanobody or a sybody. As used herein, non-antibody protein refers to a protein that is not a whole antibody which is a multidomain protein. By contrast, a nanobody, also known as a single domain antibody, is an antibody fragment consisting of a single monomeric variable antibody domain. Like a whole antibody, it is able to bind selectively to a specific antigen. With a molecular weight of only 12-15 kDa, single-domain antibodies are much smaller than common antibodies (150-160 kDa) which are composed of two heavy protein chains and two light chains, and are also smaller than $F_{ab}$ fragments (~50 kDa, one light chain and half a heavy chain) or single-chain variable fragments (scFv, ~25 kDa fusion of two variable domains, one from a light and one from a heavy chain).

Camelids naturally produce one class of antibodies composed only of heavy chains in which the target recognition module is composed of a single variable domain (VHH or Nb). (De Meyer, et al., 2014; Kubala, et al., 2010). The nanobody offers unique properties, simpler structural conformation and high stability in a range of different conditions (De Meyer, et al., 2014).

In one preferred embodiment, the devices include a nanobody, which binds to an antigen from the SAR-CoV-2, such as the spike protein. Specific nanobodies against SARS-CoV-2 Spike protein (S protein) or, more specifically, against the Receptor Binding Domain (RBD) within the S1 subunit of the protein are available (Wrapp, et al., Science 2020, 367 (6483), 1260-1263). Sybodies are disclosed for example, in Walter, et al., doi: https://doi.org/10.1101/2020.04.16.045419. Examples include the SARS 1/2 nanobody (VHH72), shown below:

QVQLQESGGGLVQAGGSLRLSCAASGRTFSEYAMGWFRQAPGKERE
FVATISWSGGSTYYTDSVKGRFTISRDNAKNTVYLQMNSLKPDDTA
VYYCAAAGLGTVVSEWDYDYDYWGQGTQVTVSSGS (SEQ ID NO: 17).

Suitable biorecognition elements can also include other antibody fragments and variants and fusion proteins thereof (e.g., and synthetic proteins containing at least the antigen binding variable domain of an antibody). For example, an antigen binding fragment of an antibody can be used as the biorecognition element. In some embodiments, the antigen binding fragment binds to a SARS-CoV and/or MERS-CoV antigen, such as the spike protein or nucleocapsid. In some embodiments, the antigen binding fragment binds to a SARS-CoV-2 antigen, such as the S1 (e.g., the receptor-binding domain (RBD)) or S2 domains of the spike protein. Exemplary antibody fragments and variants and fusion proteins thereof include, without limitation, Fab, Fab', F(ab')2, Fv, Fc, scFv, di-scFv, tri-scFv, scFv-Fc, Fab-Fc, scFv-zipper, scFab, and crossFab.

*b. Other Proteins*

In some embodiments, the devices can include an antibody, as the biorecognition element for example. In some embodiments, the antibody binds to a SARS-CoV and/or MERS-CoV antigen, such as the spike protein or nucleocapsid. In some embodiments, the antibody binds to a SARS-CoV-2 antigen, such as the S1 (e.g., the receptor-binding domain (RBD)) or S2 domains of the spike protein.

Antibodies that can be used include whole immunoglobulin (i.e., an intact antibody, such as IgA, IgD, IgE, IgG, and IgM, and including subclasses or isotypes, e.g., IgG1, IgG2, IgG3, IgG4, IgA1 and IgA2). Suitable antibodies encompass, but are not limited to, monoclonal antibodies, polyclonal antibodies, chimeric antibodies, bispecific antibodies, multispecific antibodies, heteroconjugate antibodies, mutants thereof, humanized antibodies, human antibodies, and any other modified configuration of the immunoglobulin molecule that includes an antigen recognition site of the required specificity,

In some embodiments, the antibody can be a humanized antibody. Methods for humanizing non-human antibodies are well known in the art. Generally, a humanized antibody has one or more amino acid residues introduced into it from a source that is non-human. These non-human amino acid residues are often referred to as "import" residues, which are typically taken from an "import" variable domain. Antibody humanization techniques generally involve the use of recombinant DNA technology to manipulate the DNA sequence encoding one or more polypeptide chains of an antibody molecule.

Native antibodies, and some other antigen binding proteins including some non-antibody antibody fragments and fusion proteins, can be heterotetrameric glycoproteins having two identical light chains and two identical heavy chains. Other antigen binding proteins including some non-antibody antibody fragments and fusions proteins may have few or more domains. Each heavy chain has at one end a variable domain ("VH") followed by a number of constant domains ("CH"). Each light chain has a variable domain at one end ("VL") and a constant domain ("CL") at its other end. The variable domains differ in sequence among antibodies and are used in the binding and specificity of each particular antibody for its particular antigen. However, the variability is not usually evenly distributed through the variable domains of antibodies. It is typically concentrated in three segments called complementarity determining regions (CDRs) or hypervariable regions both in the light chain and the heavy chain variable domains. The more highly conserved portions of the variable domains are called the framework (FR). The variable domains of native heavy and light chains each contain four FR regions, largely adopting a beta-sheet configuration, connected by three CDRs, which form loops connecting, and in some cases forming part of, the beta-sheet structure. The CDRs in each chain are held together in close proximity by the FR regions and, with the CDRs from the other chain, contribute to the formation of the antigen binding site of antibodies. Therefore, the antibodies typically contain at least the CDRs necessary to recognize and/or bind a target antigen (e.g., a SARS-CoV-2 antigen). Non-antibody antigen binding proteins such as antibody fragments and fusion proteins may also include at least the CDRs necessary to recognize and/or bind a target antigen (e.g., a SARS-CoV-2 antigen).

In some embodiments, the antibodies or non-antibody antigen binding proteins such as antibody fragments and fusion proteins may include one of each of a heavy chain CDR1, CDR2, and CDR3 in combination with one of each of a light chain CDR1, CDR2, and CDR3.

Anti-SARS-CoV-2 antibodies (e.g., used for detection and/or neutralization) are known in the art and are suitable for use herein. For example, suitable antibodies include commercially available antibodies such as MAB105801, MAB105802, MAB108271, LMAB10869, MAB105406, MAB105806, MAB105807, and MAB105808 from R&D Systems; and MA1-7403, MA5-35943, MA5-36271, MA5-35945, MA1-41093, and MA1-41173 from ThermoFisher Scientific.

Anti-SARS-CoV-2 antibodies are also described in U.S. Patent Nos. 10,975,139 and 11,021,532. Any antibody or antibody fragment disclosed in U.S. Patent Nos. 10,975,139 and 11,021,532 can be used.

In some embodiments, a suitable antibody or non-antibody antigen binding protein such as an antibody fragment or fusion protein includes a VH having an amino acid sequence of SEQ ID NO:22 or a sequence that is at least 80%, 85%, 90%, 95%, 98%, 99%, or 100% identical to SEQ ID NO:22, and a VL having an amino acid sequence of SEQ ID NO:23 or a sequence that is at least 80%, 85%, 90%, 95%, 98%, 99%, or 100% identical to SEQ ID NO:23.

QVQLVQSGAEVKKPGASVKVSCKASG<u>YTFTNYGIS</u>WVRQAP

GQGLEWM<u>GWISARNGNTNYA</u>QKFQGRVTMTRDTSTSTVYMELSSL

RSEDTAVYY<u>CTTDPVLEWFGYSIW</u>GQGTMVTVSS (SEQ ID NO:22;

CDR1-3 are the underlined sequences, respectively, from left to right).

DIVMTQSPDSLAVSLGERATINC<u>KSSQSVFSSSNNKNYLAWY</u> QQKPGQPPKLLIY<u>WAS-TRES</u>GVPDRFSGSGSGTDFTLTISSLQAEDVA VYYC<u>QQYYSTPLTF</u>GGZTKVEIKR (SEQ ID NO:23; CDR1-3 are the underlined sequences, respectively, from left to right).

In some embodiments, a suitable antibody or non-antibody antigen binding protein such as an antibody fragment or fusion protein includes one or more of the CDRs of SEQ ID NO:22 or SEQ ID NO:23. For example, a suitable antibody can include CDR1, CDR2, and/or CDR3 of SEQ ID NO:22, CDR1, CDR2, and/or CDR3 of SEQ ID NO:23, or any combination thereof.

c. *Fusion Proteins*

In some embodiments, the biorecognition elements are expressed as fusion proteins that contain a first polypeptide domain, a linker domain and a purification tag. Biorecognition elements such as nanobodies, sybodies, antibody fragments, antibodies, etc. can readily be expressed in various formats by fusion to other proteins, peptides or effector domains, thereby tailoring their utility. Taking advantage of this feature, the disclosed biorecognition element are expressed as fusion proteins, which include a linker and preferably, a protein purification tag; and can be represented by the following general formula II:

$$AP_1/AP_2\text{-}L_2\text{-}\textbf{B-C-P}T \qquad \text{Formula II,}$$

Where $AP_1/AP_2$ and $L_2$ are as defined above for formula I, and PT is a purification tag (an amino acid sequence that allows the polypeptides to be captured onto an affinity matrix) and C is an optional cleavage site, for example, the 3C cleavage site. In one embodiment, the biorecognition element B is oriented first in the fusion protein sequence (i.e., N-terminal). However, the biorecognition element B can be at the C-terminal end of the fusion protein. For example, a tag such as c-myc, hemagglutinin, polyhistidine, or Flag™ (Kodak) can be used to aid polypeptide purification. Such tags can be inserted anywhere within the polypeptide, including at either the carboxyl or amino terminus. The polyhistidine affinity tag, also known as the His-tag or $His_6$, usually consists of six consecutive histidine residues, but can vary in length from two to ten histidine residues; glutathione S-transferase (GST); Maltose binding protein (MBP), calmodulin binding peptide (CBP); the intein-chitin binding domain (intein-CBD), the streptavidin tag, etc.

One preferred fusion protein is MTGQVQLQESGGGLVQAGGSLRLSCAASGRTFSEYAMGWFRQAPG KER-EFVATISWSGGSTYYTDSVKGRFTISRDNAKNTVYLQMNSLKPD DTAVYYCAAAGLGTVVSEWDYDY-DYWGQGTQVTVSSGS<u>GSGSGS</u> GSVDTLSGLSSEQGQSGDMTIEEDSATHIKFSKRDEDGKELAGATME LRDSSGKTISTWISDGQVKDFYLYPGKYTFVETAAPDGYEVATAITFT VNEQGQVTVNGKATKGDAHISG<u>LE-VLFQGPTG</u>**HHHHHHHH** (SEQ ID NO: 15) where the underlined residues constitute a linker (8 amino acids long), between the SAR1/2-nanobody and SpyCatcher-3C cleavage site-His8. His8 is shown in bold font and the HRV (Human rhinovirus) 3C protease cleavage site is within the underlined sequence. HRV 3C Protease is a recombinant 3C protease derived from human Rhinovirus type 14 expressed in *E. coli.* The enzyme has the same activity as the native protein and cleaves a specific amino acid sequence (LEVLFQ↓GP) (SEQ ID NO:16).

**FIG. 1B** is an illustration of gate functionalization layers of a section **(100)** of the gate electrode shown in step 2 of **FIG. 1A**. Chemical and biological monolayers (Chem-SAM **(204)** and Bio-SAM **(204)**) are self-assembled on the gate electrode surface, which is shown exemplifying the attachment of virus spike **(200)**, to its binding partner on the Bio-SAM **(204)**. The molecular architecture of a section **(206)** of the gate functionalized layers shown in **FIG. 1B** is shown in **FIG. 1C.** As exemplified therein, a synthetic SpyTag peptide is chemically coupled to the 1,6-hexanedithiol (HDT) monolayer to form a Chem-SAM **(204)**. The nanobody-SpyCatcher fusion protein then attaches itself to this chemical layer through the autocatalytic formation of a covalent SpyCatcher-SpyTag bond, forming the Bio-SAM. The nanobody domain defines sensor specificity and is interchangeable (examples below demonstrate the device design and its function with GFP, SARS-CoV, and MERS-CoV).

### iii. Blocking agents

Optionally, the biorecognition layer includes a blocking agent. Exemplary blocking agents include, but are not limited to, bovine serum albumin (BSA), ethanolamine (ETA) and Casein. In some preferred embodiments, the blocking agent included in the biorecognition layer is BSA. While not being bound by theory, the blocking agent provided to capture potentially contaminating proteins in samples included analyte, which are contacted with the biorecognition layer for binding of the analyte to its binding partner (i.e., biorecognition element) present in the biorecognition layer.

### B. OECT components

The disclosed device includes a biorecognition layer described above and its integration with a high gain, ion-to-electron transducing device, the OECT. The device may include one OECT channel or more than one OECT channel in the form of an array. The OECT or each OECT in an array of OECT (i.e. two or more OECTs) in the device is a three-terminal transistor which includes a source electrode, a drain electrode, a channel, and a gate electrode. The channel electronically connects the source electrode and drain electrode. The gate electrode that is integrated/functionalized with a biorecognition layer.

The source electrode and the drain electrode are placed apart and connected electronically by a corresponding channel. In some embodiments, the source electrode, the drain electrode, and the channel can be patterned on a supporting substrate, such as a glass substrate, a silicon substrate, or a plastic substrate, such as a polyimide substrate or a textile. The gate electrode is placed separately from the source electrode, the drain electrode, and the channel separated by an electrolyte. The channel is in direct contact with an electrolyte solution, into which the gate electrode is also immersed. Optionally, the OECT includes a reservoir to contain the electrolyte solution. The reservoir (i.e. PDMS well) is placed on top of the channel to contain the electrolyte solution such that the channel and the gate electrode are in contact with the electrolyte solution. The source and drain contacts are also in contact with the electrolyte but they are insulated with an insulator such as parylene or SU-8. The source electrode, drain electrode, and channel are patterned on a glass substrate.

When the device contains an array of OECTs, each OECT may contain a source electrode, a drain electrode, and a corresponding channel, and all the OECTs in the array use a common gate electrode. For example, if the OECT array includes a plurality of OECTs, for example 2, 3, 4, 5 or 6 OECTs, where each OECT contains a source electrode, a drain electrode, and a corresponding channel. However all the OECTs in the array use a common gate electrode that is integrated with a biorecognition layer. The source electrode, drain electrode, and corresponding channel of each of the OECTs are patterned on a supporting substrate'. Optionally, when the device contains an array of OECTs, each OECT may contain a source electrode, a drain electrode, a corresponding channel, and a gate electrode, such that each gate electrode can be engineered with the same or different biorecognition element for detecting one or more than one analyte simultaneously. The device may be incorporated into a microfluidics configuration.

The channel is typically made of an ion-permeable organic electronic material, through which holes or electrons flow from the source electrode to the drain electrode. The OECT relies on ions that are injected from the electrolyte solution into the ion-permeable organic electronic material, thereby changing its doping state and thus its conductivity. The operation of OECT is controlled by voltages applied to the gate electrode (gate voltage, $V_G$) and to the drain electrode (drain voltage, $V_D$), which are referenced with respect to the source electrode. The drain voltage induces a current (drain current, $I_D$), which is proportional to the quantity of mobile holes or electrons in the channel, and thus probes the doping state of the organic electronic material. The gate voltage controls the injection of ions into the channel and thus the doping state of the organic electronic material, resulting in a change in $I_D$. The change in $I_D$ may be expressed as a normalized response (also referred herein as "NR") which can be calculated based on equation 1:

$$NR = (|I_D - I_0|)/I_0$$

where $I_0$ is the drain current obtained after incubation of the OECT with a blank solution (i.e. a solution in the absence of the target analytes) under the same measurement conditions as the biological sample.

The doping changes in OECT occur over the entire volume of the channel because of the injection barrier-free penetration of electrolyte ions into the bulk of the organic channel, causing a large modulation of the carrier density therein. The device translates small ionic fluxes in the electrolyte into a large electrical readout from the channel. Therefore, the transducing event is coupled with amplification, and endows the OECT with high gain at low voltages (<1V).

### i. Source and Drain Electrodes

The source and drain electrodes are made from materials capable of conducting an electric current. The electrode materials can be organic or inorganic in nature, as long as it is able to conduct electrons and inject electronic charges into the channel material. The electrodes can be a polymeric electrode, a metallic electrode, a carbon-based material, a metal oxide, or a modified electrode. In some embodiments, the source and drain electrodes are made from an electrochemically inert material such as gold, platinum, or a conductive form of carbon. In some embodiments, the source and drain electrodes are gold electrodes. They are insulated using an insulator such as parylene. In some embodiments, SU-8 (epoxy based photoresist used to pattern electronics with photolithography (also used as an insulator)) is used.

In some embodiments, the electrodes are made from a metallic conductor. Suitable metallic conductors include but are not limited to gold, chromium, platinum, iron, nickel, copper, silver, stainless steel, mercury, tungsten and other metals suitable for electrode construction. The metallic conductor can be a metal alloy which is made of a combination of metals disclosed above, such as gold/chromium. In addition, conductive substrates which are metallic conductors can be constructed of nanomaterials made of gold, cobalt, diamond, and other suitable metals.

In other embodiments, the electrodes are made from carbon-based materials. Exemplary carbon-based materials are conducting polymers (in the form of films or fibers) carbon cloth, carbon paper, carbon screen printed electrodes, carbon paper, carbon black, carbon powder, carbon fiber, singe-walled carbon nanotubes, double-walled carbon nanotubes, multi-walled carbon nanotubes, carbon nanotube arrays, diamond-coated conductors, glassy carbon and mesoporous carbon. In addition, other exemplary carbon-based materials are graphene, graphite, uncompressed graphite worms, delaminated purified flake graphite, high performance graphite and carbon powders, highly ordered pyrolytic graphite, pyrolytic graphite, and polycrystalline graphite.

The electrodes can be doped semiconductors. Suitable semiconductors are prepared from silicon and germanium, which can be doped (i.e., the intentional introduction of impurities into an intrinsic semiconductor for the purpose of modulating its electrical and structural properties) with other elements. The semiconductors can be doped with phosphorus, boron, gallium, arsenic, indium or antimony, or a combination thereof.

Other electrode materials can be metal oxides, metal sulfides, main group compounds, and modified materials. Exemplary materials of this type are nanoporous titanium oxide, tin oxide coated glass, glass, cerium oxide particles, molybdenum sulfide, boron nitride nanotubes, aerogels modified with a conductive material such as gold, solgels modified with conductive material such as carbon, ruthenium carbon aerogels, and mesoporous silicas modified with a conductive material such as gold.

The source and drain electrodes can be any shape appropriate such as cuboid, cubic, circular, and cylindrical. In some preferred embodiments, the electrodes are cuboid gold electrodes. In some embodiments, each of the source and drain electrodes has a first dimension (i.e. width), a second dimension (i.e. length), and a third dimension (i.e. thickness). For example, each of the source and drain electrodes has a width in a range from 100 $\mu$m to 1 mm (e.g. 0.8 mm), a length in a range from 100 $\mu$m to 1 mm (e.g. 0.8 mm), and a thickness of about 100 nm.

### ii. Channel

Generally, the channel is configured to establish the electrochemical connection between a pair of source and drain electrodes such that holes or electrons flow from the source electrode to the drain electrode.

The channel is typically made of an ion-permeable organic electronic material, such as a conducting polymer disclosed in, for example, Rivnay, et al., Nature Reviews, 3:17086 (2018) and Sun, et al., J. Mater. Chem. C, 6:11778-11784 (2018). An exemplary conducting polymer for the channel is poly(3,4-ethylenedioxythiophene) doped with poly(styrene sulfonate) (PEDOT:PSS). The conducting PEDOT is p-type doped (oxidized), which leads to mobile holes that can hop from one chain to another, resulting in a hole current once a drain voltage is applied. These holes are compensated by the sulfonate anions of PSS. Channels made of PEDOT:PSS can work as depletion mode OECTs. For example, in the absence of a gate voltage, a hole current flows in the channel. Once a positive gate voltage is applied, cations from the electrolyte are injected into the channel and the anions are compensated, resulting in a decrease in the number of holes in the channel. This results in a decrease in the drain current. Alternatively, the channel can be made of materials that work in accumulation mode OECT, such as a semiconductor based on a polythiophene with a sulfonate group attached to the backbone with a hexyl chain (PTHS) (Inal, et al., *Adv. Mater.,* 26:7450-7455 (2014)), an ethylene glycol unit attached to bithiophenes (Moser, et al., Adv. Mater., 32:2002748 (2020)) or a g7-NDI-Br and NDI-T2 copolymer (P-90) (Giovannitti, et al., Chem. Mater., 30:2945-2953 (2018)). In accumulation mode p-type OECT, application of a negative gate voltage causes injection of anions into the channel and a corresponding accumulation of holes, leading to an increase in the drain current $I_D$. One can also use n-type semiconductors and build accumulation mode n-type OECTs. In accumulation mode n-type OECT, application of a positive gate voltage causes injection of cations into the channel and a corresponding accumulation of electrons, leading to an increase in the drain current $I_D$.

Other suitable conducting polymers for the channel include, but are not limited to, conductor based on PEDOT with a pendant sulfonate group (PEDOT-S), PEDOT doped with tosylate (PEDOT:TOS), PEDOTOH:ClO4, PEDOT-co-PEDOTOH:ClO4 (Schmode, et al., Chem. Mater., 31(14):5286-5295 (2019)), poly(2-(3,3'-bis(2-(2-(2-methoxyethoxy) ethoxy)ethoxy)-[2,2' bithiophen]-5 yl)thieno[3,2 b] thiophene), p(g2T TT), poly((ethoxy)ethyl 2-(2-(2 methoxyethoxy) ethoxy)acetate)-naphthalene 1,4,5,8 tetracarboxylic-diimide-co 3,3' bis(2-(2-(2 methoxyethoxy)ethoxy) ethoxy)-(bithiophene)) p(gNDI g2T), P3HT (poly(3-hexylthiophene-2,5-diyl)), BBL (polybenzimidazo-benzoisoquioline), PTHS-TMA+-co-P3HT (poly[(6-thiophen-3-yl)hexane-1-sulfonate-co-3-(hexylthiophene)]), poly(N,N'-bis(7-glycol)-naphthalene-1,4,5,8-bis(dicarboximide)-co-2,2'-bithiophene-coN,N'-bis(2-octyldodecyl)-naphthalene-1,4,5,8-bis(dicarboximide), naphthalene-1,4,5,8-tetracarboxylic-diimide-bithiophene (NDI-T2) based polymer with 90% glycol chain percentage (P-90), and glycolated Diketopyrrolopyrroles (DPPs) or glycolated thiophenes, bithiophenes such as p(g0T2-g6T2) (Moser, et al., Advanced Materials, 32:2002748 (2020)).

Examples of suitable polymers for the channel in a depletion mode OECT are PEDOT polymers or copolymers thereof, such as polymers based on PEDOT with a pendant sulfonate group (PEDOT-S), PEDOT doped with tosylate (PEDOT:TOS), PEDOTOH:ClO4, PEDOT-co-PEDOTOH:ClO4, and PEDOT:PSS.

Examples of suitable polymers for the channel in an accumulation mode OECT are poly(2-(3,3'-bis(2-(2-(2-methoxyethoxy) ethoxy)ethoxy)-[2,2' bithiophen]-5 yl)thieno[3,2 b] thiophene), p(g2T TT), poly((ethoxy)ethyl 2-(2-(2 methoxyethoxy) ethoxy)acetate)-naphthalene 1,4,5,8 tetracarboxylic-diimide-co 3,3' bis(2-(2-(2 methoxyethoxy)ethoxy) ethoxy)-(bithiophene)) p(gNDI g2T), P3HT (poly(3-hexylthiophene-2,5-diyl)), BBL (polybenzimidazo-benzoisoquioline), PTHS-TMA+-co-P3HT (poly[(6-thiophen-3-yl)hexane-1-sulfonate-co-3-(hexylthiophene)]), poly(N,N'-bis(7-glycol)-naphthalene-1,4,5,8-bis(dicarboximide)-co-2,2'-bithiophene-co-N,N'-bis(2-octyldodecyl)-naphthalene-1,4,5,8-bis(dicarboximide), naphthalene-1,4,5,8-tetracarboxylic-diimide-bithiophene (NDI-T2) based polymer with 90% glycol chain percentage (P-90), and glycolated Diketopyrrolopyrroles (DPPs) or glycolated thiophenes, bithiophenes such as p(g0T2-g6T2).

Typically, the channel has a first dimension (i.e. width) and a second dimension (i.e. length). The length is the distance between the source and drain electrodes. Width is the remaining dimension of that rectangle The length and width of the channel can be a value between about 5 $\mu$M and about 5mm, for example, between 5 $\mu$m and 1000 $\mu$m, between 5 $\mu$m and 100 $\mu$m, between 5 $\mu$m and 100 $\mu$m, or between 10 $\mu$m and 100 $\mu$m, for example, about 10 $\mu$m or about 100 $\mu$m. The width of the channel can be between 10 $\mu$m and 1000 $\mu$m, between 20 $\mu$m and 500 $\mu$m, or between 20 $\mu$m and 200 $\mu$m, for example, about 100 $\mu$m.

The first dimension and the second dimension can be the same or different. In some embodiments, the width of the channel is the same as the length of channel. For example, the channel has a width of about 100 $\mu$m and a length of about 100 $\mu$m. In some embodiments, the width of the channel is smaller than the length of the channel. In some preferred embodiments, the width of the channel is larger than the length of the channel. For example, the channel has a width of about 100 $\mu$m and a length of about 10 $\mu$m. In some embodiments, the OECT having a small channel (e.g. a width of about 100 $\mu$m and a length of about 10 $\mu$m) is more sensitive (i.e. higher NR) than the same OECT but having a larger channel (e.g. a width of about 100 $\mu$m and a length of about 100 $\mu$m) under the same measurement conditions (e.g. $V_G$, $V_D$, incubation time, temperature, and pressure).

Typically, the channel remains unmodified, i.e., it is not chemically modified to anchor binding partners. The analyte recognition takes place at the functionalized gate electrode, which is not in physical contact with the channel. This configuration allows prolonged shelf life of the device compared with OECT containing channels modified with binding partners. For example, the OECT disclosed herein can preserve its drain current (i.e. change of drain current is less than about 5% compared with a fresh sensor) after storing at ambient conditions (i.e. about 25°C at 1 atm) for at least 6 months in air or for at least 1 year in nitrogen or vacuumed environment For example, the OECT disclosed herein can preserve its NR determined with a standard solution containing the same amount of the same analyte (i.e. change of NR is less than about 5% compared with a fresh sensor) after storing at ambient conditions (i.e. about 25°C at 1 atm) for 6 months in air or 1 year in nitrogen or vacuumed environment.

The channel materials can be spin casted, drop casted or inkjet or screen printed from solutions.

### iii. Gate Electrode

The gate electrode is configured to control the injection of ions into the channel, typically placed in the measurement solution with the source electrode, the drain electrode and the channel, but not in physical contact with these components.

The gate electrode can be any shape appropriate such as cuboid, cubic, circular, and cylindrical. In some embodiments, the gate electrode is a circular electrode having a diameter in a range from 0.5 mm to 10 mm, from 1 mm to 10 mm, from 0.5 mm to 5 mm, or from 1 mm to 5 mm, such as 2.8 mm. In some preferred embodiments, the gate electrode is a square electrode having a length in a range from 0.1 mm to 10 mm, from 0.1 mm to 8 mm, from 0.1 mm to 5 mm, from 0.5 mm to 10 mm, or from 0.5 mm to 5 mm, such as about 0.8 mm or about 4.8 mm. In some preferred embodiments, the gate electrode is a square electrode having a length of about 0.8 mm.

In some embodiments, the OECT contains a gate electrode having any shape and size as described above and a channel having any shape and size as described above. In some preferred embodiments, the OECT contains a square electrode having any length as described above and a rectangular channel having any size (i.e. width and length) as described above. In a particularly preferred embodiment, the OECT contains a square gate electrode having a length of about 0.8 mm and a rectangular channel having a width of about 100 $\mu$m and a length of about 10 $\mu$m.

Generally, the gate electrode can be made from a substrate coated with any conducting materials descried above for the source and the drain electrodes (e.g., Pt or Au) or a conducting polymer, such as poly(fluorine)s, polyphenylenes, polypyrenes, polyazulenes, polynaphthalenes, poly(pyrrole)s, polycarbozoles, polyindoles, polyzaepines, polyanilines, poly(thiophene)s, poly(3,4-ethylenedioxythiophene), poly(p-phenylene sulfide), poly(acetylene)s, or poly(p-phenylene vinylene). In some embodiments, the gate electrode contains two or more conducting materials. For example, the gate electrode contains two conducting materials, where the first conducting material can be a material disclosed above for the source and the drain electrodes and a second conducting material can be a conducting polymer. In some preferred embodiments, the gate electrode contains two conducting materials, where the first conducting material is a metallic conductor of a first type and the second conducting material is a metallic conductor of a second type. The two conducting materials may be coated simultaneously or subsequently on the substrate. A preferred gate electrode is formed from a Kapton (polyimide) substrate sputter coated with Cr and Au.

Typically, the gate electrode is engineered to include a biorecognition layer. For example, the gate electrode is modified sequentially with a SAM, a linker, and a biorecognition element, such as nanobody, to form the biorecognition layer on the gate electrode.

### iv. Electrolyte Solution

The electrolyte solution is in electrically contact with the channel and the gate electrode, and the source electrode and the drain electrode (the last two are insulated with an insulator). The electrolyte solution is a solution that contains ions or molecules that have lost or gained electrons, and is electrically conductive and mostly aqueous. Electrolyte solutions include, but are not limited to, water, buffers such as phosphate buffered solution, phosphate buffered saline, salt water, MES buffer, Bis-Tris buffer, ADA, ACES, PIPES, MOPSO, Bis-Tris propane, BES, MOPS, TES, HEPES, DIPSO, MOBS, TAPSO, Trizma, HEPPSO, POPSO, TEA, EPPS, Tricine, Gly-gly, Bicine, HEPBS, TAPS, AMPD, TABS, AMPSO, CHES, CAPSO, AMP, CAPS, CABS. The electrolyte solution can have a pH between about 4 and about 8.5, between about 4.5 and about 8.5, between about 5 and about 8.5, between about 5.5 and about 8.5, between about 6 and about 8, or between about 6.5 and about 7.5, preferably about 7.4.

### v. Reservoir

The device may include a reservoir to contain the electrolyte solution. The reservoir may be incorporated in the OECT by any suitable methods. For example, the reservoir is glued on top of the channel. The reservoir can also be a microfluidic channel.

The reservoir is typically defined by a side wall and a bottom surface, and contains an opening configured to allow the electrolyte solution to enter the reservoir. The reservoir may have any suitable shapes, such as a cylindrical well, a cubic shape, or a cuboid shape. The bottom surface is formed from at least a portion of the channel and optionally, at least a portion of the side wall is formed from the source electrode and the drain electrode respectively. For example, the reservoir is a cylindrical well and has a bottom surface formed from at least a portion of the channel. The side wall is perpendicular to the surface of the channel and at least a portion of the side wall is in contact with the source electrode and drain electrode. The cylindrical reservoir contains an opening to allow the electrolyte solution to enter the reservoir. An exemplary OECT containing a cylindrical reservoir to contain the electrolyte solution is shown in **FIG. 1A.**

For example, the reservoir is a cuboid defined by a bottom surface and four side walls. The bottom surface is formed by at least a portion of the channel and each of the first and second side walls are parallel to each other and vertically placed on top of each of the source electrode and drain electrode. The third and fourth side walls are parallel to each other and perpendicular to the first and second side walls, such that the reservoir contains an opening to allow the electrolyte solution to enter the reservoir.

For example, the reservoir is a cuboid and has a bottom surface formed by the channel, a first side wall formed from a portion of the source electrode, a second side wall formed from a portion of the drain electrode, and a third side wall and a fourth side wall that are parallel to each other and perpendicular to the first side wall and second side wall, such that the reservoir contains an opening to allow the electrolyte solution to enter the reservoir. When an electrolyte solution is in the reservoir, the channel, at least a portion of the source electrode and the drain electrode, and the gate electrode are in contact with the electrolyte solution.

The reservoir can be made from any suitable inert material, such as plastic, glass, or a polymeric material, such as polydimethylsiloxane (PDMS).

## III. METHODS OF MAKING

The disclosed biosensors integrate OECT technology engineered to include a biorecognition layer by functionalizing the sensing electrode (i.e. gate electrode), sequentially, via a SAM, a binding partners, preferably, covalent binding partners or an affinity pair (e.g. the SpyTag/SpyCatcher conjugation system), and a biorecognition element. Optionally, the biorecognition layer is also exposed to a blocking agent, which can be introduced simultaneously with the biorecognition element or subsequently to the biorecognition element.

Methods of making an OECT or an array of two or more OECTs are known. An exemplary method of making an OECT array is described in the Examples. The biorecognition elements, such as nanobody units, can be immobilized with uniform orientations using the methods described herein; this allows the biorecognition layer to be more precise and compact.

Generally, methods of integrating a biorecognition layer on an electrode, such as a gate electrode of an OECT, include: (i) contacting at least a portion of the surface of the electrode with a first solution containing a plurality of organic molecules under conditions that result in self-assembly of the organic molecules, to produce a SAM-modified OECT surface, (ii) contacting the SAM-modified surface with a second solution containing a first peptide to produce a first peptide-SAM-modified surface, referred herein as Chem-SAM, and (iii) contacting the Chem-SAM -modified surface with a third solution containing a second peptide (which is a binding partner to the first peptide), a biorecognition element for the analyte of interest and optionally, a blocking agent, where the first peptide conjugates with the second peptide to form a second monolayer, referred to herein as Bio-SAM. The OECT electrode modified by Chem-SAM and BioSAM results in a modified OECT gate electrode surface, with the recognition element exposed for interaction with an analyte (to which it binds) from a sample. (See **FIGs. 1B and 1C**). The second solution preferably includes a blocker molecule such as BSA with, or without a mild detergent for example, Tween®20 (Polyethylene glycol sorbitan monolaurate).

Referring to Formula I, (i) the surface of the electrode is contacted with a solution containing organic molecules (N) capable of self-assembling into a SAM, to form a first SAM; (ii) the first SAM is contacted with a composition including $AP_1$ (with/or without $L_1$) under conditions that result in chemical coupling of the $AP_1$ to the first SAM, thus forming a Chem-SAM on the OECT electrode surface, and (iii) contacting the Chem-SAM with a composition including $AP_2$-$L_2$-B- under conditions resulting in conjugation of $AP_1$ and $AP_2$ to form a biologically self-assembled monolayer, herein Bio-SAM. (FIG. 1B and C). The composition in step (iii) preferably includes a blocker molecule such as BSA with, or without a mild detergent for example, Tween®20 (Polyethylene glycol sorbitan monolaurate).

### A. Modification with Chem-SAMs

Generally, a plurality of organic molecules are dissolved in a solvent to form a first incubation solution. A portion of or the entire surface of the electrode to be modified, such as the gate electrode of an OECT, is immersed in the first composition containing the plurality of organic molecules. The electrode surface is incubated with the first composition, optionally in the dark, for a time period sufficient to form a SAM on the surface of the electrode. Typically, the incubation time period sufficient to form the SAM on the surface of the electrode is in a range from 10 minutes to 36 hours, from 10 minutes to 24 hours, from 10 minutes to 12 hours, from 10 minutes to 10 hours, from 10 minutes to 5 hours, from 10 minutes to 2 hours, or from 10 minutes to 1 hour, such as about 1 hour.

Suitable solvents that can be used to dissolve the plurality of organic molecules used to form the first SAM on the surface of the OECT electrode, are known. Exemplary solvents for the incubation solution for SAM formation include, but are not limited to, methanol, ethanol, Dimethyl sulfoxide (DMSO), toluene, hexane, water, and a buffer as described above, and a combination thereof.

Suitable organic molecules that can form a SAM on an electrode are known, for example, thiol molecules, phosphine molecules, or silicon oxide derivatives. In some preferred embodiments, the organic molecules forming the SAM on the electrode surface of the OECT, such as the gate electrode surface, are thiol molecules. Any thiols as described above or a mixture thereof can be used to form the SAM layer on the electrode surface of the OECT. The SAM may be formed from a single type of thiol or a mixture of two or more types of thiols. For example, the SAM may be formed from HDT or PDT, or a mixture of PDT and MPA.

Typically, the total concentration of the organic molecules in the incubation solution is in a range from 0.1 mM to 50 mM, from 0.1 mM and 40 mM, from 0.1 mM and 30 mM, from 0.1 mM and 25 mM, from 0.1 mM and 20 mM, from 0.1 mM and 15 mM, from 0.1 mM and 12 mM, from 0.1 mM and 10 mM, from 0.1 mM and 5 mM, or from 0.5 mM and 5 mM. The term "total concentration of organic molecules" refers to the total mol of the organic molecules relative to the total volume of the solvent. For example, when the incubation solution contains a single type of thiol molecules, such as HDT, the concentration of the HDT molecules in the incubation solution is in a range from 0.1 mM to 50 mM, from 0.1 mM and 40 mM, from 0.1 mM and 30 mM, from 0.1 mM and 25 mM, from 0.1 mM and 20 mM, from 0.1 mM and 15 mM, or from 0.1 mM and 12 mM, from 0.1 mM and 10 mM, from 0.1 mM and 5 mM, or from 0.5 mM and 5 mM, such as about 1 mM. For example, when the incubation solution contains two types of thiol molecules, such as PDT and MPA, the total concentration of the PDT molecules and MPA molecules in the incubation solution is in a range from 0.1 mM to 50 mM, from 0.1 mM and 40 mM, from 0.1 mM and 30 mM, from 0.1 mM and 25 mM, from 0.1 mM and 20 mM, from 0.1 mM and 15 mM, or from 0.1 mM and 12 mM, such as about 12 mM.

When the incubation solution contains two or more types of organic molecules, the concentration of each type of the organic molecules can be in a suitable range to provide a total concentration of the two or more types of organic molecules in a range from 0.1 mM to 50 mM, from 0.1 mM and 40 mM, from 0.1 mM and 30 mM, from 0.1 mM and 25 mM, from 0.1 mM and 20 mM, from 0.1 mM and 15 mM, or from 0.1 mM and 12 mM, such as about 12 mM. For example, the incubation solution contains a mixture of PDT and MPA, where the concentration of PDT is in a range from 0.05 mM to 49.9 mM, from 0.1 mM and 30 mM, from 0.1 mM and 25 mM, from 0.1 mM and 20 mM, from 0.1 mM and 15 mM, or from 0.1 mM and 12 mM, such as about 10 mM, and the concentration of MPA is in a range from 0.05 mM to 49.9 mM, from 0.1 mM and 30 mM, from 0.1 mM and 25 mM, from 0.1 mM and 20 mM, from 0.1 mM and 15 mM, or from 0.1 mM and 12 mM, such as about 2 mM, such that the total concentration of PDT and MPA is in a range from 0.1 mM to 50 mM, such as about 12 mM.

Optionally, following incubation, the SAM-modified electrode surface is rinsed with a first rinsing composition and optionally dried with a gas stream, such as Nitrogen gas. Exemplary rinsing compositions include, but are not limited to methanol, ethanol, and water, and a combination thereof.

## B. Modification with a First Peptide

Generally, a first peptide (of a binding pair) is dissolved in a buffer as described above, such as PBS to form a second incubation composition. The fist peptide can be a modified peptide, which includes a short linker and/or a chemical modification such as modified with a maleimide functional group(s). The SAM-modified electrode surface is immersed in the second incubation composition containing the first peptide for a time period sufficient to couple the first peptide to the SAM (i.e., the first SAM) on the surface of the electrode such that a first peptide-SAM modified surface is produced. Typically, the incubation time period sufficient to couple the first peptide to the SAM on the surface of the electrode is in a range from 10 minutes to 24 hours, from 10 minutes to 20 hours, from 10 minutes to 15 hours, from 10 minutes to 12 hours, from 10 minutes to 10 hours, from 10 minutes to 5 hours, from 10 minutes to 2 hours, from 10 minutes to 1.5 hours, or from 10 minutes to 1 hour, such as about 1 hour.

The first peptide can couple to the first SAM and conjugate with a second peptide as described below. An exemplary first peptide is SpyTag peptide, unmodified, or preferably, which is modified with a short linker (SEQ ID NO:1) (disclosed in Zakeri, et al., Proc. Natl. Acad. Sci., 109:E690-E697 (2012)), preferably modified with a maleimide functional group;); this allows the SpyTag peptide to conjugate with the free thiol group(s) in the SAM via thiol-maleimide "click" chemistry reaction.

Typically, the concentration of the first peptide in the second incubation solution is in a range from 0.01 mg/mL to 20 mg/mL, from 0.01 mg/mL to 15 mg/mL, from 0.01 mg/mL to 10 mg/mL, from 0.05 mg/mL to 20 mg/mL, from 0.05 mg/mL to 15 mg/mL, from 0.05 mg/mL to 10 mg/mL, from 0.01 mg/mL to 5 mg/mL, from 0.01 mg/mL to 1 mg/mL, from 0.05 mg/mL to 5 mg/mL, or from 0.05 mg/mL to 1 mg/mL, such as about 0.1 mg/mL.

Optionally, following incubation, the first peptide-SAM-modified electrode surface is rinsed with a second rinsing composition, such as a buffer as described above.

## C. Method for Producing Biorecognition element-containing fusion proteins

Fusion proteins of Formula II can be obtained by, for example, by chemical synthesis, and more preferably, by recombinant production in a host cell. To recombinantly produce a fusion proteins of Formula II, a nucleic acid containing a nucleotide sequence encoding the polypeptide can be used to transform, transduce, or transfect a bacterial or eukaryotic host cell (e.g., an insect, yeast, or mammalian cell). In general, nucleic acid constructs include a regulatory sequence operably linked to a nucleotide sequence encoding a fusion proteins of Formula II. Regulatory sequences (also referred to herein as expression control sequences) typically do not encode a gene product, but instead affect the expression of the nucleic acid sequences to which they are operably linked. The nucleotide sequences encoding the fusion protein are usually inserted into a recombinant vector which may be any vector, which may conveniently be subjected to recombinant DNA procedures, and the choice of vector will often depend on the host cell into which it is to be introduced. Thus, the vector may be an autonomously replicating vector, i.e. a vector, which exists as an extrachromosomal entity, the replication of which is independent of chromosomal replication, e.g. a plasmid. Alternatively, the vector may be one which, when introduced into a host cell, is integrated into the host cell genome and replicated together with the chromosome(s) into which it has been integrated. The vector is preferably an expression vector in which the DNA sequence encoding the fusion protein is operably linked to additional segments required for transcription of the DNA. In general, the expression vector is derived from plasmid or viral DNA, or may contain elements of both. The term, "operably linked" indicates that the segments are arranged so that they function in concert for their intended purposes, e.g. transcription initiates in a promoter and proceeds through the DNA sequence coding for the fusion protein. Expression vectors for use in expressing the fusion protein will comprise a promoter capable of directing the transcription of a cloned gene or cDNA. The promoter may be any DNA sequence, which shows transcriptional activity in the host cell of choice and may be derived from genes encoding proteins either homologous or heterologous to the host cell. Expression vectors for use in expressing the fusion protein will comprise a promoter capable of directing the transcription of a cloned gene or cDNA. The promoter may be any DNA sequence, which shows transcriptional activity in the host cell of choice and may be derived from genes encoding proteins either homologous or heterologous to the host cell. Examples of suitable promoters for directing the transcription of the DNA in mammalian cells are the SV40 promoter (Subramani et al., Mol. Cell. Biol. 1 (1981), 854-864), the MT-1 (metallothionein gene) promoter (Palmiter et al., Science 222 (1983), 809-814), the CMV promoter (Boshart et al., Cell 41:521-530, 1985) or the adenovirus 2 major late promoter (Kaufman and Sharp, Mol. Cell. Biol, 2:1304-1319, 1982).

Useful prokaryotic and eukaryotic systems for expressing and producing polypeptides are well known in the art include, for example, *Escherichia coli* strains such as BL-21, and cultured mammalian cells such as CHO cells.

In eukaryotic host cells, a number of viral-based expression systems can be utilized to express fusion proteins of Formula II. Viral based expression systems are well known in the art and include, but are not limited to, baculoviral, SV40, retroviral, or vaccinia based viral vectors.

The expressed tagged or fusion proteins produced by the cells may be recovered from the culture medium by conventional procedures including separating the host cells from the medium by centrifugation or filtration, releasing the fusion protein by mechanical cell disruption, such as ultrasonication or pressure, precipitating the proteinaqueous components of the supernatant or filtrate by means of a salt, e.g. ammonium sulphate.. After sonication a suitable concentration of NaCl can be added to further decrease the ability of host cell contaminants to bind to the cation exchange matrix. After cation-exchange chromatography the fusion protein may be eluted in a salt gradient and eluate fractions containing the fusion protein are collected. In some preferred forms, fusion protein is captured from lysate through its His tag. So IMAC (immobilized metal affinity chromatography) was used and then, after concentration of protein-containing fractions, they are subjected to size exclusion chromatography (SEC) for final purification. In particularly preferred embodiments for nanobody purification, the nanobody is purified from the periplamic space, where the host cell is bacteria, for example, E. coli. This would include (1) centrifugation, (2) osmotic shock to release the protein from the cell wall compartment, (3) IMAC (Immobilized Metal Ion Affinity Chromatography), (4) SEC (Size Exclusion Chromatography).

### D. Modification with Biorecognition Elements

Generally, a second peptide-biorecognition element conjugate and a blocking agent are dissolved in a binding buffer solution, such as HEPES, to form a third incubation solution. Preferably, the blocking agent contained in the third incubation solution is BSA.

Typically, the concentration of the blocking agent in the third incubation solution is in a range from 0.01% w/v to 10% w/v, from 0.01% w/v to 5% w/v, from 0.01% w/v to 1% w/v, from 0.05% w/v to 10% w/v, from 0.05% w/v to 5% w/v, or from 0.05% w/v to 1% w/v, such as about 0.1% w/v.

The first peptide-SAM-modified electrode surface is immersed in the third incubation solution containing the second peptide-biorecognition element conjugate and the blocking agent. Generally, the second peptide of the peptide-biorecognition element conjugate is capable of conjugating with the first peptide to form a biomolecular linker and thus attached the biorecognition element on the electrode surface. An exemplary second peptide is SpyCatcher peptide as disclosed in Zakeri, et al., Proc. Natl. Acad. Sci., 109:E690-E697 (2012). For example, a SpyTag/SpyCatcher linker is formed to control the oriented immobilization of the biorecognition elements (e.g., nanobodies) on the electrode surface. For example, after linker formation, the biorecognition element (e.g., nanobody) is functionalized on the gate electrode surface of the OECT, in a configuration vertical to electrode surface and is vertical relative to the channel surface, allowing for binding with an analyte in a sample, when the sample contacts the biorecognition element-functionalized electrode. The biorecognition elements can vary depending on the analytes of interest. For example, the biorecognition element is a nanobody such as an anti-GFP nanobody or an anti-SARS-1-RBD nanobody or an anti-MERS RBD nanobody *(See* TABLE 2).

The conjugation between SpyTag and SpyCatcher is a robust method for conjugating the recombinant proteins where the peptide SpyTag can spontaneously react with the protein SpyCatcher in a facile manner and with high specificity (Zakeri, et al., Proc. Natl. Acad. Sci., 109:E690-E697 (2012); Keeble et al., 2017). Maleimide functional groups can be constructed in the SpyTag peptide and nanobodies as fusion to the SpyCatcher proteins, and expressed as fusion protein in any suitable protein expression system, preferably with a tag such as a His tag, to aid in its purification.

The first peptide-SAM-modified electrode surface is incubated with the third incubation solution for a time period sufficient to form the linker (i.e. first peptide/second peptide conjugate), such that a biorecognition element-linker-SAM modified electrode surface is produced (biorecognition layer integrated on the electrode surface.

Typically, the incubation time period sufficient to form the second peptide/first peptide conjugate on the surface of the electrode is in a range from 5 minutes to 24 hours, from 10 minutes to 20 hours, from 10 minutes to 15 hours, from 10 minutes to 12 hours, from 10 minutes to 10 hours, from 10 minutes to 5 hours, from 10 minutes to 2 hours, from 10 minutes to 1.5 hours, or from 10 minutes to 1 hour, such as about 1 hour.

Typically, the concentration of the second peptide-recognition element conjugate in the third incubation solution is in a range from 1 $\mu$M to 100 $\mu$M, from 1 $\mu$M to 100 $\mu$M, from 1 $\mu$M to 90 $\mu$M, from 1 $\mu$M to 80 $\mu$M, from 10 $\mu$M to 100 $\mu$M, from 10 $\mu$M to 90 $\mu$M, from 10 $\mu$M to 80 $\mu$M, from 5 $\mu$M to 100 $\mu$M, from 5 $\mu$M to 90 $\mu$M, from 5 $\mu$M to 80 $\mu$M, from 20 $\mu$M to 100 $\mu$M, from 20 $\mu$M to 90 $\mu$M, or from 20 $\mu$M to 80 $\mu$M, such as about 50 $\mu$M.

Optionally, following incubation, the biorecognition layer-integrated electrode surface is rinsed with a third rinsing solvent, such as a buffer as described above.

### E. Exemplary Methods of Electrode Functionalization

An exemplary method of preparing a biorecognition layer-integrated electrode surface is described in the Examples.

A preferred method uses p-type depletion (PEDOT:PSS) and accumulation (such as p(g0T2-g6T2)) mode materials in the OECT channel while the gate electrode is gold. The gate electrode (preferably gold) surface is functionalized with a biorecognition layer, i.e., the nanobody. The gate electrode surface is electrochemically cleaned and then exposed to 1,6-hexanedithiol (HDT) to form a thiol-self assembled monolayer (SAM). Particularly preferred is a modified peptide (SpyTag) with a terminal of maleimide functional group, conjugated with the thiol SAM layer via thiol-maleimide "click" chemistry reaction. After that, the specific nanobody, which is the recognition element-spycatcher conjugate is diffused into the SpyTag proteins to form the biofunctional layer.

### III. METHODS OF USING

The disclosed methods above can be used to make OECT devices containing a nanobody whose binding partner is any analyte of interest, for example an antigen from any pathogen. The disclosed device is used to measure the presence, the absence, or the concentration of any analyte in a sample, where the analyte is a binding partner to the biorecognition element functionalized onto the device. The biorecognition element functionalized-OECT Sensor features: improved sensitivity high sensitivity over prior art devices (aM or zM compared to fM) and selectivity, delivering accurate results in accordance with gold standard tests (where possible and applicable, correlated to DNA or RNA detection results with the conventional PCR method); fast detection time (less than 15 minutes after the sample exposure to the sensor surface to the read-out); and high limit of detection (lower limit of detection (LOD) exemplified below for SARS-CoV-2 S1 in saliva is $1.2 \times 10^{-21}$ M).

The OECT sensor relies on the channel made from ion-permeable organic electronic material, through which holes or electrons flow from the source electrode to the drain electrode. When ions are injected from the electrolyte solution into the channel, its doping state is changed and thus its conductivity is changed. The operation of OECT is controlled by voltages applied to the gate electrode (gate voltage, $V_G$) and to the drain electrode (drain voltage, $V_D$), which are referenced with respect to the source electrode. The drain voltage induces a current (drain current, $I_D$), which is proportional to the quantity of mobile holes or electrons in the channel, and thus probes the doping state of the organic electronic material. The gate voltage controls the injection of ions into the channel and thus the doping state of the organic electronic material, resulting in a change in $I_D$. The change in $I_D$ may be expressed as a normalized response (also referred herein as "NR") which can be calculated based on equation 1:

$$NR = (|I_D - I_0|)/I_0$$

where $I_0$ is the drain current obtained after incubation of the OECT with a blank solution (i.e. a solution in the absence of the target analytes) under the same measurement conditions as the biological sample.

As the nanobody selectively captures its target, in this case GFP protein or SAR-CoV-2 protein or MERS-CoV protein, this binding event changes the capacitance of the gate electrode and induces the potential across the gate/-electrolyte interface, suppressing the gating of the OECT. Therefore, the OECT signal varies depending on the concentration of GFP down to femtomolar and attomolar range. This strategy of utilizing nanobody units will open up a new avenue for the design of electronic biosensors and can be further expanded into different sensing platforms and various target proteins.

Generally, a method of using the disclosed device for testing the presence, the absence, and/or concentration of analytes in a biological sample includes: (i) adding an electrolyte solution into the reservoir; (ii) incubating the gate electrode (functionalized with a biorecognition layer as described above) with a blank solution, for example a buffer like PBS, universal transport medium (UTM), or virus transport medium (VTM); (iii) placing the gate electrode on top of the channel covered by the electrolyte, (iv) applying a $V_G$ and a $V_D$; (v) measuring a first $I_D$; (vi) incubating the gate electrode with the biological sample for a time period sufficient to allow binding between the analyte and the biorecognition element; (vii) rinsing the gate electrode with a rinsing buffer; and (viii) measuring a second $I_D$, where a difference between the second $I_D$ and the first $I_D$ is indicative of the absence, the presence, or the concentration of the analyte in the biological sample. The second $I_D$ may be larger, the same or substantially the same as, or smaller than the first $I_D$. Optionally, steps (v)-(vii) are repeated one or more times.

The electrolyte solution added into the reservoir can be any electrolyte solution described above, for example, a PBS at pH about 7.4. The blank solution may be a buffer solution that does not contain the analyte of interest and is used to establish a baseline drain current, i.e. $I_0$. Typically, a drop of the blank solution is applied onto the gate electrode and incubated for about 10 minutes. Generally, the time period for incubating the gate electrode with the blank solution is the same as the incubation time period sufficient to allow binding between the analyte and the biorecognition element. Optionally, the gate electrode is rinsed with a rinsing buffer following incubation with the blank solution for about 10-15 second. The rinsing buffer may be any electrolyte solution as described above, such as PBS.

Typically, the $V_G$ and $V_D$ are applied simultaneously to the gate electrode and the drain electrode, respectively. In some embodiments, the $V_G$ is applied to the gate electrode by sweeping from a first gate voltage to a second gate voltage at a gate voltage step and the $V_D$ is applied to the drain electrode by sweeping from a first drain voltage to a second drain voltage at a drain voltage step. For example, the $V_G$ is applied to the gate electrode by sweeping from -1 V to 1 V, from -0.8 V to 0.8 V, or from -0.6 V to 0.6 V, at a gate voltage step of 0.05 V, 0.1 V, 0.2 V, or 0.5 V and the $V_D$ is applied to the drain electrode by sweeping from 0 V to 1 V, from 0.5 V to -1 V, from 0 V to -1 V, from 0 V to -0.8 V, or from 0 V to -0.6 V, at a drain voltage step of 0.05 V, 0.1 V, 0.2 V, or 0.5 V. In some preferred embodiments, the $V_G$ is applied to the gate electrode by sweeping from -0.6 V to 0.6 V at a gate voltage step of 0.1 V and the $V_D$ is applied to the drain electrode by sweeping from 0 V to -0.6 V at a drain voltage step of 0.1 V. When both $V_G$ and $V_D$ are applied by sweeping, a range of $I_D$s can be collected.

In some embodiments, a fixed $V_G$ is applied to the gate electrode and a fixed $V_D$ is applied to the drain electrode. The fixed $V_G$ and fixed $V_D$ may be the same or different. For example, a fixed $V_G$ in the range from -1 V to 1V, from -0.6 V to 0.6 V, or from -0.1 V to 0.1 V, such as -0.6V, -0.1 V, or 0.6 V is applied to the gate electrode, and a fixed $V_D$ in the range from -1 V to 1V, from -0.6 V to 0.6 V, 0 V to -0.6 V, or from -0.1 V to 0.1 V, such as - 0.6 V, -0.1 V, or 0.6 V, is applied to the drain electrode. In some embodiments, a $V_G$ of -0.1 V is applied to the gate electrode and a $V_D$ of -0.1 V is applied to the drain electrode. In some embodiments, a $V_G$ of 0.1 V is applied to the gate electrode and a $V_D$ of 0.1 V is applied to the drain electrode. In some embodiments, a $V_G$ of -0.1 V is applied to the gate electrode and a $V_D$ of 0.1 V is applied to the drain electrode. In some embodiments, a $V_G$ of 0.1 V is applied to the gate electrode and a $V_D$ of -0.1 V is applied to the drain electrode. In some embodiments, a $V_G$ of -0.6 V is applied to the gate electrode and a $V_D$ of -0.6 V is applied to the drain electrode. In some embodiments, a $V_G$ of 0.6 V is applied to the gate electrode and a $V_D$ of 0.6 V is applied to the drain electrode. In some embodiments, a $V_G$ of 0.6 V is applied to the gate electrode and a $V_D$ of -0.6 V is applied to the drain electrode. In some embodiments, a $V_G$ of -0.6 V is applied to the gate electrode and a $V_D$ of 0.6 V is applied to the drain electrode. When a fixed $V_G$ and a fixed $V_D$ are applied, a single $I_D$ can be collected.

The biological sample is typically in a liquid form and applied onto the gate electrode as a drop and incubates for a period of time sufficient to allow binding between the analytes and the biorecognition element (e.g. nanobody) immobilized on the gate electrode of the OECT. Typically, the volume of the biological sample sufficient for incubation is small. For example, the volume of the biological sample sufficient for incubation is less than 20 $\mu$L, less than 10 $\mu$L, or preferably, $\leq$5 $\mu$L, and more preferably, about 5 $\mu$L.

The biological samples can be a bodily fluid, such as whole blood, plasma, serum, saliva, nasal swab, mucus, sputum (processed or unprocessed), bronchial alveolar lavage (BAL), bronchial wash (BW), cerebrospinal fluid (CSF), and urine.

**In** some embodiments, the biological sample is not a bodily fluid, but is a liquid obtained from a solid specimen, such as tissue (e.g., biopsy material), feces, rectal swab, nasopharyngeal swab, and throat swab. When the biological sample is not a bodily fluid, the above-described exemplary method can be modified to include an initial step of processing the specimen t to obtain a sample in liquid form, which is then subjected to steps (i)-(v) described above for a method of using the disclosed device for testing the presence, the absence, and/or concentration of analytes in a biological sample. Processing methods to transform a specimen (which is not a fluid) into a liquid form are known. For example, when the specimen is a nasopharyngeal swab, it is processed by placing the proximal portion of the swab in a buffer to produce the biological sample in a liquid form (Lopez, et al., Pediatr. Res., 86(5):651-654 (2019)). Optionally, when specimen processing is needed, the method also includes a desalting step prior to specimen processing, where the buffer is run through a desalting column to remove any redox reagents, such as dithiothreitol (DTT), to reduce interference signals caused by non-specific interactions of the redox reagents with the gate electrode surface. Optionally, the desalting step is performed following specimen processing and prior to step (v), where the obtained biological sample in a liquid form is run through a desalting column to remove any redox reagents introduced during the specimen processing step.

In some embodiments, the above-described exemplary method includes a step of adding a protease inhibitor into the biological sample prior to other steps, such as prior to any one of steps (i)-(v), particularly prior to step (vi). The protease inhibitor can prevent or reduce the damage to the sensor's protein-based recognition layer caused by protease activity in the biological or environmental sample.

In some embodiments, the above-described exemplary method includes a step of diluting the biological sample using a sensor binding buffer, prior to other steps, such as prior to any one of steps (i)-(vi), particularly prior to step (vi). The dilution factor for the biological sample following the dilution step can be from 0.1:1 to 1:10, from 0.5:1 to 1:10, from 0.5:1 to 1:5, from 1:1 to 1:10, or from 1:1 to 1:5, such as about 1:1 or about 1:3 in the sensor binding buffer.

For example, the above-described exemplary method includes a step of diluting a biological sample collected on a nasal swab using a sensor binding buffer. Generally, the sensor binding buffer contains one or more buffering agents, one or more salts, one or more detergents, and one or more protease inhibitors. Optionally, the sensor binding buffer also contains one or more antimicrobial agents and/or one or more blocking agents as described above. The sensor binding buffer is at the physiological pH, i.e. about pH 7.4.

The buffering agents in the sensor binding buffer can stabilize pH of the buffer and can be tolerated by proteins. Examples of buffering agents that are suitable for use in the sensor binding buffer include, but are not limited to, Tris, HEPES, sodium hydrogen phosphate, sodium dihydrogen phosphate, potassium hydrogen phosphate, potassium dihydrogen phosphate, sodium borate, potassium borate, citric acid, TAPS, Bicine, Tricine, TAPSO, TES, MOPS, PIPES, cacodylate, and MES, and a combination thereof. The total concentration of the one or more buffering agents in the sensor binding buffer can be in a range from about 5 mM to about 100 mM, from about 10 mM to about 100 mM, from about 5 mM to about 50 mM, from about 10 mM to about 50 mM, or from about 20 mM to about 50 mM, such as about 20 mM or about 50 mM.

The salts in the sensor binding buffer can maintain the solubility and structural integrity of proteins. These can be salts that are near the middle of the Hofmeister series and thus not promoting "salting out" (precipitation of proteins) but also not promoting denaturing of proteins. Examples of salts that are suitable for use in the sensor binding buffer include, but are not limited to, potassium fluoride, potassium bromide, potassium hydrogen phosphate, potassium acetate, potassium citrate, lithium fluoride, lithium bromide, lithium hydrogen phosphate, lithium acetate, and lithium citrate, and a combination thereof. The total concentration of the one or more salts in the sensor binding buffer can be in a range from about 50 mM to about 1 M, from about 100 mM to about 1 M, from about 150 mM to about 1 M, from about 100 mM to about 500 mM, or from about 150 mM to about 500 mM, such as about 150 mM, about 250 mM, or about 500 mM.

The detergents in the sensor binding buffer are typically non-ionic and non-denaturing detergents, such as detergents with polyoxyethylene head groups that contain alkylpolyethylene ethers with the general formula $C_nH_{2n+1}$ $(OCH_2CH_2)xOH$ or a phenyl ring between the alkyl chain and the ether group (not denaturing detergents such as SDS and ethyl trimethyl ammonium bromide). Examples of detergents that are suitable for use in the sensor binding buffer include, but are not limited to, Tween, Triton-X100, Nonidet P40, IGEPAL CA-630, Brij-35, Brij-58, and Triton X-114, and a combination thereof. The total concentration of the one or more detergents (by volume) in the sensor binding buffer can be in a range from about 0.01% to about 10%, from about 0.01% to about 5%, from about 0.05% to about 5%, from about 0.05% to about 2%, from about 0.05% to about 1%, or from about 0.1% to about 1%, such as about 0.05% or 1%. The total concentration of the one or more detergents (by weight) in the sensor binding buffer can be in a range from about 0.01% to about 10%, from about 0.01% to about 5%, from about 0.05% to about 5%, from about 0.05% to about 2%, from about 0.05% to about 1%, or from about 0.1% to about 1%, such as about 0.05% or 1%.

The protease inhibitors in the sensor binding buffer can be inhibitors capable of inhibiting protease activity in a biological sample, such as in saliva. Examples of protease inhibitors that are suitable for use in the sensor binding buffer are commercially available, such as cOmplete protease inhibitor cocktail from Sigma Aldrich and SigmaFast protease inhibitor tablets. The concentration of the protease inhibitors in the sensor binding buffer can follow the by the manufacture recommendations, or can be 2X, 3X, 4X, 5X, or 10X the concentration recommended by the manufacture, such as 4X the concentration recommended by the manufacture.

The sensor binding buffer optionally contains one or more antimicrobial agents and/or one or more blocking agents. The blocking agents that are suitable for use in the sensor binding buffer can be any of the blocking agents described above, such as BSA. The total concentration of the one or more blocking agents (by volume) in the sensor binding buffer can be in a range from about 0.01% to about 5%, from about 0.05% to about 5%, from about 0.05% to about 2%, from about 0.05% to about 1%, from about 0.1% to about 1%, or from about 0.1% to about 0.5%, such as about 0.1% or 0.5%. The total concentration of the one or more detergents (by weight) in the sensor binding buffer can be in a range from about 0.01% to about 5%, from about 0.05% to about 5%, from about 0.05% to about 2%, from about 0.05% to about 1%, from about 0.1% to about 1%, or from about 0.1% to about 0.5%, such as about 0.1% or 0.5%.

The antimicrobial agents in the sensor binding buffer can extend the shelf-life of the buffer solution by preventing microbial growth, such as sodium azide. The total concentration of the one or more antimicrobial agents (by volume) in the sensor binding buffer can be in a range from about 0.002% to about 0.2%, from about 0.005% to about 0.2%, from about 0.01% to about 0.2%, from about 0.01% to about 0.1%, or from about 0.01% to about 0.05%, such as about 0.02%. The total concentration of the one or more detergents (by weight) in the sensor binding buffer can be in a range from about 0.002% to about 0.2%, from about 0.005% to about 0.2%, from about 0.01% to about 0.2%, from about 0.01% to about 0.1%, or from about 0.01% to about 0.05%, such as about 0.02%.

In some embodiments, the sensor binding buffer is a non-lysis buffer containing a detergent that can keep the virus of interest intact. Such non-lysis buffers can contain one or more the above-described buffering agents, one or more the above-described salts, Tween, one or more the above-described protease inhibitors, and optionally one or more the above-described antimicrobial agents. Each of the components in the non-lysis buffers can have any of the concentration ranges described above for the respective component. For example, the non-lysis buffer contains HEPES at a concentration in a range from about 10mM to about 50 mM, an above-described salt at a concentration in a range from about 100 mM to about 300 mM, Tween at a concentration in a range from about 0.01% to about 1% (by volume or weight), a protease inhibitor cocktail or tablet, and an above-described antimicrobial agent at a concentration in a range from about 0.01% to about 0.05% (by volume or weight). An exemplary non-lysis buffer contains 100 mM HEPES (pH 7.4), 150 mM NaCl, 0.05% vol/vol Tween-20, 0.02% wt/vol $NaN_3$ and 4x cOmplete, which can be used to dilute the biological sample to achieve any of the dilution factors described above, such as a 1:1 dilution in the buffer, prior to any one of steps (i)-(vi), particularly prior to step (vi).

In some embodiments, the sensor binding buffer is a lysis buffer that can dissolve the virus membrane in the biological sample, thereby inactivate the virus and dissolve the target protein from the virus membrane for detection. Such lysis buffers can contain one or more the above-described buffering agents, one or more the above-described salts, one or more detergent that can dissolve virus membrane and thus deactivate the virus, one or more the above-described protease inhibitors, one or more the above-described blocking agent, and optionally one or more the above-described antimicrobial agents. Examples of detergent that are suitable for use in the lysis buffers include, but are not limited to, Triton-X100, Nonidet P40, IGEPAL CA-630, Brij-35, Brij-58, and Triton X-114, and a combination thereof. Each of the components in the lysis buffers can have any of the concentration ranges as described above for the respective component. For example, the lysis buffer contains Tris or HEPES or phosphate salts at a concentration in a range from about 10mM to about 100 mM, an above-described salt at a concentration in a range from about 200 mM to about 1 M, a virus-inactivating detergent as described above, such as Nonidet P40 or TritonX-100, at a concentration in a range from about 0.5% to about 2% (by volume or weight), an above-described protease inhibitor cocktail or tablet, an above-described blocking agent, such as BSA, at a concentration in a range from about 0.05% to 1% (by volume or weight), and optionally an above-described antimicrobial agent at a concentration in a range from about 0.01% to about 0.05% (by volume or weight). Optionally, the lysis buffer further includes an RNA digestive enzyme, such as an RNAse A enzyme, at a concentration in a range from about 1 mg/L to about 50 mg/L, from about 1 mg/L to about 20 mg/L, from about 2 mg/L to about 25 mg/L, from about 2 mg/L to about 20 mg/L, or from about 1 mg/L to about 10 mg/L, such as about 5 mg/L of the lysis buffer. Other examples of RNA digestive enzymes that are suitable for use in the lysis buffer include, but are not limited to, RNase H, RNase III, RNase L, RNase P, RNase PhyM, RNase T1, RNase T2, RNase U2, RNase V, RNase E, and RNase G, and a combination thereof. Specific examples of lysis buffer suitable for use in the disclosed method are as follows:

Lysis buffer A: 50 mM Tris pH 7.4, 250 mM NaCl, 1% Nonidet P40 (NP40), 0.02% $NaN_3$, 0.5% BSA, and 4x protease inhibitor cOmplete with EDTA;

Lysis buffer B: 20 mM HEPES pH 7.4, 500 mM NaCl, 1% TritonX-100, 0.02% $NaN_3$, 0.1% BSA, and 4x protease inhibitor cOmplete with EDTA; and

Lysis buffer B': 20 mM HEPES pH 7.4, 500 mM NaCl, 1% TritonX-100, 0.02% $NaN_3$, 0.1% BSA, 4x protease inhibitor cOmplete with EDTA, and RNAse A enzyme added to a final concentration of 5 mg/L.

For example, the above-described exemplary method includes a step of diluting a biological sample, such as saliva, using a lysis buffer, such as Lysis buffer A, B, or B' described above, to achieve any of the dilution factors described above, such as a 1:3 dilution in the lysis buffer, prior to any one of steps (i)-(vi), particularly prior to step (vi).

In some embodiments, methods using a lysis buffer, such as Lysis buffer A, B, or B', particularly Lysis buffer B or B', can reduce the noise level for measurements and thereby increase the sensitivity of the sensor, compared to methods using a sensor binding buffer that leaves the virus intact upon dilution, such as a buffer containing 100 mM HEPES (pH 7.4), 150 mM NaCl, 0.05% vol/vol Tween-20, 0.02% wt/vol $NaN_3$ and 4x cOmplete.

The biological sample is incubated with the gate electrode for a time period sufficient to allow binding between the analytes in the biological sample and the biorecognition element (e.g. nanobody) modified on the gate electrode surface. Typically, the incubation time period is up to 60 minutes, up to 50 minutes, up to 40 minutes, up to 30 minutes, up to 20 minutes, or up to 10 minutes. For example, the biological sample is incubated with the gate electrode for about 10 minutes to allow binding between the analytes in the biological sample and the nanobody modified on the gate electrode surface. The incubation may be performed statically, under shaking, or with up and down pipetting, and in some embodiments, for about 3- 5 minutes. In some preferred embodiments, the incubation is performed with pipetting to facilitate binding between the analytes in the biological sample and the biorecognition element (e.g. nanobody) modified on the gate electrode surface and thereby reduce the time needed for the incubation. When pipetting is performed during incubation, the biological sample is mixed up and down with a pipette for a time period in a range from 5 seconds to 1 minute, from 5 seconds to 50 seconds, from 5 seconds to 40 seconds, from 10 seconds to 1 minute, from 10 seconds to 50 seconds, from 10 seconds to 40 seconds, or from 20 seconds to 1 minute, such as about 30 seconds and the pipetting is repeated for at least one time or at least two times. For example, during a 10-minute incubation, the biological sample is mixed up and down with pipetting for about 30 seconds and the pipetting is performed for a total of 3 times (i.e. 3×30-second pipetting or a 30-second pipetting every 3 minutes during the 10-minute incubation period).

Following this incubation step (v), the gate electrode is rinsed with a rinsing buffer to remove any unbounded analytes, for example, it can be rinsed for up to 1 min, 30 secs, 15 secs etc., and in some embodiments, it is rinsed by dipping in (for about 15 secs) and out of the rinsing buffer, and repeating this, twice. The rinsed gate electrode is then brought back in contact with the electrolyte solution prior to step (vii) measuring a second $I_D$. The rinsing buffer may be any electrolyte solution as described above, such as PBS.

Optionally, the above-described method includes a step of exposing the gate electrode surface to a glycine solution at a pH less than 5, less than 4, or less than 3, such as about 2, following step (vii). The acidic glycine solution can disrupt the binding between the analyte and the recognition element (e.g. the analyte/nanobody binding) and thus regenerate the OECT sensor for further sample measurements. For example, following the regeneration step, the $I_D$ generated from the OECT is comparable to the $I_0$ (i.e. the difference between the $I_D$ obtained after regeneration and $I_0$ is less than about 10%).

The exemplified methods can be used to make OECT devices containing a nanobody whose binding partner is any analyte of interest, for example an antigen from SAR-CoV-2, such as the spike protein. Specific nanobodies against SARS-CoV-2 Spike protein (S protein) or, more specifically, against the Receptor Binding Domain (RBD) within the S protein are available. Nanobodies are ultra-compact, robust single-chain antibody fragments engineered from camelid antibodies. Two nanobodies that were originally targeting SARS-1 RBD have very recently been shown to also recognize SARS-CoV-2 RBD with high affinity[9]. There are other nanobodies, engineered from a human antibody framework, that were specifically developed for SARS-CoV-2 Wu, et al., Fully human single-domain antibodies against SARS-CoV-2. bioRxiv 2020. The human ACE2 receptor protein, to which the virus binds with high affinity, can be used an alternative recognition module. The subject can be symptomatic or asymptomatic. In some embodiments, the subject has been exposed to the virus, however, the subject may have no known exposure to the virus.

Particular preferred embodiments of the device, methods of making and methods of using are exemplified below.

## EXAMPLES

### 1. Materials and Methods

#### Materials

Sodium chloride, Tween-20, glycerol, HEPES, bovine serum albumin (BSA), (3-glycidyloxypropyl) trimethoxysilane (GOPS), sodium dodecylbenzenesulfonate (DBSA), ethylene glycol (EG), 1,6-hexanedithiol (HDT), 1,3-propanedithiol (PDT), 3-mercaptopropionic acid (MPA), human serum, and PBS (pH 7.4) were purchased from Sigma-Aldrich and used as received. Poly(3,4-ethylenedithiophene) doped with poly(styrene sulfonate), PEDOT:PSS, (PH1000) was received from Heraeus. All aqueous solutions were prepared with ultrapure water (Millipore Milli-Q). p(g0T2-g6T2) was synthesized according to a procedure reported previously[35]

Protein purification materials: Agar, LB broth, 2xYT Broth, Kanamycin, Glucose, Isopropyl β-D-1-thiogalactopyranoside (IPTG), BugBuster (Novagen), cOmplete Protease Inhibitor mix (Sigma), Benzonase (Novagen), Egg-Lysozyme (Fluka), Tris(2 carboxyethyl)phosphine (TCEP), Tris(hydroxymethyl)aminomethane hydrochloride (Tris-HCl), Imidazole, Glycerol, Dithiothreitol (DTT), Ethylenediaminetetraacetic acid (EDTA), D-Desthiobiotin, 10K Amicon ultra spin concentrators (Milipore).

Purification columns and SPR materials were purchased from GE Healthcare: HisTrap-HP 5 ml, StrepTrap-HP 5 ml, Superdex75 16/600, Biacore NTA SPR sensor chips (BR100034). Maleimide -SpyTag peptide (Genscript, peptide synthesis).

Viral target proteins were purchased from Sino Biological: SARS-CoV RBD (40150-V08B2), SARS-CoV-2 RBD (40592-V08H), SARS-CoV-2 S1 (40591-V08B1), MERS-CoV S1 (40069-V08H). Universal Transport Medium (UTM) Kit was purchased from Noble Biosciences, Inc.

#### Fabrication of the organic electrochemical transistor (OECT) and the gate electrode

OECTs were microfabricated on glass substrates based on established protocols using standard photolithography and Parylene-C peel-off techniques[38,39]. The process starts with the first layer of photoresist (AZ5214) spin coated and exposed to UV light using contact aligner to create Au electrodes and interconnection pads. The photoresist patterns were generated with AZ 726 MIF developer, followed with metal sputtering of 10 nm of Cr and 100 nm of Au and a standard lift-off process using hot DMSO. Next, the second layer of photoresist AZ9260 was coated on the substrates and developed using AZ developer. A parylene-C layer was deposited to insulate the gold interconnects. The OECT channel was patterned by reactive ion etching (RIE) and using a second layer of a Parylene C which was peeled-off to yield a length

of 10 $\mu$m in and width of 100 $\mu$m. The aqueous dispersion of poly(3,4-ethylenedioxythiophene) doped with poly(styrene sulfonate) (PEDOT:PSS) containing EG (5 vol%), DBSA (0.25 vol%), and GOPS (1 wt%) was sonicated for 30 min and then spin coated (3000 rpm, 45s) on the substrates leading to a film thickness of about 160 nm. The PEDOT:PSS OECTs were annealed at 140 °C for 1 h to activate GOPS and avoid dissolution of the polymer film in aqueous medium. P(g0T2-g6T2) films were spin coated (800 rpm, 45 s) from a chloroform solution (5 g/L) on the substrates to yield a film thickness of about 70 nm in the channel. All devices were rinsed with DI water before use.

For the fabrication of the gate electrode, 175 $\mu$m-thick Kapton (polyimide) substrates were used. The Kapton was first cut with a $CO_2$ laser (Universal Laser Systems - PLS6.75) into a circular geometry that defined the sensor active area (the final diameter used in sensors is 0.8 $\mu$m); then the substrates were sonicated, first in isopropyl alcohol and then in deionized (DI) water for 30 min each. 10 $\mu$m of chromium or titanium was sputtered and 100 $\mu$m of Au on these cleaned substrates. Before the functionalization, the electrodes were electrochemically cleaned in 10 mM sulfuric acid ($H_2SO_4$) using cyclic voltammetry (CV). 10 CV cycles with a potential between -0.2 V to 1.2 V were applied at scan rate 100 mV s$^{-1}$.

### Biofunctionalization of gate electrodes

The chemical SAM solution was prepared in absolute ethanol containing 1 mM of HDT, as described by others[40]. The Au electrodes were immersed in this solution for one hour[30]. Electrodes were rinsed with absolute ethanol and dried under a $N_2$ stream. Electrodes were then incubated for one hour with the synthetic Maleimide-modified SpyTag peptide (0.1 mg/mL) in PBS, and washed in PBS. Electrodes were then incubated for one hour with Nanobody-SpyCatcher fusion protein (anti-GFP, anti-SARS-CoV-1, or anti-MERS-CoV) diluted to 50 $\mu$M in sensor binding buffer (100 mM HEPES pH 7.4, 150 mM NaCl, 0.05% v/v Tween-20, 0.02% w/v $NaN_3$, 0.1% w/v BSA) and then rinsed with PBS. Nanobody-functionalized gate electrodes were stored for up to one week at 4 °C in the sensor binding buffer until use.

### X-ray photoelectron spectroscopy (XPS)

XPS analysis was performed using a KRATOS Axis SUPRA instrument equipped with a monochromatic Al K$\alpha$ X-ray source (1468.6 eV). The source was operated at 75 W under UHV condition (~$10^{-9}$ mbar). The spectra were recorded in a hybrid mode using electrostatic and magnetic lenses and an aperture slot of 300 by 700 $\mu$m. The survey and high-resolution spectra were acquired at fixed analyzer pass energies of 80 eV and 20 eV, respectively. The samples were mounted in a floating mode to avoid differential charging. The spectra were then acquired under charge neutralization conditions. The spectra was calibrated to reference of C 1s at 284.8 eV and deconvoluted it using Gaussian and Lorentzian methods. The Tougaard method was used for the background subtraction.

*Quartz crystal microbalance with dissipation monitoring (QCM-D)* QCM-D measurements were conducted using a Q-sense analyzer (QE401, Biolin Scientific). The piezoelectrically active gold sensor (0.7854 cm$^2$) was firstly modified with HDT SAM under the same reaction conditions and then mounted into the QCM -D setup. First, the QCM-D signals, including the change in frequency ($\triangle f$) and dissipation ($\triangle D$) were stabilized in PBS.

Second, the peptide solution (0.1 mg/mL SpyTag peptide in PBS) was injected into the chamber with a flow rate of 100 $\mu$L/min controlled by a peristaltic pump. After ensuring that the sensor was fully covered with the solution, the pump was stopped for an hour and rinsed the sensor surface with PBS injected to the system for 15 min. The same procedure was employed to treat the surface with SpyCatcher-linked nanobody solution (50 $\mu$M in the binding buffer). To quantify the mass accumulating on the sensor ($\triangle m$) during the functionalization steps, the Sauerbrey equation[41] was used:

$$\Delta m = \frac{-17.7}{n} \Delta f_n \tag{1}$$

where *n* is the overtone number selected for the calculations and -17.7 is a constant calculated based on the resonant frequency, active area, density and shear modulus of the quartz crystal sensor. The mass of the binding molecules on the sensor surface was then estimated using their molecular weight. The (Sauerbrey) thickness (nm), *d*s, was calculated as:

$$ds = \frac{\Delta m}{Ar100}$$

where $\Delta m$ is the change in mass (ng), A is the area (cm$^2$) and *r* is the estimated density of the layer (g cm$^{-3}$; assumed to be the same as water, which is taken as 1 g cm$^{-3}$).

*Electrochemical characterization*

The electrochemical characteristics of the gold electrode were investigated before and after the formation of Chem-SAM and Bio-SAM using electrochemical impedance spectroscopy (EIS) and cyclic voltammetry (CV) performed in a three-electrode setup using a potentiostat (Autolab PGstat128N, MetroOhm). A platinum wire was used and an Ag/AgCl electrode as the counter electrode and reference electrodes, respectively, while the gold electrode was connected as the working electrode. All measurements were carried out in 5 mL of 10 mM PBS (pH 7.4) containing 10 mM of $[Fe(CN)_6]^{3-/4-}$. For CV measurements, the potential window was between -0.2 V and 0.5 V and the scan rate was 100 mV s$^{-1}$. Electrochemical impedance spectra were recorded at a DC voltage of 0 V versus open circuit potential ($V_{oc}$) and an AC modulation of 10 mV over a frequency range of 0.1-100000 Hz. For the analysis of GFP nanobody-functionalized electrodes, the electrodes were incubated with GFP solutions for 10 min and washed in 10 mM PBS before the measurements. The data was analyzed using Nova software.

· *Proteins and Peptides*

Nanobody-SpyCatcher fusion proteins were designed based on available structures (nanobody: PDB 4PFE[42]; SpyCatcher: PDB 4MLI[43]) with the nanobody placed at the N-terminal end of the fusion protein in order to orient the common VHH target-binding interface towards the bulk solution, away from the sensing surface. Protein sequences were reverse-translated and codon-optimized for expression in *E. coli* with an in-house Python script based on DNAChisel[44]. Plasmids for protein expression were gene synthesized by Twist Bioscience (U.S.A.) or BioBasic (Canada) in a customized expression vector pJE411c with kanamycin resistance and modified with an RBS insulator (BCD2) cassette[45] for improved translation initiation. Plasmids were transformed into *E. coli* BL21 (DE3) and starter cultures were inoculated overnight from a single colony. 1L production cultures in 2xYT medium with 50 mg/L kanamycin and 1% glucose were inoculated 1:100, grown at 37°C and 250 rpm to $OD_{600}$ 0.8, induced with 0.5 mM IPTG and incubated shaking for 18 h at 25°C. Cells were harvested by centrifugation for 10 min at 6,000 *g* at 4 °C, washed once with cold PBS, resuspended in lysis buffer [25 mM Tris-HCl (pH 7.4), 500 mM NaCl, 10 mM Imidazole, 10% glycerol, SigmaFAST protease inhibitor, 25 U/ml Benzonase HC (Milipore), 2 mM DTT], and homogenized with a cell disruptor (Constant Systems, UK). Earlier purifications of mCherry, GFPnanobody and msfGFP used different lysis methods (chemical lysis with BugBuster (Novagen) for mCherry, sonication for the other two). Lysates were cleared by centrifugation at 87,000 g for 45 min, the supernatant filtered through Miracloth tissue (Milipore) and subjected to affinity chromatography on an Äkta FPLC (GE Healthcare) using either StrepTrap HP or HisTrap HP columns (GE Healthcare), depending on the purification tag. The Strep-tag binding buffer was 100 mM Tris-HCl (pH 8), 150 mM NaCl, 1 mM EDTA, 5% glycerol, 0.5 mM TCEP and elution was performed with 2.5 mM Desthiobiotin in binding buffer. The His-tag binding buffer was 25 mM Tris-HCl pH 7.4, 500 mM NaCl, 10 mM Imidazole, 10% Glycerol, 2 mM DTT and elution was performed with a four-step imidazole gradient up to 0.5M. Fractions were pooled and concentrated using 10K Amicon ultra (Milipore) followed by gel filtration on a Superdex75 16/600 column (GE Healthcare) into 20 mM HEPES pH 7.5, 300 mM NaCl, 10% Glycerol, 50 μM EDTA. After spin-concentration, aliquots were snap-frozen in liquid nitrogen and stored at -80 °C. Protein purity and quality was monitored by SDS-PAGE as well as SEC-MALS on a Dawn Heleos II & OptiLab T-rEx (Wyatt, U.S.A.). Protein concentrations were determined on a Nanodrop spectrophotometer by absorbance at 280 nm using sequence-specific extinction coefficients[47].

SARS-CoV-1/2 and MERS target proteins, expressed and purified from HEK293 or insect cell culture, were received lyophilized from Sino Biological (China) and dissolved to a standard concentration of 0.25 mg/ml as per manufacturer instructions, then aliquoted, snap-frozen in liquid nitrogen and stored at -80 °C. N-terminally Maleimide-labelled SpyTag peptide was synthesized by GenScript Biotech (Singapore), received lyophilized, dissolved in PBS and stored at -20 °C.

*Protein Dilutions*

Target and non-target proteins were thawed on ice and centrifuged at 15.000 rpm at 4 °C for 30 to 45 min in order to remove any potential aggregates (although no aggregation was observed). Sino Biologicals proteins were then used as-is for the preparation of a dilution series starting at 320 or 640 nM. Equivalent dilutions of the Sino Biologicals storage buffer by itself did not give any sensor response. Weak background sensor signals were recorded from dilutions of DTT. In-house proteins were therefore stored or exchanged into DTT-free buffer before use. The higher-concentrated proteins from in-house production were first diluted to intermediate concentrations that could still be validated and corrected spectrophotometrically. Protein dilutions were prepared in standard sensor binding buffer (100 mM HEPES pH 7.4, 150 mM NaCl, 0.05% v/v Tween-20, 0.02% w/v NaN$_3$, 0.1% w/v BSA) which was modified for saliva measurements to include cOmplete protease inhibitor cocktail with EDTA (Sigma) at 4 times the manufacturer-recommended concentration (giving a two-fold concentration in the final 1:1 mixture with saliva). BSA was not included in this saliva buffer. For measurements in the regular binding buffer, 4-fold dilution series were prepared in 96-well microplates over 23 steps starting from 320 nM. For measurements in serum, saliva and UTM, target protein dilution series were prepared in the appropriate buffer (standard or saliva binding buffer) starting from 640 nM so that final concentrations were identical after 1:1 mixture with serum, saliva or UTM.

*SPR Measurements*

Surface plasmon resonance measurements were performed on a Biacore T200 instrument using Nickel-NTA sensor chips (GE Healthcare) and a modified running buffer (20 mM HEPES pH 7.4, 150 mM NaCl, 0.05% Tween-20, 0.02% w/v NaN$_3$, 50 $\mu$M EDTA, prepared at room temperature and filtered) mirroring the sensor binding buffer. All analyte proteins were desalted into the running buffer (HiPrep 26/10 column, GE Healthcare) before the preparation of dilution series in the same running buffer. The GFP nanobody: GFP interaction was measured with the His$_{10}$-tagged nanobody immobilized (ligand) and msfGFP in solution. For the remaining measurements, since all viral target proteins arrived with a non-cleavable His-tag, target proteins were immobilized instead of the nanobody. All ligand proteins gave stable immobilization responses with minimal signal loss over time. His tags were removed from nanobody-spyCatcher proteins by overnight cleavage with excess 3C protease (produced in-house), followed by gel filtration on a Superdex200 Increase 10/300GL column. All ligand proteins were immobilized to equal loading levels of around 100 RU at 10 $\mu$l/min flow rates. Binding and unbinding experiments were run at a flow rate of 80 $\mu$l/min. Two replicates were prepared of each dilution series and measured in the course of the same experiment. Between each measurement cycle, the sensor surface was regenerated with 0.35 M EDTA in running buffer and re-charged with 0.5 mM NiSO$_4$ in water. Biacore results were analyzed with the manufacturer analysis software version 2.1 following standard procedures (double-subtraction of reference channel and buffer injection signal) applying a 1:1 binding model and simultaneous curve-fitting. SARS-CoV-2 RBD results were instead fit a heterologous ligand binding model.

*Fluorescence imaging*

The GFP nanobody functionalized gold electrodes before and after its incubation with a GFP solution together with the negative controls (the biofunctional electrode before and after incubation with mCherry) were imaged using fluorescence microscopy. Imaging was performed on a DMI8 inverted fluorescence microscope (Leica Microsystems) coupled with a pE-4000 fluorescence illumination system (CoolLED), and the images were processed using ImageJ software. Electrodes were placed between two cover slips in the presence of PBS in order to keep the flexible material in plane and in focus. The presence or absence of BSA was studied by adding fluorescently labeled BSA (albumin-fluorescein isothiocyanate conjugated bovine protein) to the nanobody-SpyCatcher solution during functionalization of the sensing surface. The capture of GFP was examined by imaging already functionalized gold electrodes before and after incubation with GFP or control solutions. Pictures were recorded on a fluorescent inverted microscope DMI8 (Leica Microsystem) coupled with pE-4000 fluorescence illumination system (CoolLED).

*OECT characterization and sensor operation*

A Keithley source meter was used to apply drain ($V_D$) and gate voltages ($V_G$) and obtain gate and channel currents ($I_G$ and $I_D$) in ambient conditions. A PDMS well was glued on top of the channels and filled with 75 $\mu$L of 10 mM PBS. The steady-state transistor characteristics were obtained by measuring $I_D$ vs. $V_D$ at various $V_G$, for PEDOT:PSS applied between -0.6 V to 0.6 V with 0.1 V step (2.5 V/s) and for p(g0T2-g6T2) from 0.2 V to -0.6 V with 0.05 V step (1.25 V/s). $V_D$ was swept from 0 V to - 0.6 V. A channel was first chosen and obtained its transfer curve ($I_D$ vs. $V_G$) in PBS using the nanobody-functionalized gate electrode incubated for 10 min in the buffer solution (buffer, saliva, UTM or serum, in the absence of target proteins). The currents obtained were used as the baseline signal ($I_0$). The same gate electrode was then incubated with 5 $\mu$L of the solution (buffer, saliva, UTM or serum) that contains the protein target for 10 min (pipetting 30 s every 3 min). The electrode was washed thoroughly with PBS to remove any unbound proteins. To obtain calibration curves, various concentrations of the target protein were prepared and collected device data starting from the most diluted one. The normalized response (NR) of the sensor was calculated according to the following equation:

$$NR = \frac{|I_D - I_0|}{I_0} \qquad (2)$$

where $I_D$ is the current response of the sensor to an analyte solution that the gate was exposed to.

According to IUPAC, the LOD was calculated as the concentration leading to a response that equals to the average of the noise level plus three times the noise standard deviation[14,46]. The level-of-noise ( $\frac{\Delta I}{I_0} \pm 3\delta$ ) was taken from the relative current variation in negative control sensors.

$$LOD = \frac{(\frac{\Delta I}{I_0} \pm 3\delta) - a}{b} \qquad (3)$$

where $\frac{\Delta I}{I_0}$ is the average response of the blank sample, $\delta$ is the relative standard deviation, $a$ and $b$ are the intercept and slope of the calibration curve, respectively.

### *Clinical Sample Preparation and Testing*

The clinical samples (saliva samples and nasopharyngeal swabs) used in this study (**FIG. 5A-5E**) were collected from subjects as part of registered protocols approved by the Institutional Review Board of King Faisal Specialist Hospital & Research Center and KAUST Institutional Biosafety and Bioethics Committee (IBEC). All volunteers provided signed consent to participate in the study. Nasopharyngeal swabs collected from COVID-19 patients and healthy subjects were stored in UTM and saliva was collected inside empty tubes. The samples were stored at 4 °C and tested within 1-2 days after collection using the same protocol described above under "protein dilutions". Saliva was collected in 50-ml tubes and always measured the same day. The saliva samples used for original sensor characterization with recombinant proteins (Fig. 4A-4G) were self-collected in the morning before food or tooth brushing, and stored in aliquots at -20 °C. The volunteers provided signed consent to participate in the study and provide saliva. The buffer composition for measurements in clinical samples was modified in the course of the study: nasal swabs were measured after 1:1 dilution in the original buffer used for the saliva spike-in experiments (100 mM HEPES (pH 7.4), 150 mM NaCl, 0.05% vol/vol Tween-20, 0.02% wt/vol NaN3 and 4x cOmplete). Raw saliva samples were measured after 1:3 dilution in a virus-inactivating lysis buffer, herein, lysis buffer A (50 mM Tris (pH 7.4), 250 mM NaCl, 1% Nonidet P-40, 0.02% NaN3, 0.5% BSA and 4x cOmplete). Human serum from human male AB plasma was purchased from Sigma-Aldrich and used as-received. All protocols and procedures involving human saliva, nasopharyngeal swabs and serum were approved by the KAUST IBEC under approval number 18IBEC11 and 20IBEC25, NCBE, KSA registration number HAP-02-J-042.

### **Lysis buffer modifications**

Additional lysis buffers were prepared having the following components. Lysis buffer B: 20 mM HEPES pH 7.4; 500 mM NaCl; 1% TritonX-100; 0.02% $NaN_3$; 0.1% BSA; 4x protease inhibitor cOmplete with EDTA. Lysis buffer B' was prepared with the same components as Lysis buffer B, however, RNAse A enzyme added to final concentration of 5 mg / L.

### **1.8 Construct design**

Sensor protein modules are described in Tables 1 and 2

## Table 1. Protein constructs

| Construct Name | Description | Molecular weight (kDa) | Isoelectric Point | E(280 nm) M$^{-1}$ cm$^{-1}$ |
|---|---|---|---|---|
| Peptide | MCA-SpyTag | 1.76 | 8.24 | 1,490 |
| GFP Nanobody | GFPnanobody-8aa-SpyCatcher-His10 | 28.39 | 5.81 | 38,390 |
| SARS-CoV nanobody (VHH72) | SARS 1 / 2 nanobody(VHH72)-SpyCatcher-3C-His8 | 29.05 | 5.2 | 46,870 |
| MERS-CoV nanobody (VHH04) | MERS nanobody(VHH04)-SpyCatcher-3C-His10 | 28.90 | 5.77 | 41,370 |
| Target (msfGFP)* | snoopTag-msfGFP-3C-TwinStrep | 33.59 | 6.06 | 29,910 |
| Negative control (mCherry)** | FRB-24-mCherry-Wp3-TwinStrep | 42.91 | 6.1 | 74,830 |

| | | | | |
|---|---|---|---|---|
| SARS-CoV-1 RBD | (Sino Biological 40150-V08B2); Spike Receptor Binding Domain residues R306 - F527 His tagged | 26.51 | 8.51 | 40,340 |
| SARS-CoV-2 RBD | (Sino Biological 40592-V08H); Spike Receptor Binding Domain residues R319 - F541 His tagged | 26.5 | 8.9 | 33,350 |
| SARS-CoV-2 S1 | (Sino Biological 40591-V08B1); Spike Protein S1 subunit residues 1 - 685(R) His tagged | 76.45 | 8.28 | 90,650 |
| MERS-CoV S1 | (Sino Biological 40069-V08H); Spike Protein S1 subunit residues 1 - 725 His tagged | 79.9 | 6.19 | 100,510 |

* **E(485 nm) M$^{-1}$ cm$^{-1}$** = 83,300; ** **E(587 nm) M$^{-1}$ cm$^{-1}$** = 60,000

**Table 2. Protein sequences**

| Construct Name | Description | Sequence |
|---|---|---|
| Peptide | MCA-SpyTag | SGSG*AHIVMVDAYKPTK* (SEQ ID NO:1) (linker sequence is underlined) |
| GFP Nanobody | GFPnanobody-8aa-Spy-Catcher-3C cleavage site -His$_{10}$ | MASQVQLVESGGALVQPGGSLRLSCAASGFPVNRYSMRW YRQAPGKEREWVAGMSSAGDRSSYEDSVKGRFTISRDDAR NTVYLQMNSLKPEDTAVYYCNVNVGFEYWGQGTQVTVSS K*GSGSGSGS*VDTLSGLSSEQGQSGDMTIEEDSATHIKFSKRD EDGKELAGATMELRDSSGKTISTWISDGQVKDFYLYPGKY TFVETAAPDGYEVATAITFTVNEQGQVTVNGKATKGDAHI SGLEVLFQGPTG**HHHHHHHHHH** (SEQ ID NO:2) |
| SARS-CoV-1/2 nanobody (VHH72) | VHH72-8aa-spyCatcher-3C cleavage site-His$_{8}$ | MTGQVQLQESGGGLVQAGGSLRLSCAASGRTFSEYAMGW FRQAPGKEREFVATISWSGGSTYYTDSVKGRFTISRDNAKN TVYLQMNSLKPDDTAVYYCAAAGLGTVVSEWDYDYDYW GQGTQVTVSSGS*GSGSGSGS*VDTLSGLSSEQGQSGDMTIEE DSATHIKFSKRDEDGKELAGATMELRDSSGKTISTWISDGQ VKDFYLYPGKYTFVETAAPDGYEVATAITFTVNEQGQVTV NGKATKGDAHISGLEVLFQGPTG**HHHHHHHH** (SEQ ID NO: 15) |
| MERS-CoV na-nobody (VHH04) | VHH04-8aa-spyCatcher-3C cleavage site -His10 | MTGEVQLQESGGGSVQAGGSLRLSCEASGTISSMYCMGWF RQAPGKEREGVALFNRSTGVEYYRASVKGRFTISHDNAKN TVYLQMNSLKLEDTAVYYCAAGPTCGGWYPGLYNYWGQ GTQVTVSS*GSGSGSGS*VDTLSGLSSEQGQSGDMTIEEDSAT HIKFSKRDEDGKELAGATMELRDSSGKTISTWISDGQVKDF YLYPGKYTFVETAAPDGYEVATAITFTVNEQGQVTVNGKA TKGDAHISGLEVLFQGPTG**HHHHHHHHHH** (SEQ ID NO: 17) |
| Target (msfGFP) | snoopTag-msfGFP-3C cleavage site - TwinStrep | MASKLGDIEFIKVNKGSGSGSGSVSKGEELFTGVVPILVELD GDVNGHKFSVRGEGEGDATNGKLTLKFICTTGKLPVPWPT LVTTLTYGVQCFSRYPDHMKQHDFFKSAMPEGYVQERTIS FKDDGTYKTRAEVKFEGDTLVNRIELKGIDFKEDGNILGHK LEYNFNSHNVYITADKQKNGIKANFKIRHNVEDGSVQLAD HYQQNTPIGDGPVLLPDNHYLSTQSKLSKDPNEKRDHMVL LEFVTAAGITHGMDELYKTGSTGLEVLFQGPTGSAWSHPQ FEKGTGSGTGSGTGSWSHPQFEK (SEQ ID NO:3) |

(continued)

| Construct Name | Description | Sequence |
|---|---|---|
| Negative control (mCherry) | FRB-24-mCherry-Wp3-TwinStrep | MTGILWHEMWHEGLEEASRLYFGERNVKGMFEVLEPLHA MMERGPQTLKETSFNQAYGRDLMEAQEWCRKYMKSGNV KDLLQAWDLYYHVFRRISKTGGSGSGSGSGSGSGSGSGSGS TGVSKGEEDNSAIIKEFMRFKVHMEGSVNGHEFEIEGEGEG RPYEGTQTAKLKVTKGGPLPFAWDILSPQFMYGSKAYVKH PADIPDYLKLSFPEGFKWERVMNFEDGGVVTVTQDSSLQD GEFIYKVKLRGTNFPSDGPVMQKKTMGWEASSERMYPED GALKGEIKQRLKLKDGGHYDAEVKTTYKAKKPVQLPGAY NVNIKLDITSHNEDYTIVEQYERAEGRHSTGGSGPLPPYTSA WSHPQFEKGGGSGGGSGGGSWSHPQFEK (SEQ ID NO:4) |

## 2. Results and discussion

There are hardly any reports of label-free OECT immunosensors[18]. Presumably, the large size of regular antibodies and lack of control over their immobilization density or orientation has impeded progress in this area[19].

Therefore, the present studies devised an alternative functionalization strategy that relies on the self-assembling binding partners, exemplified herein by SpyTag/SpyCatcher system[20], to covalently capture chemically unmodified recombinant proteins in a defined orientation at very high physical density on the sensing electrode. The protein design disclosed herein maximizes the molecular flexibility of these nanobodies in order to create an extended recognition layer that can capture and kinetically trap antigens at ultra-low concentrations on a disposable gate electrode. The sensor performance was further enhanced by optimizing the transistor geometry and the biochemical blocking and incubation strategies. Last but not least, a novel organic semiconductor was used which improved signal strength and stability and reduced the power requirements as compared to the classic OECT channel material, poly(3,4-ethylenedioxythiophene) doped with poly(styrene sulfonate) (PEDOT:PSS). The development of this platform (**FIGs. 1A-1C**) is described as well as its validation with different protein targets (green fluorescent protein (GFP), SARS-CoV, and MERS-CoV spike proteins) in human saliva and serum. The platform was demonstrated to reach detection limits in the femto- to attomolar range for the target molecules using two types of organic electronic materials cast in the microscale channel. Initial tests with clinical nasopharyngeal swab samples and saliva samples indicate a SARS-CoV-2 detection performance comparable to RT-PCR.

### *Biofunctionalization*

Nanobodies (and modified versions thereof) can be efficiently expressed in *Escherichia coli*. A recombinant protein was designed where the well-characterized anti-GFP nanobody[26] is fused, through a flexible peptide linker, to a SpyCatcher domain. The SpyCatcher domain is specifically recognized by a short SpyTag peptide, triggering the autocatalytic formation of a covalent isopeptide bond of very high stability between both of them. Originally engineered from a bacterial adhesion protein[20], the SpyTag/SpyCatcher protein conjugation system has been used for several applications[27], but not yet for FET nor OECT biosensors. The only related application of a nanobody-SpyTag fusion was a passive, impedance-based sensor for the detection of microalgae[28] which relied on the chemical immobilization of separate SpyCatcher proteins as a capture reagent.

The disclosed design instead wanted to avoid any steps where any protein (neither nanobody nor SpyCatcher domain) is chemically modified or immobilized in a way that could partially impair its function. Instead, the design opted for the immobilization of chemically modified SpyTag, obtained through regular commercial peptide synthesis, on a 1,6-hexanedithiol (HDT) self-assembling monolayer (SAM), thus forming a combined Chem-SAM on top of the gold gate electrode. The anti-GFP nanobody[26]-SpyCatcher fusion protein was genetically encoded, gene synthesized and produced in *E. coli*. The purified protein was then incubated under physiological conditions with the Chem-SAM, thus completing the sensing surface with a self-assembled Bio-SAM. This strategy leads to a defined biofunctionalization layer in a controllable molecular configuration and orientation, which can be modelled at atomic resolution (**Fig 1D**). A flexible 8-amino acid glycine-serine linker separates nanobody and SpyCatcher domains dynamically and sterically. Taking into account unstructured residues contributed by the nanobody and, in particular, SpyCatcher domains, the 4 nm long nanobody domain is thus separated from the underlying SpyCatcher adapter by an about 30 amino acid or ~12 nm long flexible molecular leash. Additional flexibility is introduced by short glycine-serine and alkane linkers that separate the chemically synthesized SpyTag sequence from the underlying HDT, thus also giving the SpyCatcher domain some freedom to move and rearrange itself above the Chem-SAM.

All three immobilization steps (HDT, SpyTag, and nanobody-SpyCatcher) were monitored through X-ray photoelectron spectroscopy (XPS), quartz crystal microbalance with dissipation (QCM-D), cyclic voltammetry (CV), and electrochemical impedance spectroscopy (EIS). Upon functionalization with the HDT layer, the gold electrode displayed characteristic thiol-gold (S-Au) and free thiol (-SH) peaks in its high-resolution S 2p XPS spectrum, indicating the upright orientation of HDT linked to gold through only one of the two -SH moieties (with the other terminal is vertical to, and pointing away from the surface) (**FIG. 2A**)[29,30]. As SpyTag peptide and then the nanobody fusion protein on top of the Chem-SAM was successively introduced, a new peak appeared at about 163.2 eV, which is attributed to the S-C bond from protein methionine residues (**FIG. 2A**)[31]. High resolution C 1s and N 1s XPS spectra further demonstrated the Bio-SAM formation by revealing C-O, C-N, C-OOR bonds, and nitrogen groups on the surface (**FIGs. 2B-2C**). The S 2p XPS spectra presented in Figure 2A show four deconvoluted peaks with a binding energy at 161.4, 162.6, 162.8, and 164 eV for the HDT-functionalized gold electrode, as summarized in the Table below. The spectrum of the Au-HDT layer was deconvoluted using S(2p) doublets which contain two peak components. They are attributed to the spin-orbit splitting doublet S2p3/2 and S2p1/2 separated by $\Delta E \cong 1.2$ eV with an intensity ratio of 2:1 and fixed FWHM.

**Table: Deconvolution of the XPS S 2p spectra (shown in Figure 2c) of the gate electrode after functionalization with HDT, SpyTag peptide, and nanobody-SpyCatcher.**

| Bonds of interest | | HDT | SpyTag peptide | Nanobody -SpyCatcher |
|---|---|---|---|---|
| S2p$_{3/2}$ Au-$\underline{S}$ | B.E. (eV) | 161.4 | 161.4 | 161.4 |
| | Area (%) | 39.2 | 39.3 | 38.2 |
| | FWHM (eV) | 0.742 | | |
| S2p$_{1/2}$ Au-$\underline{S}$ | E.B. (eV) | 162.6 | 162.6 | 162.6 |
| | Area (%) | 19.6 | 19.7 | 19.1 |
| | FWHM (eV) | 1.278 | | |
| S2p$_{3/2}$ $\underline{S}$-H | E.B. (eV) | 162.8 | 162.8 | 162.7 |
| | Area (%) | 27.5 | 18.4 | 16 |
| | FWHM (eV) | 0.745 | | |
| S2p$_{1/2}$ $\underline{S}$-H | E.B. (eV) | 164 | 164 | 164 |
| | Area (%) | 13.7 | 9.1 | 8 |
| | FWHM (eV) | 0.79 | | |
| $\underline{S}$-C | E.B. (eV) | - | 163.2 | 163.2 |
| | Area (%) | - | 13.5 | 18.7 |
| | FWHM (eV) | 1.13 | | |
| B.E. = Binding energy; FWHM = Full width at half maximum | | | | |

The peaks at 161.4 and 162.6 eV stem from the sulfur chemisorbed on the gold surface through a thiolate bond (Au-S)1,2. The resolution of these peaks indicates the high-quality HDT formed on the surface. The signal at 162.8 and 164 eV, on the other hand, corresponds to the free thiol group (terminal R-SH), suggesting two types of sulfur species present in the monolayer; thiolate-type sulfur (Au-SR) and tail thiol sulfur (R-SH). These results suggest that the HDT molecules are in a standing-up configuration with an upright molecular structure. They are bound to gold via the thiolate link using one of their thiol groups, while the other thiol group is free and located at the SAM-air interface. If the HDT molecules were present exclusively in a lying-down configuration, we would be only detecting thiolate components, as all S atoms would be thiolate linked to the gold surface. As we introduced the SpyTag peptide and nanobody-SpyCatcher on this surface, a new peak appeared at ca. 163.2 eV, originating from the Sulphur in methionine.

In the high-resolution of C 1s, the C-C peak of the HDT-functionalized gold was detected, attributed to the six-carbon chain of the HDT molecule. The C-O, C-N and C-OOR peaks originate from the amino acids in SpyTag peptide and the nanobody-SpyCatcher. The N 1s spectra show that nitrogen groups appear on the gold surface upon the introduction of the Bio-SAM proteins. Formation of all layers was further corroborated by electrochemical impedance spectroscopy (EIS) and cyclic voltammetry measurements, which showed a decrease in the electrochemical capacitance of the gold electrode and an increase in its charge transfer resistance with the addition of (insulating) Chem-SAM and Bio-SAM (**Fig.2F-2I**).

The gold electrode exhibits the well-known reversible peaks for the $[Fe(CN)_6]^{3-/4-}$ redox couple with a peak potential separation of approximately 180 mV (**FIG. 2F**, bottom panel). After the modification with HDT, the electron transfer of the redox couple is inhibited, suggesting that a continuous HDT layer has blocked the gold electrode surface. When the SpyTag peptide and nanobody-SpyCatcher are immobilized on the HDT assembled gold electrode, the electrical communication of the Au layer with the redox probe is further suppressed as indicated from the reduced current values. **FIGs. 2G** and **2H** show the representative Bode and Nyquist plots of the Au gate electrode after each functionalization step. The Randles equivalent circuit model was used to quantitatively analyze the impedance spectra (inset of **FIG. 2H**). It consists of the electrolyte resistance ($R_s$), electric double layer capacitance ($C_{dl}$) formed at the electrode/electrolyte interface, charge transfer resistance ($R_{ct}$) of the electrode, and Warburg impedance resulting from the diffusion of ions from the electrolyte to the electrode surface. As HDT was assembled, the diameter of the semicircle in the high frequency region of the Nyquist trace increased significantly, indicating an increase of impedance. $R_{ct}$ increased from 0.5 to 2.3 k$\Omega$ with HDT addition, confirming the charge blocking behavior of the HDT (**FIG. 2I**). The impedance further increases and the Nyquist plot becomes a large semicircle that extends across the entire range of frequencies after the immobilization of SpyTag peptide ($R_{ct} \approx 46.5$ k$\Omega$) and SpyCatcher/nanobody ($R_{ct} \approx 49.2$ k$\Omega$). The effect of SAMs on $C_{dl}$ is summarized in **FIG. 2I**, which shows a monotonic decrease after each functionalization step, consistent with the trend observed in CV curves. These measurements suggest that the SAMs reduce the electrochemical capacitance and increase the charge transfer resistance of the Au electrode, verifying their successful immobilization on the surface.

To assess the packing density of biomolecules on the surface, the immobilization of (i) the maleimide-modified SpyTag peptide and, (ii) the nanobody-SpyCatcher fusion protein on the Chem-SAM was monitored by QCM-D. The mass gained from the two conjugates was quantified to be 114 and 406 ng cm$^{-2}$, respectively (**FIGs. 2D and 2E**). The gold QCM-D sensor comprising an HDT SAM was subjected to the two-step functionalization protocol consisting of the SpyTag peptide coupling followed by the nanobody-SpyCatcher immobilization. In this case, nanobody-SpyCatcher solution also contained BSA as a blocking agent (**FIG. 2E**). Optimization of the blocking protocol and evaluation of the BSA effect was as follows. QCM-D was used to monitor the real-time changes in the mass of the Au electrode upon each biofunctionalization step, i.e., the SpyTag coupling followed by the nanobody-SpyCatcher capture4. When mass (e.g., peptide, nanobody, BSA) is accumulated on the gold sensor, a decrease in its oscillation frequency is observed. Two measurements were performed with two nanobody-SpyCatcher buffers. In the first measurement, the buffer contained only the GFP nanobody-SpyCatcher (Figure 2D). In a second experiment, the final formulation of the nanobody buffer containing BSA was used (Figure 2E). As shown in Figures 2D and 2E, the frequency (top panel) decreases and the mass density (bottom panel) increases upon injection of SpyTag peptide and nanobody-SpyCatcher on the HDT coated surface. The mass density increased to 114 ng cm-2 after SpyTag-peptide attachment through maleimide conjugation between sulfhydryl groups of the HDT and maleimide groups of the peptide. The SpyTag sequence on the peptide can specifically couple with the SpyCatcher domain linked to the nanobody. After thorough rinsing with PBS, a mass density of 406 ng cm-2 was gained for the immobilization of the GFP nanobody-SpyCatcher protein, corresponding to $8.6 \times 10^{12}$ nanobodies per cm2. A larger mass density of 622 ng cm-2 was obtained when the immobilization solution also contained BSA. SARS-CoV (VHH72) nanobody immobilization was also monitored (data not shown). In the absence of BSA, a nanobody-SpyCatcher mass density of 407 ng cm-2 corresponding to $8.4 \times 10^{12}$ nanobodies per cm$^2$ was estimated. With BSA in the nanobody solution, the mass increased slightly to 430 ng cm-2. Switching to PBS does not cause any visible detachment of unbound molecules in the presence of BSA in the GFP nanobody immobilization solution (unlike in the non-BSA immobilization trace shown in the main manuscript Figure 2D). By contrast, VHH72 (SARS) nanobody immobilization looked similar without or with BSA (data not shown). To further investigate BSA action, the gold surface was functionalized, this time with a VHH72-nanobody solution that contained a fluorescently labelled BSA. Fluorescence could not be detected from the BSA on the surface after the nanobody immobilization (data not shown). Including BSA in the spyCatcher reaction solution ensured high sensor performance as shown in Figure 2F where we compare SARS-CoV-2 Spike protein detection in saliva with devices prepared and tested in the absence and presence of BSA in the functionalization and the sample solution.

Based on molecular weights of 1.76 kDa and 28.4 kDa for the SpyTag peptide and nanobody-SpyCatcher protein, respectively, $39 \times 10^{12}$ SpyTag peptides and $8.6 \times 10^{12}$ nanobody-SpyCatcher molecules were coupled per cm$^2$. This density translates to mean inter-particle distance of only 3.4 nm between the SpyCatcher domains at the base of the Bio-SAM. Given the size of this domain (about $4 \times 2.5$ nm), it is thus approaching the maximum coupling density that is physically feasible. The formation of this exceptionally high-density biorecognition layer is further facilitated by the compact nanobody, which has similar dimensions to the SpyCatcher domain but is given additional freedom to pack and reposition itself through the flexible inter-domain linker (FIG. 1D).

Operating as a very sensitive balance, QCM-D can quantitatively monitor the real-time changes in the mass of the Au electrode upon each biofunctionalization step, i.e., the SpyTag coupling followed by the nanobody-SpyCatcher capture.[7] When mass (e.g. peptide, nanobody, BSA) is accumulated on the gold sensor, a decrease in its oscillation frequency is observed. As shown in **FIGs. 2C** and **2E,** the frequency (top panel) decreases and the mass density (bottom panel) increases upon injection of SpyTag peptide and nanobody-SpyCatcher on the HDT coated surface. The mass density increased to 111 ng cm$^{-2}$ after SpyTag-peptide attachment through maleimide conjugation between sulfhydryl groups of the HDT and maleimide groups of the peptide. The SpyTag sequence on the peptide can specifically couple with the SpyCatcher domain linked to the nanobody. Two measurements were performed with two binding buffers. One buffer contained only the nanobody-SpyCatcher (**FIG. 2C**). In a second experiment, the final formulation of sensor binding buffer containing also the BSA was used (**FIG. 2E**). After thorough rinsing with PBS, a mass density of 406 ng cm$^{-2}$ was gained with nanobody-SpyCatcher, corresponding to $8.6 \times 10^{12}$ nanobodies per cm$^2$. A larger mass density of 622 ng cm$^{-2}$ was obtained when the immobilization solution also contained BSA. These results suggest two scenarios or a combination of them: 1) BSA binds to and blocks: sulfhydryl groups exposed to the solution, gaps between peptide linkers or other surface imperfections. 2) BSA acts as a blocking agent *in solution* by capturing contaminating or partially unfolded proteins so that only intact nanobody-SpyCatcher fusions can couple to the surface. In support of scenario (2), switching to PBS does not cause any visible detachment of unbound molecules when BSA was added to the immobilization solution (unlike in the non-BSA immobilization trace shown in the main manuscript **FIG. 2D**). Moreover, adding BSA directly to the immobilization solution outperformed protocols using a separate BSA blocking step.

## Optimization of protein detection

Detection of GFP with the anti-GFP nanobody-conjugated gate electrode was used as a model to ascertain multiple parameters of the sensor design. The biofunctionalized gate electrode was incubated with increasing GFP concentrations and then stacked vertically on top of the channel separated by PBS (phosphate-buffered saline, pH 7.4, ionic strength 0.162 M) to complete the OECT biosensor (**FIG. 1A**). Initial experiments addressed nonspecific binding, which was eliminated by adding bovine serum albumin (BSA) and a mild detergent (Tween-20) to both the nanobody-SpyCatcher protein immobilization and the target-binding solutions. The co-addition of BSA with SpyCatcher protein outperformed other blocking strategies. Comparing QCM-D binding traces with (**Fig 2E**) and without BSA (**Fig 2D**), BSA may be primarily capturing contaminating proteins in solution rather than binding to and blocking the surface in a classical sense. This view is supported by QCM-D traces recorded with another nanobody construct and by the absence of fluorescently labeled BSA on the gate electrode surface (data not shown). Nevertheless, the inclusion of BSA in the functionalization protocol was important for the sensor performance as the addition of BSA to SpyCatcher protein and to the target-binding buffer outperformed other blocking strategies (FIG. 2F, top panel).The OECT working geometry was investigated through systematic variation of gate and channel sizes. The highest sensitivity towards GFP was attained with an OECT having a gate electrode area of 0.64 mm$^2$, and a channel width of 100 $\mu$m and length of 10 $\mu$m (**FIG. 3A**). The sensor response was optimized by tuning the geometry of the gate electrode and the channel. Three differently sized gate electrodes (0.8 mm in square, 2.8 mm in circle, and 4.8 mm in square) were fabricated to gate OECT channels with two distinct geometries where the channel length was varied, $L$ = 10 $\mu$m or $L$ = 100 $\mu$m, while keeping the channel width constant at 100 $\mu$m. The gate electrodes were functionalized with the GFP nanobody and sensor performance was evaluated as shown below. The smaller gate and channel exhibited the highest change in current upon GFP binding.

The optimized poly(3,4-ethylenedioxythiophene) doped with poly(styrene sulfonate) (PEDOT:PSS) OECTs were operated in PBS under ambient conditions by varying the gate voltage ($V_G$) between -0.6 V and 0.6 V while the drain voltage ($V_D$) was swept from 0 to -0.6 V. The sensing signal was assessed by monitoring the drain current ($I_D$) as a function of the $V_G$ at a fixed $V_D$ = -0.6 V. The reference (blank) response of the sensor was acquired by exposing the nanobody-functionalized gate electrode to the binding buffer without GFP (PBS) (**FIG. 3B, bottom panel**). The same electrode was then incubated for ten minutes with a 5 $\mu$L drop of binding buffer containing GFP, rinsed twice in PBS, and then stacked vertically on top of the channel separated by PBS (pH 7.4; ionic strength = 0.162 M) to complete the OECT biosensor). See Fig. 3B, top panel, for a comparison with a longer incubation time. After gate exposure to GFP, $I_D$ underwent a notable decrease at all gate voltages applied. As the GFP concentration increased, this sensor response became more evident (**FIG. 3B, bottom panel**). Similarly, the transconductance of the devices decreased as GFP interacted with the gate electrode (**FIG. 3H**). In contrast, the device characteristics did not change if the gate electrode was incubated in solutions containing equivalent molar concentrations of non-target molecules such as a FRB-mCherry fusion (**FIG. 3C, FIG. 3I**) and lysozyme (**FIG. 3J**). Lysozyme is abundant in saliva which could be a potential biological medium for the SARS-CoV-2 application. mCherry is a red fluorescent protein with low sequence homology but very high structural similarity to GFP and thus challenged sensor specificity.

The trend in $I_D$ and $g_m$ can be clearly attributed to the specific binding of GFP to the nanobody-functionalized gate electrode. Fluorescence images confirmed the specific capture of GFP, but not mCherry (**data not shown**). As GFP is bound to the nanobody functionalized Au electrode, the film, which is originally nonfluorescent at the chosen wavelength emits green fluorescence. As the electrode is incubated in the mCherry solution, only a few spots on the edge of the sensor show red fluorescence. This signal is caused by the mCherry which can be non-specifically absorbed, mostly on the rough or damaged edges of the surface resulting from the laser cutting process (data not shown). The GFP-nanobody interaction increases the impedance at the gate/electrolyte interface (**FIGs. 4A and 4B**), resulting in an increase in the potential decrease therein, which leads to less efficient capacitive coupling between the gate and the channel.

The capacitance of the gate electrode is affected by the molecular binding events, i.e., upon generation of a non-conducting/dielectric layer. Our impedance data suggest that binding events cause the gate capacitance to decrease ($C_g$ -> $C_{g,\,eff}$). In the OECT, we consider two double layer capacitancesformed at the channel and gate electrode interfaces, connected in series13. The applied gate voltage isdistributed at these two interfaces, depending on the magnitude of the capacitance at each surface. The fractional voltage drop at the gate electrode is described by the following set of equations:

$$U_{Ch} = \frac{1}{j\omega C_{Ch}} / \left( \frac{1}{j\omega C_g} + \frac{1}{j\omega C_{Ch}} + R_{sol} \right)$$
(4.1)

where, $U_{Ch}$ is the voltage drop at the channel surface, $j$ is the complex number, $\omega$ is the angular frequency, $C_{Ch}$ is the channel capacitance, $C_g$ is the gate capacitance and $R_{sol}$, is the solution resistance. For the DC regime, the equation 4.1 can be simplified to:

$$(4.2)\ U_{Ch} = 1/(1 + C_{Ch}/C_g)$$

The corresponding fractional voltage drop at the gate electrode can be expressed as:

$$(4.3)\ U_g = 1 - U_{Ch}$$

As proteins bind to the gate electrode, $C_g$ decreases. A lower $C$! leads to a larger proportion of the applied gate voltage to drop at the gate/electrolyte interface. The binding between the nanobody and the protein at the gate electrode therefore results in a less efficient transistor operation, where all the gate voltage should ideally drop at the channel/electrolyte interface.

Hence, a reduction in the current and its modulation was measured as GFP-binding weakens the electrical driving force acting on the cations. The decrease in $I_D$ is not due to a degradation of the channel over consecutive I-V cycles (**FIG. 4C**). There was no change in gate current ($I_G$) upon GFP binding, and the value of IG was six orders of magnitude lower than the ON current of the OECT (**FIGs. 4D and 4E**). Within the gate voltage range in which in the devices operated in, no Faradaic reactions that generate current occur at the gate electrode upon analyte binding.

To quantify the sensor response and to minimize device-to-device variations, the normalized response (NR) of the OECT was calculated by normalizing the target protein-induced change in $I_D$ at a single $V_D$ and $V_G$, to its value previously measured after exposure to blank solution. The normalized response (NR) *vs*. GFP calibration curve revealed that the nanobody-functionalized OECT detects GFP with a lowest limit of detection (LOD) of 23 fM (**FIG. 3D**). The NR was less than 5% even for the highest concentrations of negative controls (mCherry and lysosome) while it exceeded 30% for higher (nM) GFP concentrations. Overall, PEDOT:PSS-based OECT biosensors were sensitive to the presence of $6 \times 10^4$ GFP molecules in 5 $\mu$L of buffer and responded within a dynamic range of six orders of magnitude. This sensitivity is on-par with commercial colorimetric ELISA assays which, however, take hours to complete, require larger sample volumes and have a far lower dynamic range[33].

### *Sensing with accumulation mode OECTs*

OECTs can be constructed for either depletion- or accumulation-mode operation. The channel often used is PEDOT:PSS, which is intrinsically doped and thus operates in depletion mode, that is, $I_D$ decreases with an increase of $V_G$.

Application of a positive gate voltage VG injects cations into the channel, which compensate for the depleted holes and switch the transistor off.. For continuous and long-term use, the depletion-mode operation may negatively affect material stability and power consumption as it implies high operating currents and relatively high VG to run the device. Recent studies expanded the available OECT channel materials through the chemical design of undoped, conjugated backbones which were functionalized with hydrophilic side chains that facilitate ion injection and transport in the film[16,34]. OECTs made using these materials operate in accumulation mode, that is, the channel is initially OFF and generates a current only upon application of a small gate voltage[15]. This operation mode allows for low power electronics applications, improves device stability, and is more compatible with integrated circuit designs that conventionally involve accumulation mode transistors. Nevertheless, accumulation mode OECTs for biochemical sensing have not yet been reported. PEDOT:PSS was therefore replaced with a novel conjugated polymer, p(g0T2-g6T2)[35].

p(g0T2-g6T2) is a mixed (ionic and electronic) conductor. A negative $V_G$ pushes anions into the film that compensate for the holes injected from the metal contacts, turning the device ON (**FIGs. 5A-D**). **FIGs. 5A-B** shows typical output (FIG. 5A) and transfer (FIG. 5B) curves o**f** an Au electrode gated p(g0T2-g6T2) channel. The slope of the transfer curve represents the transconductance, $g_m$, and exhibits a peak value of 15 mS at $V_G$ = -0.3 V. The subthreshold region can be seen in the log scale plot of $I_D$ *vs.* $V_G$ with the maximum slope of 60 mV dec$^{-1}$, at $V_G \approx 0.15$ V (**FIG. 5C**). The devices have the highest gain and efficiency at low $V_G$ range (**FIG. 5D**).

The maximum g$_m$ (15 mS) occurs at a $V_G$ of ca. -0.3 V (FIG. 5B) and the transconductance efficiency ($g_m$ obtained per unit current), is very high at low $V_G$ ((with the maximum slope of 60 mV dec$^{-1}$ at VG $\approx$ 0.15 V; FIG. 5C) due to the exponential $I_D$ vs. $V_G$ characteristics. This behavior is similar to the subthreshold region of operation in traditional inorganic metal oxide semiconductor FETs[36]. The material also shows exceptional operational and environmental stability (**FIG. 5E**), thus overcoming the limitations of the currently available organic mixed (semi)conductors[35]

**FIG. 3E** shows the typical transfer characteristics of p(g0T2-g6T2) OECTs gated with the nanobody-functionalized Au electrodes incubated in solutions with varying GFP concentrations. As GFP bound to the gate, $I_D$ decreased for all gate voltages. This decrease was accompanied by a significant shift in the threshold voltage ($V_{th}$) towards more negative values (**FIG. 6A**). Similar to PEDOT:PSS sensors, p(g0T2-g6T2) OECTs showed no significant response to mCherry (**FIG. 3F**) and lysozyme (**FIG. 6B**) (at closer inspection, our data suggest a minor cross-reaction of the anti-GFP nanobody with the structurally similar mCherry). The sensor responded to a GFP concentration as low as 4.7 aM (NR = 7%) (**FIG. 3G**) with a dynamic range spanning 10 orders of magnitude. The lowest limit of detection (LOD) for these devices was calculated to be 14 aM, corresponding to 42 molecules in 5 μL of sample. Compared to the otherwise identical PEDOT:PSS-based sensor, the accumulation mode OECT thus showed a 1000-fold improved sensitivity (aM compared to fM) and yet larger dynamic range (10 orders of magnitude, from aM to nM, compared to 6 orders of magnitude). Moreover, a much lower operating voltage was observed. The target response was maximized (and off-target response minimized) at $V_G = V_D$ = -0.1 V compared to $V_G = V_D$ = -0.6V for the PEDOT:PSS OECT (**FIGs. 6C** and **6D**). Such characteristics would in principle allow for nanobody-OECT reader devices with very compact built and long battery life.

From a biochemical point of view, the observation of binding at such low concentrations was initially surprising. The 14 aM detection limit lies 30 million-fold below the consensus equilibrium dissociation constant ($K_D$~0.5 nM) reported for the interaction between nanobody and GFP[37]. Previous sutues using electrolyte gated organic FET recognizing IgG attributed single-molecule detection to a combination of: (1) the 1000-fold amplification of input to output signal provided by the transistor; and (2) the perturbation of a tight hydrogen-bonding network in their chem-SAM, which was suggested to propagate as a defect over larger areas of the electrode. In contrast, the sensor disclosed herein does not have a hydrogen-bonding network in or near the chem-SAM. However, the OECTs developed in this work have higher gains (the gm is on the order of mS rather than μS of typical electrolyte-gated organic FETs), permitting the amplification of input signals by a factor of one million. Indeed, without the amplification endowed by the OECT circuitry, gate capacitance changes cannot be resolved at such low concentrations (FIG. 4A and 4B). A further improvement over previous designs is the very dense coupling of compact and intact receptor units in a, presumably, near-optimal binding orientation. Given the density of nanobody molecules ($8.6 \times 10^{12}$ / cm$^2$, see above) and assuming the domain is diffusing within a 20 nm layer above the sensor surface (**FIG. 1D**) a local nanobody concentration of about 700 μM is obtained, which is far above the dissociation constant. Any target molecule diffusing into this capture layer will therefore necessarily bind.

Nanobody - analyte binding:

At (local) equilibrium, analyte binding can be described by the simple mass action law:

$$K\text{-} = [/0]$$

*(5.1) A + R $\rightleftharpoons$ AR; with the dissociation constant:* $\quad [/][0]$

KD is the dissociation constant and [A], [R], [AR] denote the respective concentrations. Replacing [A] = [A0] - [RA], the fraction of bound analyte at a certain free receptor concentration is obtained:

(5.2)

$$Y_A = \frac{[AR]}{[A_0]} = \frac{[R]}{K_D + [R]}$$

As long as the local free receptor concentration $[R] \gg K\text{-}$, we can therefore assume ~100% binding of analyte molecules entering the capture area on top of the sensor surface. The inverse question can also be asked: At which free analyte concentration [A] will the very first receptor capture a single analyte molecule: Starting point is the Langmuir adsorption model

(5.3.)

$$Y = \frac{AR}{R_0} = \frac{[A]}{[A] + K_D}$$

Y is now the fraction of the receptor bound to analyte. Instead of as concentration, AR is expressed as a number or density on the surface. For one out of 55 billion receptors bound Y=1/R0 and it follows:

(5.4)

$$[A]_{limit} = \frac{K_D}{R_0 - 1}$$

Since $R0 >> 1$:

(5.5)

$$[A]_{limit} = \frac{K_D}{R_0}$$

Assuming KD= $0.5 \times 10^{-9}$ M (for the GFP nanobody) and $5.5 \times 10^{10}$ binding sites, we obtain the analyte concentration limit of $9 \times 10^{-20}$ mol/L or 9 zM. For the binding of SARS-CoV-2 spike protein the dissociation constant is higher (weaker affinity) KD= $2.3 \times 10^{-8}$ M leading to a limit of analyte concentration of 418 zM or 0.4 aM. Note that this is only an upper (less favorable) boundary for detection limits for an infinite incubation time. In reality, the larger binding volume cannot reach equilibrium in reasonable time scales and unbinding is very unlikely. Binding is kinetically driven by the molecular on-rate near the surface and is dominated by diffusion under mass transport limitation in the larger measurement volume. Equation 5.5 does however more readily apply to unspecific binders (i.e., contaminants) which have much weaker affinity (higher KDs with very fast koff rates). Binding of contaminants can therefore be eliminated through washing steps that ensure their sufficient dilution into a larger washing volume.

Once bound, the interaction's low $k_{off}$ rate[37] (~$1.5 \times 10^{-4}$ s$^{-1}$ corresponding to 80 min half-life) will trap the target molecule throughout all washing steps. In fact, our surface plasmon resonance (SPR) measurements indicate an even lower, essentially unmeasurable, off rate for this complex with our own protein and buffer conditions (**FIG. 7A-7F**). The likelihood of analyte escape is further reduced by a high possibility of re-binding within the high-density receptor phase. The high-density nanobody-functionalized gate electrode thus operates as a kinetic trap for target molecules, while nonspecific contaminants are washed away due to their faster off rates. Sensitivity is likely not dictated by nanobody:target affinity ($K_D$) but by target diffusion and the kinetic stability of the complex ($k_{off}$). The high receptor density makes unproductive collisions between analyte and surface very unlikely. The large gate area and high receptor density also combine to a high binding capacity (corresponding to an analyte concentration of 18 nM in the 5 $\mu$l solution volume), which increases the sensor's dynamic range.

### *Detection of SARS-CoV-2 and MERS antigens*

VHH72-SpyCatcher fusion construct expressed well in E. coli and could be purified to a high yield (54 mg 1-1 culture) as a monomeric protein. Although SARS-CoV-2 was the initial diagnostic target, constructs for the detection of MERS-CoV based on the previously reported nanobodies VHH83, VHH04 (Raj, et al., Sci. Adv. 4, eaas9667 (2018)) and VHH55 (Wrapp, et al. Cell 181, 1004-1015.e15 (2020)) were also designed and expressed. The VHH04-SpyCatcher fusion yielded high-quality monomeric protein and was advanced to OECT experiments. Both VHH72 and VHH04 bind to the RBD of the homotrimeric (SARS or MERS, respectively) coronavirus spike protein.

SARS-CoV nanobody functionalized OECTs showed an excellent response to SARS-CoV-2 receptor binding domain (RBD) and SARS-CoV-2 S1, regardless of the channel material used (**FIGs. 8A and 8B**). Incubation of these gate electrodes with various concentrations of GFP did not evoke a current response, demonstrating the specificity of the VHH72 nanobody for the viral proteins. The same devices also responded to the much smaller SARS-CoV-1 RBD (**FIG. 8G-8H**). In a direct comparison, the higheraffinity of VHH72 for the SARS-CoV-1 RBD compared with the SARS-CoV-2 RBD only translated into a small signal increase. In contrast, the larger SARS-CoV-2 S1 spike protein generated larger current changes than the isolated RBD. This increase in the effective gate voltage can stem from at least two effects. The trimeric S1 provides a higher local concentration of the binding domain and is larger than the monomeric RBD. Hence S1 binds more strongly and covers a larger part of the nanobody-functionalized surface. As it was the case with GFP sensors, p(g0T2-g6T2) OECTs showed higher sensitivities than PEDOT:PSS devices. p(g0T2-g6T2) OECT detected SARS-CoV-2 S1 at 4.7 aM with 30% change in the NR ($o_{SD}$ - 7% at most), translating to an LOD of 18 zM, corresponding to less than one molecule in 5 $\mu$L.

Having confirmed that the SARS-CoV-1 nanobody-functionalized OECTs are sensitive and specific to SARS-2 proteins in the buffer, the focus shifted to the requirements of point-of-care applications. Considering the ease of collection, the minimum patient discomfort during sample collection and the presence of the virus therein, human saliva samples were chosen as the preferred specimen medium. Human saliva samples were collected and mixed with predetermined amounts of target and non-target proteins to characterize the sensing ability of SARS-CoV-1 nanobody-functionalized OECTs comprising p(g0T2-g6T2) channels. The NR increased with the concentration of SARS-CoV-2 proteins in saliva and showed only a negligible change when the same gate electrode was exposed to GFP at the same concentration range (**FIG. 8C**). Cross-reactivity measurements were performed to evaluate the interactions between the nanobody and MERS-CoV S1, a structurally similar protein to SARS-CoV-2. The SARS-CoV-2 sensor did not recognize MERS-CoV S1, verifying the specificity of the sensor to SARS-CoV proteins (**FIG. 8C**). The LOD values obtained for SARS-CoV-2 S1 in saliva (LOD = 1.2 $\times$ 10$^{-21}$ M) were comparable to those in buffer (LOD = 1.8 $\times$ 10$^{-20}$ M) indicating that the complex composition of saliva did not hamper the association between the nanobody and its target. The sensor could also detect SARS-CoV-2 S1 spiked in human serum (**FIG. 8I**). Table 3 summarizes the LODs of all the sensors fabricated in this study. These findings demonstrate the performance of the SARS-CoV-2 sensor in two different complex biological media and the sensor's ability to selectively differentiate physiologically and clinically relevant protein concentrations.

### Table 3 LOD of the OECT biosensors.

| Channel Material | Nanobody on the Gate | Targets | Sensors in Buffer | | |
| --- | --- | --- | --- | --- | --- |
| | | | LOD | Fitting Equation | Highest SD |
| PEDOT:PSS | GFP | GFP | 2.3 x 10$^{-14}$ M | $y =1.065 + 0.078x$, $R^2$=0.99 | 11 % |
| | | mCherry | -- | -- | 4 % |
| | | Lysozyme | -- | -- | 4 % |
| | SARS-CoV-1 | SARS-1 RBD | 1.5 x 10$^{-15}$ M | $y =0.616 +0.037x$, $R^2$=0.89 | 9% |
| | | SARS-2 RBD | 2.4 x 10$^{-14}$ M | $y =0.317 +0.015x$, $R^2$=0.962 | 5% |
| | | SARS-2 Spike | 2.8 x 10$^{-16}$ M | $y =1.406 +0.073x$, $R^2$=0.995 | 14 % |
| | | GFP | -- | -- | 9% |
| p(g0T2-g6T2) | GFP | GFP | 1.4 x 10$^{-17}$ M | $y =1.381 +0.076x$, $R^2$=0.94 | 9 % |
| | | mCherry | -- | -- | 3% |
| | | Lysozyme | -- | -- | 3 % |
| | SARS-CoV-1 | SARS-1 RBD | 1.6 x 10$^{-17}$ M | $y =1.372 +0.077x$, $R^2$=0.88 | 9% |
| | | SARS-2 RBD | 4.8 x 10$^{-14}$ M | $y =1.477 +0.105x$, $R^2$=0.93 | 12 % |
| | | SARS-2 Spike | 1.8 x 10$^{-20}$ M | $y =1.678 +0.081x$, $R^2$=0.98 | 7% |
| | | GFP | -- | -- | 9% |
| | | | Sensors in Saliva | | |
| PEDOT:PSS | MERS-CoV | MERS-CoV Spike | 6.1 x 10$^{-19}$ M | $y =0.666 +0.034x$, $R^2$=0.959 | 5 % |
| p(g0T2-g6T2) | SARS-CoV-1 | SARS-1 RBD | -- | -- | - |
| | | SARS-2 RBD | 2.3 x 10$^{-14}$ M | $y =1.28 +0.066x$, $R^2$=0.91 | 11% |
| | | SARS-2 Spike | 1.2 x 10$^{21}$ M | $y =1.409 +0.065x$, $R^2$=0.98 | 10% |
| | | GFP | -- | -- | 2% |
| | MERS-CoV | MERS-CoV Spike | 5.7 x 10$^{-19}$ M | $y =1.914 +0.102x$, $R^2$=0.993 | 6% |

The gate electrode was then exposed to saliva solutions that contained random concentrations of SARS-CoV-2 S1 to evaluate the performance of the sensor in an environment closer to actual screening conditions. The NR values varied according to the protein content in saliva and corresponded to the values obtained in dose curves (**FIG. 8D**). Moreover, the versatility of the biosensor design by using a MERS-CoV nanobody-functionalized gate electrode for the detection of nominal concentrations of MERS-CoV S1 (detection range from 100 zM to 1 nM) in saliva (**FIG. 8F**) was demonstrated.

Our findings thus demonstrate the sensitivity and selectivity of nanobody-OECTs in complex biological media and at physiologically and clinically relevant protein concentrations.A comparison of our OECT sensor performance with other similar thin-film transistor-based devices developed for immunosensing indicates the superiority of this sensor technology (for example, in terms of dynamic range, power consumption, miniaturization and compatibility with unprocessed human samples; Table 4).

**Table 4: A comparison of the characteristics of organic thin film transistor-based devices developed for immunosensing.**

| | Ref.[11] | Ref.[12] | Ref.[5] | This work |
|---|---|---|---|---|
| **Device** | EGOFET | EGOFET | OECT | OECT |
| **Channel material** | P3HT | P3HT | PEDOT:PSS | 1) PEDOT:PSS 2) p(g0T2-g6T2) |
| **Bioreceptor** | Antibody | Antibody | Antibody | Nanobody |
| **Analyte** | generic IgG & IgM | generic IgG | generic IgG | 1) GFP protein 2) SARS-CoV-2 Spike protein 3) MERS Spike protein |
| **Sample medium** | Buffer | 1) Buffer; 2) centrifuged and 5000-fold diluted saliva | Buffer | 1) Buffer 2) raw saliva (1:1, 1:4 dilutions) 3) human serum 4) UTM (nasal swabs) |
| **Electrolyte** | 300 $\mu$L DI water | 300 $\mu$L DI water | 300 $\mu$L PBS | 100 $\mu$L PBS |
| **Operation** | $V_D$=-0.4V $V_G$=-0.3V | $V_D$= -0.4V $V_G$= -0.35V | $V_D$= -0.4V $V_G$= -0.5V | $V_D$=-0.1V $V_G$= -0.1V |
| **Incubation time** | 10 min | 10 min | 10 min | 10 min |
| **LOD** | 10.7 zM | 10 zM | 6 aM | 1.2 zM |
| **Dynamic range** | zM - fM | zM - fM | aM - pM | zM - nM |

OECTs also compare favorably to optical methods for single-molecule detection, such as flow-based immunoassays , and plasmonic nanosensors: Targets are not limited by size, and the lack of labeling allows for very short sample preparation and device operation times.

### *Detection of SARS-CoV-2 in Clinical Samples*

Finally, the sensors were validated using human patient nasopharyngeal swabs and saliva. The swabs were stored in a universal transport medium (UTM) whereas saliva was collected and kept without an additional buffer. The calibration curves obtained for SARS-CoV-2 S1 protein in UTM using anti-SARS-CoV-1 and anti-GFP gates confirm that sensor operation is maintained in this medium (LOD for SARS-CoV-2 S1 in UTM was $1.9 \times 10^{-14}$ M) (**FIG. 9A**). All four nasal swab samples that had previously been tested as positive were confirmed to be positive by the OECT. Notably, all samples had low viral loads (RT-PCR cycle threshold = 30 to 37; close to the detection limit (cycle threshold = 40)). For a direct comparison of detection limits, a tenfold dilution series in UTM of one patient sample was prepared, each dilution 1:1 mixed with the binding buffer and subjected it side by side to sensor measurements, as well as RNA extraction and RT-PCR (Fig. 9D). The sensor's above-noise response appeared to outperform RT-PCR by one order of magnitude.

The control measurements were performed using multiple anti-GFP gate electrodes and the results were verified independently by RT-PCR on the same samples. In a second validation, saliva was collected from six healthy volunteers and seven volunteers who had recently been confirmed as COVID-19 positive. Each sample was diluted fourfold in a modified, virus-inactivating lysis buffer (lysis buffer A) and subjected to three independent measurements. Positive samples were additionally measured with one GFP nanobody gate electrode each. Quantitative PCR with reverse transcription (RT-qPCR) on each sample (calibrated against N-gene complementary DNA) confirmed the six negatives and revealed a wide range of viral loads from 2,700 to $1.8 \times 1012$ copies per ml for the others (Fig. 9E-9H). Despite substantial variation in individual sensor readings, average OECT signals reliably agreed with RT-qPCR on the presence or absence of SARS-CoV-2 in all samples (Fig. 9B and Fig. 9E). However, in contrast with the previous protein measurements, there was no correlation between sensor signal and the viral loads established by RT-PCR. However, the dilution series of virus material from a nasal swab in commercial UTM shows that a concentration-dependent signal can be achieved (Fig. 9D). The data with patient samples shows a qualitative sensor performance on par with RT-PCR and RT-qPCR,

In direct comparison of lysis buffers used with saliva samples, buffer B' performed better than buffer A in experiments performed on two fresh saliva samples from two SARS-CoV-2 positive patients. This buffer is a lysis buffer as well but uses a different detergent. It also has increased salt concentration. Moreover, RNAse A enzyme was added to reduce viscosity that may arise from viral RNA in the sample. The results are shown in FIG 10.

## Conclusion

A modular nanobody was developed and optimized - OECT architecture that allows the detection of very low antigen concentrations in 10 min with a sample volume of $\leq 5$ $\mu$L and close to single-molecule sensitivity. Autocatalytic immobilization with the SpyTag/SpyCatcher coupling strategy is here shown to be a robust method for conjugating recombinant proteins on biosensor surfaces with very high packing density, full control over molecular orientation and no need for chemical activation or modification of the protein. This work is the first demonstration of a nanobody-functionalized OECT sensor for fast detection of target antigens. Rapid detection of various analytes at close to single-molecule concentrations in buffer, serum and saliva were achieved, respectively. The OECT sensors are thus capable of detecting protein antigens with high sensitivity and selectivity but negligible non-specific binding in complex biological media. The platform is highly modular and can be adapted for the detection of any target analyte for which a specific nanobody is available.

## References

1. World Health Organization. WHO Coronavirus Disease (COVID-19) Dashboard. https://covid9.who.int (2020).
2. Kissler, et al., Science 368, 860-868 (2020).
3. Lewis, Nature 583, 510-513 (2020).
4. Oran, et al., Ann. Intern. Med. (2020) doi:10.7326/M20-3012.
5. Long. et al., Nat. Med. 26, 1200-1204 (2020).
6. World Health Organization. WHO Director-General's opening remarks at the media briefing on COVID-19 - 16 March 2020. https://www.who.int/dg/speeches/detail/who-director-general-s-opening-remarks-at-the-media-briefing-on-covid-19---16-march-2020 (2020).
7. Corman, et al., Eurosurveillance 25, (2020).
8. Ferretti, et al., Science 368, (2020).
9. Moraz, et al., medRxiv 2020.07.10.20149773 (2020).
10. Pasomsub, E. et al., Saliva sample as a non-invasive specimen for the diagnosis of coronavirus disease 2019: a cross-sectional study. Clin. Microbiol. Infect. 0, (2020).
11. Wyllie, et al., N. Engl. J. Med. 383, 1283-1286 (2020).
12. Krueger, et al., medRxiv 2020.10.01.20203836 (2020)
13. Torrente-Rodriguez, et al., Matter 3:1-18, (2020).
14. Macchia, et al., Single-molecule detection with a millimetre-sized transistor. Nat. Commun. 9, 3223 (2018).
15. Rivnay, et al., Nat. Rev. Mater. 3, 1-14 (2018).
16. Inal, et al., Acc. Chem. Res. 51, 1368-1376 (2018).
17. Ohayon, et al., Adv. Mater. 32, 2001439 (2020).
18. Gentili, et al., J. Mater. Chem. B 6, 5400-5406 (2018).
19. Trilling, et al., Analyst 138, 1619-1627 (2013).
20. Zakeri, et al., Proc. Natl. Acad. Sci. U. S. A. 109, E690-E697 (2012).
21. Muyldermans, Annu. Rev. Biochem. 82, 775-797 (2013).
22. Hamers-Casterman, et al., Nature 363, 446-448 (1993).

23. Steeland, et al., Today 21, 1076-1113 (2016).

24. De Meyer, Trends Biotechnol. 32, 263-270 (2014).

25. Filipiak, et al., Sens. Actuators B Chem. 255, 1507-1516 (2018).

26. Rothbauer, et al., Mol. Cell. Proteomics 7, 282-289 (2008).

27. Keeble, et al., Methods Enzymol. 617, 443-461 (2019).

28. Oloketuyi, et al., Biosens. Bioelectron. 154, 112052 (2020).

29. Esplandiu, et al., Appl. Surf. Sci. 199, 166-182 (2002).

30. Love, et al., Chem. Rev. 105, 1103-1170 (2005).

31. Weser, U., Redox reactions of sulphur-containing amino-acid residues in proteins and metalloproteins, an XPS study. in Cation Ordering and Electron Transfer (eds. Gleitzer, C., Goodenough, J. B., Hyde, B. G., O'Keeffe, M. & Weser, U.) 145-160 (Springer, 1985). doi:10.1007/BFb0111194.

32. Macchia, et al., Flex. Print. Electron. 3, 034002 (2018).

33. Zhang, et al., Analyst 139, 439-445 (2013).

34. Moser, et al., Adv. Funct. Mater. 29, 1807033 (2019).

35. Moser, et al., Side Chain Redistribution as a Strategy to Boost Organic Electrochemical Transistor Performance and Stability. Adv. Mater. n/a, 2002748.

36. Venkatraman, et al., Adv. Sci. Weinh. Baden-Wurtt. Ger. 5, 1800453 (2018).

37. Kubala, et al., Protein Sci. Publ. Protein Soc. 19, 2389-2401 (2010).

38. Ohayon, et al., Nat. Mater. 19, 456-463 (2020).

39. Wustoni, et al., Adv. Mater. Interfaces 6, 1800928 (2019).

40. Qu, et al., J. Phys. Chem. C 114, 497-505 (2010).

41. Höök et al., Langmuir 14, 729-734 (1998).

42. Eshaghi, et al., Angew. Chem. Int. Ed Engl. 54, 13952-13956 (2015).

43. Li, et al., J. Mol. Biol. 426, 309-317 (2014).

44. Edinburgh-Genome-Foundry/DnaChisel. (Edinburgh Genome Foundry, 2020).

45. Mutalik et al., Nat. Methods 10, 354-360 (2013).

46. Long, et al., Anal. Chem. 55, 712A-724A (1983).

47. ExPASy - ProtParam tool. https://web.expasy.org/protparam/.

## Claims

1. A biosensor comprising an organic electrochemical transistor (OECT), and optionally an array of two or more OECTs, and a biorecognition layer, wherein the OECT or each of the OECTs in the array comprises a source electrode, a drain electrode, a channel, and a gate electrode,

   wherein the orientation of the biorecognition layer relative to the OECT surface is represented by the general formula:

   $$\mathbf{N}\text{-}L_1\text{-}AP_1\text{-}AP_2\text{-}L_2\text{-}\mathbf{B} \qquad \text{Formula I}$$

   wherein $\mathbf{N}$ is one or more organic molecules which self-assemble to form a fist SAM, $L_1$ is an optional first linker, $AP_1$ is a first peptide binding partner; $AP_2$ is a second peptide partner; $AP_1$ and $AP_2$ are binding partners, $L_2$ is a second linker, and $\mathbf{B}$ is a biorecognition element,
   wherein $AP_1$-$AP_2$ is selected from the group consisting of SpyTag/SpyCatcher peptide conjugate, snoopCatcher/snoopTag peptide conjugate, MoonTag/MoonCatcher peptide conjugate, SnoopTagJr/SnoopCatcher peptide conjugate, SnoopTagJr/DogTag peptide conjugate, and SdyTag/SdyCatcher peptide conjugate.

2. The biosensor of claim 1, wherein the $AP_1$-$AP_2$ is a SpyTag/SpyCatcher peptide conjugate.

3. The biosensor of claim 1 or 2, wherein the biorecognition layer comprises a Chem-SAM, wherein the Chem-SAM comprises the first SAM formed by N, chemically modified with $AP_1$.

4. The biosensor of any one of claims 1-3, wherein the biorecognition element is an antibody, or a two-domain or a single-domain antibody fragment, such as a nanobody, optionally, wherein the nanobody recognizes SAR-CoV-2 receptor binding domain.

5. The biosensor of any one of claims 1-4, wherein the one or more organic molecules in the first SAM comprise thiols, optionally, wherein the thiols comprise dithols, preferably 1,6-hexanedithiols.

6. The biosensor of any one of claims 1-5, wherein the biorecognition layer comprises a Bio-Sam comprising $AP_1$-$AP_2$-$L_2$-B.

7. The biosensor of any one of claims 1-6, (a) further comprising a blocking agent, optionally wherein the blocking agent is bovine serum albumin (BSA), and/or (b) wherein the channel is formed from a conducting polymer selected from the group consisting of PEDOT:PSS, PEDOT-S, PEDOT:TOS, PEDOTOH:$ClO_4$, PEDOT-*co*-PEDOTOH:$ClO_4$, P3HT, PTHS, BBL, p(g2T-TT), PTHS-TMA$^+$-*co*-P3HT, p(gNDI-g2T), p(g0T2-g6T2), and P-90.

8. The biosensor of any one of claims 1-7, wherein the OECT or each of the OECTs in the array is patterned on a supporting substrate.

9. A method of integrating a biorecognition layer on an electrode comprising:

(i) incubating at least a portion of the surface of the electrode with a first incubation solution comprising a plurality of organic molecules to produce a SAM-modified surface,
(ii) incubating the SAM-modified surface with a second incubation solution comprising a first peptide to produce a first peptide-SAM-modified surface, and
(iii) incubating the first peptide-SAM-modified surface with a third incubation solution comprising a second peptide-recognition element conjugate and a blocking agent, where the first peptide conjugates with the second peptide to form a linker and produces a recognition element-linker-SAM-modified surface, optionally, wherein the organic molecules comprise thiols, preferably dithiols, more preferably 1,6-hexanedithiols and/or the first peptide is a SpyTag peptide.

10. The method of claim 9, wherein: (a) the second peptide-recognition element is a SpyCatcher-nanobody conjugate; optionally, wherein the nanobody is a SAR-CoV-2 receptor binding domain binding nanobody; and/or (b) the blocking agent is BSA.

11. A method of detecting the absence, the presence, or the concentration of an analyte in a biological sample comprising contacting the sample with the biosensor of any one of claims 1-8, wherein the biosensor further comprises a reservoir,

the method comprising:

(i) adding an electrolyte solution into the reservoir;
(ii) incubating the gate electrode with a blank solution;
(iii) placing the gate electrode on top of the channel;
(iv) applying a $V_G$ and a $V_D$;
(v) measuring a first $I_D$;
(vi) incubating the gate electrode with the biological sample for a time period sufficient to allow binding between the analyte and the biorecognition element;
(vii) rinsing the gate electrode with a rinsing buffer; and
(viii) measuring a second $I_D$,

wherein a difference between the second $I_D$ and the first $I_D$ is indicative of the absence, the presence, or the concentration of the analyte in the biological sample, and wherein the biological sample is in a liquid form;
optionally, wherein step (v) is performed simultaneously with, substantially simultaneously with, or subsequent to step (iv);
optionally, wherein steps (vi)-(viii) are repeated one or more time, and
optionally, wherein the biological sample is (a) a bodily fluid selected from the group consisting of whole blood, plasma, serum, saliva, mucus, sputum, bronchial alveolar lavage (BAL), bronchial wash (BW), cerebrospinal fluid (CSF), and urine, or (b) a non-bodily fluid.

12. The method of claim 11 further comprising:

(a) a step of processing a specimen into the biological sample prior to any one of steps (i)-(vi), wherein the specimen is selected from the group consisting of tissues, feces, rectal swab, nasopharyngeal swab, and throat swab; and/or

(b) a step of adding a protease inhibitor into the biological sample prior to any one of steps (i)-(vi) and optionally, wherein (1) the volume of the biological sample is less than 20 μL, less than 10 μL, or less than 5 μL and/or (2) the gate electrode is incubated with the biological sample for a time period up to 60 minutes, up to 50 minutes, up to 40 minutes, up to 30 minutes, up to 20 minutes, or up to 10 minutes.

13. The method of claim 11 or 12 further comprising a step of diluting the biological sample using a sensor binding buffer, prior to any one of steps (i)-(vi), preferably prior to step (iv),
wherein the sensor binding buffer comprises a buffering agent at a concentration in a range from about 10mM to about 50 mM, a salt at a concentration in a range from about 100 mM to about 300 mM, Tween at a concentration in a range from about 0.01% to about 1% (by volume or weight), a protease inhibitor cocktail or tablet, such as cOmplete, and an antimicrobial agent at a concentration in a range from about 0.01% to about 0.05% (by volume or weight).

14. The method of any one of claims 11-13 further comprising a step of diluting the biological sample using a sensor binding buffer, prior to any one of steps (i)-(vi), particularly prior to step (vi),
wherein the sensor binding buffer comprises:

a buffering agent, such as Tris or HEPES, or phosphate salts, at a concentration in a range from about 10mM to about 100 mM;
a salt, such as KF, KBr, $K_2HPO_4$, potassium acetate, potassium citrate, LiF, LiBr, $Li_2HPO_4$, lithium acetate, or lithium citrate, or a combination thereof, at a concentration in a range from about 200 mM to about 1 M;
a detergent selected from the group consisting of Triton-X100, Nonidet P40, IGEPAL CA-630, Brij-35, Brij-58, and Triton X-114, and a combination thereof, at a concentration in a range from about 0.5% to about 2% (by volume or weight);
a protease inhibitor cocktail or tablet, such as cOmplete, at 1X, 2X, or 4X concentration; and
a blocking agent, such as BSA, at a concentration in a range from about 0.05% to 1% (by volume or weight); and optionally
an antimicrobial agent, such as $NaN_3$, at a concentration in a range from about 0.01% to about 0.05% (by volume or weight) and/or
an RNA digestive enzyme, such as an RNAse A enzyme, at a concentration in a range from about 1 mg/L to about 50 mg/L.

15. The biosensor of claims 1-8, wherein the biorecognition layer is integrated on the gate electrode of the OECT.

**Patentansprüche**

1. Biosensor, der einen organischen elektrochemischen Transistor (OECT), und möglicherweise eine Anordnung aus zwei oder mehr OECTs, sowie eine Bioerkennungsschicht umfasst, wobei der OECT oder jeder der OECTs in der Anordnung eine Source-Elektrode, eine Drain-Elektrode, einen Kanal und eine Gate-Elektrode umfasst,

wobei die Ausrichtung der Bioerkennungsschicht relativ zur OECT-Oberfläche der folgenden allgemeinen Formel entspricht:

$$N\text{-}L_1\text{-}AP_1\text{-}AP_2\text{-}L_2\text{-}B \qquad \text{Formel I}$$

wobei N für ein oder mehrere organische Moleküle steht, die sich selbsttätig zusammenfügen, um ein erstes SAM zu bilden, $L_1$ für einen optionalen ersten Linker steht, $AP_1$ für einen ersten Peptidbindungspartner steht; $AP_2$ für einen zweiten Peptidbindungspartner steht; $AP_1$ und $AP_2$ für Bindungspartner stehen, $L_2$ für einen zweiten Linker steht und B für ein Bioerkennungselement steht,
wobei $AP_1$-$AP_2$ aus der Gruppe ausgewählt ist, die aus pyTag/SpyCatcher-Peptidkonjugat, snoopCatcher/snoopTag-Peptidkonjugat, MoonTag/MoonCatcher-Peptidkonjugat, SnoopTagJr/SnoopCatcher-Peptidkonjugat, SnoopTagJr/DogTag-Peptidkonjugat und SdyTag/SdyCatcher-Peptidkonjugat besteht.

2. Biosensor nach Anspruch 1, wobei es sich bei $AP_1$-$AP_2$ um ein SpyTag/SpyCatcher-Peptidkonjugat handelt.

3. Biosensor nach Anspruch 1 oder 2, wobei die Bioerkennungsschicht ein Chem-SAM umfasst, wobei das Chem-SAM das erste SAM umfasst, wie es von N, chemisch modifiziert mit $AP_1$, gebildet wurde.

4. Biosensor nach einem beliebigen der Ansprüche 1 bis 3, wobei es sich bei dem Bioerkennungselement um einen Antikörper oder ein Zwei-Domänen- oder ein Einzeldomänen-Antikörperfragment wie etwa einen Nanobody handelt, wobei der Nanobody möglicherweise die Rezeptorbindungsdomäne von SAR-CoV-2 erkennt.

5. Biosensor nach einem beliebigen der Ansprüche 1 bis 4, wobei das eine oder die mehreren organischen Moleküle in dem ersten SAM Thiole umfassen, wobei die Thiole möglicherweise Dithiole, vorzugsweise 1,6-Hexandiothiole, umfassen.

6. Biosensor nach einem beliebigen der Ansprüche 1 bis 5, wobei die Bioerkennungsschicht ein Bio-Sam umfasst, das $AP_1$-$AP_2$-$L_2$-B umfasst.

7. Biosensor nach einem beliebigen der Ansprüche 1 bis 6, wobei er (a) weiterhin ein Blockiermittel umfasst, wobei es sich bei dem Blockiermittel möglicherweise um Rinderserumalbumin (BSA) handelt, und/oder wobei (b) der Kanal aus einem leitfähigen Polymer gebildet ist, das aus der Gruppe ausgewählt ist, welche aus EDOT:PSS, PEDOT-S, PEDOT:TOS, PEDOTOH:$ClO_4$, PEDOT-co-PEDOTOH:$ClO_4$, P3HT, PTHS, BBL, p(g2T-TT), PTHS-TMA$^+$-co-P3HT, p(gNDI-g2T), p(g0T2-g6T2) und P-90 besteht.

8. Biosensor nach einem beliebigen der Ansprüche 1 bis 7, wobei der OECT oder jeder der OECTs in der Anordnung strukturbildend auf ein Trägersubstrat aufgebracht ist.

9. Verfahren zum Einbau einer Bioerkennungsschicht auf einer Elektrode, umfassend:

   (i) Inkubieren zumindest eines Teilbereichs der Oberfläche der Elektrode mit einer ersten Inkubationslösung, die eine Mehrzahl an organischen Molekülen umfasst, um eine SAM-modifizierte Oberfläche zu erhalten,
   (ii) Inkubieren der SAM-modifizierten Oberfläche mit einer zweiten Inkubationslösung, die ein erstes Peptid umfasst, um eine erstes-Peptid-SAM-modifizierte Oberfläche zu erhalten, und
   (iii) Inkubieren der erstes-Peptid-SAM-modifizierten Oberfläche mit einer dritten Inkubationslösung, die ein Konjugat des Typs zweites-Peptid-Erkennungselement sowie ein Blockiermittel umfasst, wobei das erste Peptid eine Konjugation mit dem zweiten Peptid erfährt, um einen Linker zu bilden, und dadurch eine Erkennungselement-Linker-SAM-modifizierte Oberfläche bereitstellt, wobei die organischen Moleküle Thiole, vorzugsweise Dithiole, insbesondere 1,6-Hexandithiole umfassen und/oder es sich bei dem ersten Peptid um ein SpyTag-Peptid handelt.

10. Verfahren nach Anspruch 9, wobei: (a) es sich bei dem zweites-Peptid-Erkennungselement um ein SpyCatcher-Nanobody-Konjugat handelt; wobei es sich bei dem Nanobody möglicherweise um einen Nanobody handelt, der an die Rezeptorbindungsdomäne von SAR-CoV-2 bindet; und/oder (b) es sich bei dem Blockiermittel um BSA handelt.

11. Verfahren zum Nachweis der Abwesenheit, des Vorliegens oder der Konzentration eines Analyten in einer biologischen Probe, wobei es das Inkontaktbringen der Probe mit einem Biosensor nach einem beliebigen der Ansprüche 1 bis 8 umfasst, wobei der Biosensor weiterhin ein Reservoir umfasst,

   wobei das Verfahren Folgendes umfasst:

   (i) Einfüllen einer Elektrolytlösung in das Reservoir;
   (ii) Inkubieren der Gate-Elektrode mit einer Blindlösung;
   (iii) Anordnen der Gate-Elektrode oberhalb des Kanals;
   (iv) Anlegen einer $V_G$ und einer $V_D$;
   (v) Messen eines ersten $I_D$;
   (vi) Inkubieren der Gate-Elektrode mit der biologischen Probe über einen Zeitraum, der ausreicht, um eine Bindung zwischen dem Analyten und dem Bioerkennungselement zu ermöglichen;
   (vii) Spülen der Gate-Elektrode mit einem Spülpuffer; und
   (viii) Messen eines zweiten $I_D$,

   wobei eine Differenz zwischen dem zweiten $I_D$ und dem ersten $I_D$ die Abwesenheit, das Vorliegen oder die Konzentration des Analyten in der biologischen Probe anzeigt, und wobei die biologische Probe in flüssiger Form vorliegt;
   wobei der Schritt (v) möglicherweise gleichzeitig mit, im Wesentlichen gleichzeitig mit dem Schritt (iv) oder im Anschluss an diesen durchgeführt wird;

wobei die Schritte (vi)-(viii) einmal oder mehrmals wiederholt werden, und

wobei es sich bei der biologischen Probe wahlweise um (a) eine Körperflüssigkeit, die aus der Gruppe ausgewählt ist, welche aus Vollblut, Plasma, Serum, Speichel, Schleim, Auswurf, bronchoalveolärer Lavage (BAL), Bronchienspüllösung (BW), Cerebrospinalflüssigkeit (CSF) und Urin besteht, oder um (b) eine nicht-Körperflüssigkeit handelt.

12. Verfahren nach Anspruch 11, weiterhin umfassend:

(a) einen Schritt des Aufarbeitens eines Spezimens zu der biologischen Probe, im Vorfeld von jedwedem der Schritte (i)-(vi), wobei das Spezimen aus der Gruppe ausgewählt ist, die aus Gewebestücken, Kot, Rektalabstrich, Nasen-Rachen-Abstrich und Halsabstrich besteht; und/oder

(b) einen Schritt des Zusetzens eines Proteasenhemmers in die biologische Probe, im Vorfeld von jedwedem der Schritte (i)-(vi), wobei weiterhin möglicherweise (1) das Volumen der biologischen Probe weniger als 20 μL, weniger als 10 μL oder weniger als 5 μL beträgt und/oder (2) die Gate-Elektrode über einen Zeitraum von bis zu 60 Minuten, bis zu 50 Minuten, bis zu 40 Minuten, bis zu 30 Minuten, bis zu 20 Minuten oder bis zu 10 Minuten mit der biologischen Probe inkubiert wird.

13. Verfahren nach Anspruch 11 oder 12, wobei es weiterhin einen Schritt des Verdünnens der biologischen Probe unter Verwendung eine Sensorbindungspuffers, im Vorfeld von jedwedem der Schritte (i) bis (vi), vorzugsweise im Vorfeld des Schrittes (iv), umfasst, wobei der Sensorbindungspuffer ein Pufferungsmittel in einer Konzentration, welche im Bereich von ungefähr 10 mM bis ungefähr 50 mM liegt, ein Salz in einer Konzentration, welche im Bereich von ungefähr 100 mM bis ungefähr 300 mM liegt, Tween in einer Konzentration, welche im Bereich von ungefähr 0,01 % bis ungefähr 1 % (nach Volumen oder Gewicht) liegt, einen Proteasehemmer-Cocktail oder eine solche Tablette, wie etwa cOmplete, und ein antimikrobielles Mittel in einer Konzentration umfasst, welche im Bereich von ungefähr 0,01 % bis ungefähr 0,05 % (nach Volumen oder Gewicht) liegt.

14. Verfahren nach einem beliebigen der Ansprüche 11 bis 13, wobei es weiterhin einen Schritt des Verdünnens der biologischen Probe unter Verwendung eine Sensorbindungspuffers, im Vorfeld von jedwedem der Schritte (i) bis (vi), insbesondere im Vorfeld des Schrittes (vi), umfasst,

wobei der Sensorbindungspuffer Folgendes umfasst:

ein Pufferungsmittel, wie etwa Tris oder HERES, oder Phosphatsalze, in einer Konzentration, die im Bereich von ungefähr 10 mM bis ungefähr 100 mM liegt;

ein Salz, wie etwa KF, KBr, $K_2HPO_4$, Kaliumacetat, Kaliumcitrat, LiF, LiBr, $Li_2HPO_4$, Lithiumacetat oder Lithiumcitrat, oder eine Kombination davon, in einer Konzentration, die im Bereich von ungefähr 200 mM bis ungefähr 1 M liegt;

ein Detergens, das aus der Gruppe ausgewählt ist, welche aus Triton-X100, Nonidet P40, IGEPAL CA-630, Brij-35, Brij-58 und Triton X-114 und einer Kombinations davon besteht, in einer Konzentration, die im Bereich von ungefähr 0,5 % bis ungefähr 2 % (nach Volumen oder Gewicht) liegt;

einen Proteasehemmer-Cocktail oder eine solche Tablette, wie etwa cOmplete, in Konzentrationen von 1X, 2X oder 4X; und

ein Blockiermittel, wie etwa BSA, in einer Konzentration, welche im Bereich von ungefähr 0,05 % bis 1 % (nach Volumen oder Gewicht) liegt; und möglicherweise ein antimikrobielles Mittel, wie etwa $NaN_3$, in einer Konzentration, die im Bereich von ungefähr 0,01 % bis 0,05 % (nach Volumen oder Gewicht) liegt, und/oder

ein RNA-verdauendes Enzym, wie etwa das Enzym RNAse A, in einer Konzentration, die im Bereich von ungefähr 1 mg/L bis ungefähr 50 mg/L liegt.

15. Biosensor nach den Ansprüchen 1 bis 8, wobei die Bioerkennungsschicht derart integriert ist, dass sie sich auf der Gate-Elektrode des OECT befindet.

**Revendications**

1. Biocapteur comprenant un transistor électrochimique organique (OECT), et éventuellement un réseau d'au moins deux OECT, et une couche de bioreconnaissance, dans lequel l'OECT ou chacun des OECT dans le réseau comprend une électrode de source, une électrode de drain, un canal, et une électrode de grille,

dans lequel l'orientation de la couche de bioreconnaissance par rapport à la surface de l'OECT est représentée par la formule générale :

$$N\text{-}L_1\text{-}AP_1\text{-}AP_2\text{-}L_2\text{-}B \qquad \text{Formule I}$$

dans laquelle N est une ou plusieurs molécules organiques qui s'auto-assemblent pour former une première SAM, $L_1$ est un premier lieur facultatif, $AP_1$ est un premier partenaire de liaison peptidique, $AP_2$ est un deuxième partenaire peptidique, $AP_1$ et $AP_2$ sont des partenaires de liaison, $L_2$ est un deuxième lieur, et B est un élément de bioreconnaissance,
dans lequel $AP_1$-$AP_2$ est choisi dans le groupe constitué par un conjugué peptidique SpyTag/SpyCatcher, un conjugué peptidique SnoopCatcher/SnoopTag, un conjugué peptidique MoonTag/MoonCatcher, un conjugué peptidique SnoopTagJr/SnoopCatcher, un conjugué peptidique SnoopTagJr/DogTag, et un conjugué peptidique SdyTag/SdyCatcher.

2. Biocapteur selon la revendication 1, dans lequel $AP_1$-$AP_2$ est un conjugué peptidique SpyTag/SpyCatcher.

3. Biocapteur selon la revendication 1 ou 2, dans lequel la couche de bioreconnaissance comprend une Chem-SAM, dans lequel la Chem-SAM comprend la première SAM formée par N, modifiée chimiquement avec $AP_1$.

4. Biocapteur selon l'une quelconque des revendications 1 à 3, dans lequel l'élément de bioreconnaissance est un anticorps, ou un fragment d'anticorps à deux domaines ou un seul domaine, tel qu'un nanocorps, éventuellement dans lequel le nanocorps reconnaît un domaine de liaison au récepteur SAR-CoV-2.

5. Biocapteur selon l'une quelconque des revendications 1 à 4, dans lequel la ou les molécules organiques dans la première SAM comprennent des thiols, éventuellement dans lequel les thiols comprennent des dithiols, de préférence des 1,6-hexanedithiols.

6. Biocapteur selon l'une quelconque des revendications 1 à 5, dans lequel la couche de bioreconnaissance comprend une Bio-SAM comprenant $AP_1$-$AP_2$-$L_2$-B.

7. Biocapteur selon l'une quelconque des revendications 1 à 6, (a) comprenant en outre un agent bloquant, éventuellement dans lequel l'agent bloquant est l'albumine sérique bovine (BSA), et/ou (b) dans lequel le canal est formé à partir d'un polymère conducteur choisi dans le groupe constitué par PEDOT:PSS, PEDOT-S, PEDOT:TOS, PEDOTOH:$ClO_4$, PEDOT-$co$-PEDOTOH:$ClO_4$, P3HT, PTHS, BBL, p(g2T-TT), PTHS-TMA$^+$-$co$-P3HT, p(gNDI-g2T), p(g0T2-g6T2), et P-90.

8. Biocapteur selon l'une quelconque des revendications 1 à 7, dans lequel l'OECT ou chacun des OECT dans le réseau est modelé sur un substrat de support.

9. Procédé d'intégration d'une couche de bioreconnaissance sur une électrode comprenant :

(i) l'incubation d'au moins une partie de la surface de l'électrode avec une première solution d'incubation comprenant une pluralité de molécules organiques pour produire une structure SAM-surface modifiée,
(ii) l'incubation de la structure SAM-surface modifiée avec une deuxième solution d'incubation comprenant un premier peptide pour produire une structure premier peptide-SAM-surface modifiée, et
(iii) l'incubation de la structure premier peptide-SAM-surface modifiée avec une troisième solution d'incubation comprenant un conjugué deuxième peptide-élément de reconnaissance et un agent bloquant, où le premier peptide se conjugue avec le deuxième peptide pour former un lieur et produit une structure élément de reconnaissance-lieur-SAM-surface modifiée, éventuellement dans lequel les molécules organiques comprennent des thiols, de préférence des dithiols, mieux encore des 1,6-hexanedithiols, et/ou le premier peptide est un peptide SpyTag.

10. Procédé selon la revendication 9, dans lequel : (a) le conjugué deuxième peptide-élément de reconnaissance est un conjugué SpyCatcher-nanocorps ; éventuellement dans lequel le nanocorps est un nanocorps de liaison au domaine de liaison au récepteur SAR-CoV-2 ; et/ou (b) l'agent bloquant est la BSA.

11. Procédé de détection de l'absence, de la présence ou de la concentration d'un analyte dans un échantillon biologique comprenant la mise en contact de l'échantillon avec le biocapteur selon l'une quelconque des revendications 1 à 8, dans lequel le biocapteur comprend en outre un réservoir,

le procédé comprenant :

(i) l'ajout d'une solution d'électrolyte dans le réservoir ;
(ii) l'incubation de l'électrode de grille avec une solution à blanc ;
(iii) la mise en place de l'électrode de grille au-dessus du canal ;
(iv) l'application d'une $V_G$ et d'une $V_D$ ;
(v) la mesure d'une première $I_D$ ;
(vi) l'incubation de l'électrode de grille avec l'échantillon biologique pendant un laps de temps suffisant pour permettre une liaison ente l'analyte et l'élément de bioreconnaissance ;
(vii) le rinçage de l'électrode de grille avec un tampon de rinçage ; et
(viii) la mesure d'une deuxième $I_D$,

dans lequel une différence entre la deuxième $I_D$ et la première $I_D$ est indicative de l'absence, de la présence ou de la concentration de l'analyte dans l'échantillon biologique, et dans lequel l'échantillon biologique se présente sous forme liquide ;
éventuellement dans lequel l'étape (v) est effectuée de façon simultanée ou sensiblement simultanée avec, ou ultérieurement à l'étape (iv) ;
éventuellement dans lequel les étapes (vi)-(viii) sont répétées une ou plusieurs fois ; et
éventuellement dans lequel l'échantillon biologique est (a) un liquide corporel choisi dans le groupe constitué par du sang total, du plasma, du sérum, de la salive, du mucus, du crachat, un lavage broncho-alvéolaire (BAL), un lavage bronchique (BW), du liquide céphalorachidien (CSF) et de l'urine, ou (b) un liquide non corporel.

12. Procédé selon la revendication 11 comprenant en outre :

(a) une étape de traitement d'un prélèvement pour obtenir l'échantillon biologique avant l'une quelconque des étapes (i)-(vi), le prélèvement étant choisi dans le groupe constitué par des tissus, des selles, un frottis rectal, un frottis nasopharyngé, et un frottis de la gorge ; et/ou
(b) une étape d'ajout d'un inhibiteur de protéase dans l'échantillon biologique avant l'une quelconque des étapes (i)-(vi), et éventuellement dans lequel (1) le volume de l'échantillon biologique est inférieur à 20 μL, inférieur à 10 μL, ou inférieur à 5 μL et/ou (2) l'électrode de grille est incubée avec l'échantillon biologique pendant un laps de temps allant jusqu'à 60 minutes, jusqu'à 50 minutes, jusqu'à 40 minutes, jusqu'à 30 minutes, jusqu'à 20 minutes, ou jusqu'à 10 minutes.

13. Procédé selon la revendication 11 ou 12 comprenant en outre une étape de dilution de l'échantillon biologique au moyen d'un tampon de liaison de capteur, avant l'une quelconque des étapes (i)-(vi), de préférence avant l'étape (iv), dans lequel le tampon de liaison de capteur comprend un agent tampon à une concentration dans une plage d'environ 10 mM à environ 50 mM, un sel à une concentration dans une plage d'environ 100 mM à environ 300 mM, du Tween à une concentration dans une plage d'environ 0,01 % à environ 1 % (en volume ou en poids), un cocktail ou comprimé d'inhibiteurs de protéase, tel que du cOmplete, et un agent antimicrobien à une concentration dans une plage d'environ 0,01 % à environ 0,05 % (en volume ou en poids).

14. Procédé selon l'une quelconque des revendications 11 à 13 comprenant en outre une étape de dilution de l'échantillon biologique au moyen d'un tampon de liaison de capteur, avant l'une quelconque des étapes (i)-(vi), en particulier avant l'étape (vi),
dans lequel le tampon de liaison de capteur comprend :

un agent tampon, tel que du Tris ou de l'HEPES, ou des sels de phosphate, à une concentration dans une plage d'environ 10 mM à environ 100 mM ;
un sel, tel que KF, KBr, $K_2HPO_4$, l'acétate de potassium, le citrate de potassium, LiF, LiBr, $Li_2HPO_4$, l'acétate de lithium ou le citrate de lithium, ou une combinaison de ceux-ci, à une concentration dans une plage d'environ 200 mM à environ 1 M ;
un détergent choisi dans le groupe constitué par le Triton-X100, le Nonidet P40, l'IGEPAL CA-630, le Brij-35, le Brij-58, et le Triton X-114, et une combinaison de ceux-ci, à une concentration dans une plage d'environ 0,5 % à environ 2 % (en volume ou en poids) ;
un cocktail ou comprimé d'inhibiteurs de protéase, tel que du cOmplete, à une concentration de 1X, 2X, ou 4X ; et
un agent bloquant, tel que la BSA, à une concentration dans une plage d'environ 0,05 % à 1 % (en volume ou en poids) ; et éventuellement
un agent antimicrobien, tel que $NaN_3$, à une concentration dans une plage d'environ 0,01 % à environ 0,05 % (en volume ou en poids) et/ou
une enzyme de digestion de l'ARN, telle qu'une enzyme RNAse A, à une concentration dans une plage d'environ 1 mg/L à environ 50 mg/L.

15. Biocapteur selon les revendications 1 à 8, dans lequel la couche de bioreconnaissance est intégrée sur l'électrode de grille de l'OECT.

**FIG. 1A**

**FIG. 1B**

**FIG. 1C**

**FIG. 1D**

**FIG. 2A**

**FIG. 2B**

**FIG. 2C**

**FIG. 2D**

**FIG. 2E**

FIG. 2F

**FIG. 2G**

**FIG. 2H**

**FIG. 2I**

**FIG. 3A**

**FIG. 3B**

**FIG. 3C**

**FIG. 3D**

**FIG. 3E**

**FIG. 3F**

FIG. 3G

FIG. 3H

FIG. 3I

**FIG. 3J**

**FIG. 4A**

**FIG. 4B**

**FIG. 4C**

**FIG. 4D**

**FIG. 4E**

**FIG. 4F**

**FIG. 4G**

**FIG. 5A**

**FIG. 5B**

**FIG. 5C**

**FIG. 5D**

FIG. 5E

FIG. 6A

FIG. 6B

**FIG. 6C**

**FIG. 6D**

**FIG. 7A**

**FIG. 7B**

**FIG. 7C**

**FIG. 7D**

**FIG. 7E**

**FIG. 7F**

**FIG. 8A**          **FIG. 8B**

**FIG. 8C**          **FIG. 8D**

**FIG. 8E**  **FIG. 8F**

**FIG. 8G**

**FIG. 8H**

FIG. 8I

FIG. 9A

FIG. 9B

FIG. 9C

EP 4 176 262 B1

FIG. 9D

FIG. 9E

FIG. 9F

FIG. 9G

FIG. 9H

**FIG. 10**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 63047547 A
- US 63106218 A
- WO 2018048742 A1
- US 2019331673 A1
- WO 2021130525 A1
- US 10975139 B
- US 11021532 B

### Non-patent literature cited in the description

- **MACCHIA ELEONORA et al.** 2017 7th IEEE International Workshop On Advances In Sensors And Interfaces (IWASI). IEEE, 2017, 68-72
- **ZAKERI et al.** *Proc. Natl. Acad. Sci.*, 2012, vol. 109, E690-E697
- **LOVE et al.** *Chem. Rev.,*, 2005, vol. 105, 1103-1169
- **KEEBLE et al.** *Angew. Chem. Int. Ed. Engl.*, 2017, vol. 56, 16521-16525
- **KEEBLE et al.** *Proc Natl Acad Sci U S A*, 26 December 2019, vol. 116 (52), 26523-26533
- **VEGGIANI et al.** *PNAS 2316*, vol. 113 (5), 1202-1207
- **HATLEM et al.** *Int J Mol Sci.*, May 2019, vol. 20 (9), 2129
- **WRAPP et al.** *Science*, 2020, vol. 367 (6483), 1260-1263
- **RIVNAY et al.** *Nature Reviews*, 2018, vol. 3, 17086
- **SUN et al.** *J. Mater. Chem. C,*, 2018, vol. 6, 11778-11784
- **MOSER et al.** *Adv. Mater.,*, 2020, vol. 32, 2002748
- **GIOVANNITTI et al.** *Chem. Mater.*, 2018, vol. 30, 2945-2953
- **SCHMODE et al.** *Chem. Mater.,*, 2019, vol. 31 (14), 5286-5295
- **MOSER et al.** *Advanced Materials*, 2020, vol. 32, 2002748
- **SUBRAMANI et al.** *Mol. Cell. Biol.*, 1981, vol. 1, 854-864
- **PALMITER et al.** *Science*, 1983, vol. 222, 809-814
- **BOSHART et al.** *Cell*, 1985, vol. 41, 521-530
- **KAUFMAN ; SHARP.** *Mol. Cell. Biol*, 1982, vol. 2, 1304-1319
- **ZAKERI et al.** *Proc. Natl. Acad. Sci.,*, 2012, vol. 109, E690-E697
- **LOPEZ et al.** *Pediatr. Res.*, 2019, vol. 86 (5), 651-654
- **WU et al.** Fully human single-domain antibodies against SARS-CoV-2.. *bioRxiv*, 2020
- **RAJ et al.** *Sci. Adv.*, 2018, vol. 4, eaas9667
- **WRAPP et al.** *Cell*, 2020, vol. 181, 1004-1015
- *WHO Coronavirus Disease (COVID-19) Dashboard.*, 2020, https://covid9.who.int
- **KISSLER et al.** *Science*, 2020, vol. 368, 860-868
- **LEWIS.** *Nature*, 2020, vol. 583, 510-513
- **ORAN et al.** *Ann. Intern. Med*, 2020
- **LONG. et al.** *Nat. Med.*, 2020, vol. 26, 1200-1204
- *WHO Director-General's opening remarks at the media briefing on COVID-19*, 16 March 2020, https://www.who.int/dg/speeches/detail/who-director-general-s-opening-remarks-at-the-media-briefing-on-covid-19---16-march-2020
- **CORMAN et al.** *Eurosurveillance*, 2020, vol. 25
- **FERRETTI et al.** *Science*, 2020, vol. 368
- **MORAZ et al.** *medRxiv 2020.07.10.20149773*, 2020
- **PASOMSUB, E. et al.** Saliva sample as a non-invasive specimen for the diagnosis of coronavirus disease 2019: a cross-sectional study.. *Clin. Microbiol. Infect.*, 2020, vol. 0
- **WYLLIE et al.** *N. Engl. J. Med.*, 2020, vol. 383, 1283-1286
- **KRUEGER et al.** *medRxiv 2020.10.01.20203836*, 2020
- **TORRENTE-RODRIGUEZ et al.** *Matter*, 2020, vol. 3, 1-18
- **MACCHIA et al.** Single-molecule detection with a millimetre-sized transistor.. *Nat. Commun.*, 2018, vol. 9, 3223
- **RIVNAY et al.** *Nat. Rev. Mater.*, 2018, vol. 3, 1-14
- **INAL et al.** *Acc. Chem. Res.*, 2018, vol. 51, 1368-1376
- **OHAYON et al.** *Adv. Mater.*, 2020, vol. 32, 2001439

- **GENTILI et al.** *J. Mater. Chem. B*, 2018, vol. 6, 5400-5406
- **TRILLING et al.** *Analyst*, 2013, vol. 138, 1619-1627
- **ZAKERI et al.** *Proc. Natl. Acad. Sci. U. S. A.*, 2012, vol. 109, E690-E697
- **MUYLDERMANS**. *Annu. Rev. Biochem.*, 2013, vol. 82, 775-797
- **HAMERS-CASTERMAN et al.** *Nature*, 1993, vol. 363, 446-448
- **STEELAND et al.** *Today*, 2016, vol. 21, 1076-1113
- **DE MEYER**. *Trends Biotechnol.*, 2014, vol. 32, 263-270
- **FILIPIAK et al.** *Sens. Actuators B Chem.*, 2018, vol. 255, 1507-1516
- **ROTHBAUER et al.** *Mol. Cell. Proteomics*, 2008, vol. 7, 282-289
- **KEEBLE et al.** *Methods Enzymol.*, 2019, vol. 617, 443-461
- **OLOKETUYI et al.** *Biosens. Bioelectron.*, 2020, vol. 154, 112052
- **ESPLANDIU et al.** *Appl. Surf. Sci.*, 2002, vol. 199, 166-182
- **LOVE et al.** *Chem. Rev.*, 2005, vol. 105, 1103-1170
- Redox reactions of sulphur-containing amino-acid residues in proteins and metalloproteins, an XPS study.. **WESER, U.** Cation Ordering and Electron Transfer. Springer, 1985, 145-160
- **MACCHIA et al.** *Flex. Print. Electron.*, 2018, vol. 3, 034002
- **ZHANG et al.** *Analyst*, 2013, vol. 139, 439-445
- **MOSER et al.** *Adv. Funct. Mater.*, 2019, vol. 29, 1807033
- **MOSER et al.** Side Chain Redistribution as a Strategy to Boost Organic Electrochemical Transistor Performance and Stability.. *Adv. Mater. n/a*, 2002748
- **VENKATRAMAN et al.** *Adv. Sci. Weinh. Baden-Wurtt. Ger.*, 2018, vol. 5, 1800453
- **KUBALA et al.** *Protein Sci. Publ. Protein Soc.*, 2010, vol. 19, 2389-2401
- **OHAYON et al.** *Nat. Mater.*, 2020, vol. 19, 456-463
- **WUSTONI et al.** *Adv. Mater. Interfaces*, 2019, vol. 6, 1800928
- **QU et al.** *J. Phys. Chem. C*, 2010, vol. 114, 497-505
- **HÖÖK et al.** *Langmuir*, 1998, vol. 14, 729-734
- **ESHAGHI et al.** *Angew. Chem. Int. Ed Engl.*, 2015, vol. 54, 13952-13956
- **LI et al.** *J. Mol. Biol.*, 2014, vol. 426, 309-317
- Edinburgh-Genome-Foundry/DnaChisel.. Edinburgh Genome Foundry, 2020
- **MUTALIK et al.** *Nat. Methods*, 2013, vol. 10, 354-360
- **LONG et al.** *Anal. Chem.*, 1983, vol. 55, 712A-724A
- *ExPASy - ProtParam tool.*, https://web.expasy.org/-protparam